# EUROPEAN PATENT APPLICATION

(11) **EP 3 725 888 A1**
(43) Date of publication of application: **21.10.2020**
(21) Application number: 19169841.4
(22) Date of filing: 17.04.2019
(51) Int. Cl.: C12N 15/861, A61K 48/00

(54) **ONCOLYTIC ADENOVIRAL VECTOR EXPRESSING PEPTIDYLARGININE DEIMINASE AND TISSUE INHIBITOR OF METALLOPROTEINASE**

(71) Applicant: TARGOVAX ASA, 0283 Oslo (NO)
(72) Inventor: KURYK, Lukasz, 00180 Helsinki (FI); ERIKSEN, Jon Amund, 3916 Porsgrunn (NO); SKORPIL, Peter, 1447 Drøbak (NO); BURNS, Robert, Oxford, OX1 5ER (GB); MØLLER, Anne-Sophie, 1358 Jar (NO)
(74) Representative: Boco IP Oy Ab

(57) **Abstract**

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing peptidylarginine deiminase and TIMP in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

SEQUENCE LISTING

<110> Targovax ASA

<120> ONCOLYTIC ADENOVIRAL VECTOR EXPRESSING PEPTIDYLARGININE DEIMINASE AND
TISSUE INHIBITOR OF METALLOPROTEINASE

<130> B8375PEP

<160> 35

<170> BiSSAP 1.3.6

<210> 1
<211> 37454
<212> RNA
<213> Artificial Sequence

<220>
<223> Ad5-D24-PADI1-TIMP2

<400> 1
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520
taccaacatg ccaaatgtga acgagttcat gtttaccaat aagtttaagg cgcgggtgat 14580
ggtgtcgcgc ttgcctacta aggacaatca ggtggagctg aaatacgagt gggtggagtt 14640
cacgctgccc gagggcaact actccgagac catgaccata gaccttatga acaacgcgat 14700
cgtggagcac tacttgaaag tgggcagaca gaacggggtt ctggaaagcg acatcggggt 14760
aaagtttgac acccgcaact tcagactggg gtttgacccc gtcactggtc ttgtcatgcc 14820
tggggtatat acaaacgaag ccttccatcc agacatcatt ttgctgccag gatgcggggt 14880
ggacttcacc cacagccgcc tgagcaactt gttgggcatc cgcaagcggc aacccttcca 14940
ggagggcttt aggatcacct acgatgatct ggagggtggt aacattcccg cactgttgga 15000
tgtggacgcc taccaggcga gcttgaaaga tgacaccgaa cagggcgggg gtggcgcagg 15060
cggcagcaac agcagtggca gcggcgcgga agagaactcc aacgcggcag ccgcggcaat 15120
gcagccggtg gaggacatga acgatcatgc cattcgcggc gacacctttg ccacacgggc 15180
tgaggagaag cgcgctgagg ccgaagcagc ggccgaagct gccgcccccg ctgcgcaacc 15240
cgaggtcgag aagcctcaga agaaaccggt gatcaaaccc ctgacagagg acagcaagaa 15300
acgcagttac aacctaataa gcaatgacag caccttcacc cagtaccgca gctggtacct 15360
tgcatacaac tacggcgacc ctcagaccgg aatccgctca tggaccctgc tttgcactcc 15420
tgacgtaacc tgcggctcgg agcaggtcta ctggtcgttg ccagacatga tgcaagaccc 15480
cgtgaccttc cgctccacgc gccagatcag caactttccg gtggtgggcg ccgagctgtt 15540
gcccgtgcac tccaagagct tctacaacga ccaggccgtc tactcccaac tcatccgcca 15600
gtttacctct ctgacccacg tgttcaatcg ctttcccgag aaccagattt tggcgcgccc 15660
gccagccccc accatcacca ccgtcagtga aaacgttcct gctctcacag atcacgggac 15720
gctaccgctg cgcaacagca tcggaggagt ccagcgagtg accattactg acgccagacg 15780
ccgcacctgc ccctacgttt acaaggccct gggcatagtc tcgccgcgcg tcctatcgag 15840
ccgcactttt tgagcaagca tgtccatcct tatatcgccc agcaataaca caggctgggg 15900
cctgcgcttc ccaagcaaga tgtttggcgg ggccaagaag cgctccgacc aacacccagt 15960
gcgcgtgcgc gggcactacc gcgcgccctg gggcgcgcac aaacgcggcc gcactgggcg 16020
caccaccgtc gatgacgcca tcgacgcggt ggtggaggag gcgcgcaact acacgcccac 16080
gccgccacca gtgtccacag tggacgcggc cattcagacc gtggtgcgcg gagcccggcg 16140
ctatgctaaa atgaagagac ggcggaggcg cgtagcacgt cgccaccgcc gccgacccgg 16200
cactgccgcc caacgcgcgg cggcggccct gcttaaccgc gcacgtcgca ccggccgacg 16260
ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660

gccggcaacg ccacaacata aagaagcaag caacatcaac aacagctgcc gccatgggct 21720

ccagtgagca ggaactgaaa gccattgtca aagatcttgg ttgtgggcca tatttttggg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tccagatctt ctagacccgg gagcggccgc 28140

tgtcgacctg caggatccga attcgatatc actagctagt tattaatagt aatcaattac 28200

ggggtcatta gttcatagcc catatatgga gttccgcgtt acataactta cggtaaatgg 28260

cccgcctggc tgaccgccca acgacccccg cccattgacg tcaataatga cgtatgttcc 28320

catagtaacg ccaataggga ctttccattg acgtcaatgg gtggagtatt tacggtaaac 28380

tgcccacttg gcagtacatc aagtgtatca tatgccaagt ccgcccccta ttgacgtcaa 28440

tgacggtaaa tggcccgcct ggcattatgc ccagtacatg accttacggg actttcctac 28500

ttggcagtac atctacgtat tagtcatcgc tattaccatg gtgatgcggt tttggcagta 28560

caccaatggg cgtggatagc ggtttgactc acggggattt ccaagtctcc accccattga 28620

cgtcaatggg agtttgtttt ggcaccaaaa tcaacgggac tttccaaaat gtcgtaatac 28680

ccccgccccg ttgacgcaaa tgggcaagct tgccgggtcg aggtaggcgt gtacggtggg 28740

aggcctatat aagcaaccgg tataatcaaa cagaccagat tgtctgtttg ttaccggtgt 28800

ttagtgaacc gggcgcgcct catatcgcct ggagacgcca tccacgctgt tttgacctcc 28860

atagaagaca ccgggaccga tccagcctcc gcgggccgcc accatggccc caaagagagt 28920

tgtgcagctg tccctgaaga tgcctaccca tgccgtgtgt gtggtgggag tcgaggcaca 28980

tgtggacatt cacagtgatg tgcccaaggg tgccaacagc ttcagggtct ctggaagctc 29040

cggggtggag gtcttcatgg tctacaaccg cacacgtgtg aaagagccca taggcaaggc 29100

ccgttggccg ctagacactg atgcagacat ggtcgtatct gtgggcacag ccagtaagga 29160

attaaaggac ttcaaggtga gggtctccta ctttggggag caggaagacc aagctctggg 29220

ccgcagcgtg ctttacctca ctggcgtcga tatttccctt gaggttgaca caggccgcac 29280

aggcaaggtg aagaggagcc aaggggacaa gaaaacctgg cgctggggcc ctgagggcta 29340

tggggctatc ttgctggtga actgtgaccg ggacaatcac aggtccgcgg agcctgacct 29400

cacccacagc tggctgatgt cgctggctga cctgcaggac atgtccccaa tgctgctgag 29460

ctgcaatggc cccgacaagc tcttcgacag ccacaagctt gtcttgaacg tgcccttttc 29520

tgattccaaa agagtgaggg tcttctgtgc caggggtggg aattctctct cggactacaa 29580

acaggtgctg gggccccagt gtctgtccta tgaagttgag cgacagccag gggagcagga 29640

gatcaagttc tatgtggagg ggctgacctt ccccgatgcc gatttcctag ggctggtttc 29700

cctcagtgtc agcctggtgg acccggggac cctgcccgag gtgaccctct tcacagacac 29760

tgtgggcttc cgcatggccc cctggatcat gacgcccaac actcagcctc ctgaggagct 29820

gtatgtgtgc agagtgatgg acactcatgg ctccaatgag aaattcctgg aggacatgtc 29880

ttatctgaca ttgaaagcca actgcaagct gaccatctgc cctcaagttg aaaatcgaaa 29940

tgaccgctgg atccaggacg agatggagtt tggctacatc gaggcccctc acaaatcctt 30000

ccccgtggtc tttgactccc ccaggaacag gggcctgaaa gatttcccct ataagaggat 30060

cctgggtcct gactttggat atgttacccg ggagatcccg ctccctggtc cctccagcct 30120

tgactccttc ggcaacctgg acgtcagccc gcccgtcacg gggggcggca cggaataccc 30180

cctgggccgg atcctcatcg ggagcagctt ccccaagtcc ggtgggcggc agatggccag 30240

ggcagtgcgg aacttcctga aggcacagca ggtgcaggca cccgtggagc tctactcgga 30300

ctggctctct gtgggccatg tggacgagtt tctgaccttt gtgcctacct ctgaccagaa 30360

gggcttccgg ctgctcctgg ctagccccag cgcttgcccc aaactcttcc aagagaagaa 30420

agaagagggt tatggggagg cagcccagtt tgatgggtta aaacaccagg caaaaagaag 30480

cattaatgag atgctggcag acagacacct ccagagagac aatcttcatg cacagaaatg 30540

cattgactgg aaccgtaatg tgctgaagcg ggagctgggc ctggcagaga gtgacatcgt 30600

ggacattccc cagctcttct tcctgaaaaa cttctacgcg gaagccttct tcccagacat 30660

ggttaacatg gtggtcttag gcaagtacct gggcatcccc aagccctacg ggcccatcat 30720

caatggccgc tgctgcctgg aggagaaggt gcagtccctg ctggagcctc tgggcctgca 30780

ctgcatcttc attgatgact acttgtccta ccacgagctg cagggggaga tccactgtgg 30840

caccaacgtg cgcaggaagc cctttccctt caaatggtgg aacatggtgc cctgacccct 30900

ctccctcccc cccctaacgt tactggccga agccgcttgg aataaggccg gtgtgcgttt 30960

gtctatatgt tattttccac catattgccg tcttttggca atgtgagggc ccggaaacct 31020

ggccctgtct tcttgacgag cattcctagg ggtctttccc ctctcgccaa aggaatgcaa 31080

ggtctgttga atgtcgtgaa ggaagcagtt cctctggaag cttcttgaag acaaacaacg 31140

tctgtagcga ccctttgcag gcagcggaac cccccacctg gcgacaggtg cctctgcggc 31200

caaaagccac gtgtataaga tacacctgca aaggcggcac aaccccagtg ccacgttgtg 31260

agttggatag ttgtggaaag agtcaaatgg ctctcctcaa gcgtattcaa caaggggctg 31320

aaggatgccc agaaggtacc ccattgtatg ggatctgatc tggggcctcg gtgcacatgc 31380

tttacatgtg tttagtcgag gttaaaaaaa cgtctaggcc ccccgaacca cggggacgtg 31440

gttttccttt gaaaaacacg atgataagct tgccacaacc cgggatcctc tagagtcgac 31500

atgggcgccg cggcccgcac cctgcggctg gcgctcggcc tcctgctgct ggcgacgctg 31560

cttcgcccgg ccgacgcctg cagctgctcc ccggtgcacc cgcaacaggc gttttgcaat 31620

gcagatgtag tgatcagggc caaagcggtc agtgagaagg aagtggactc tggaaacgac 31680

atttatggca accctatcaa gaggatccag tatgagatca agcagataaa gatgttcaaa 31740

gggcctgaga aggatataga gtttatctac acggccccct cctcggcagt gtgtggggtc 31800

tcgctggacg ttggaggaaa gaaggaatat ctcattgcag gaaaggccga gggggacggc 31860

aagatgcaca tcaccctctg tgacttcatc gtgccctggg acaccctgag caccacccag 31920

aagaagagcc tgaaccacag gtaccagatg ggctgcgagt gcaagatcac gcgctgcccc 31980

atgatcccgt gctacatctc ctccccggac gagtgcctct ggatggactg ggtcacagag 32040

aagaacatca acgggcacca ggccaagttc ttcgcctgca tcaagagaag tgacggctcc 32100

tgtgcgtggt accgcggcgc ggcgcccccc aagcaggagt ttctcgacat cgaggaccca 32160

taaaacttgt ttattgcagc ttataatggt tacaaataaa gcaatagcat cacaaatttc 32220

acaaataaag catttttttc actgcattct agttgtggtt tgtccaaact catcaatgta 32280

tcttaactag ctagtggtac cgatcttatt ccctttaact aataaaaaaa aataataaag 32340

catcacttac ttaaaatcag ttagcaaatt tctgtccagt ttattcagca gcacctcctt 32400

gccctcctcc cagctctggt attgcagctt cctcctggct gcaaactttc tccacaatct 32460

aaatggaatg tcagtttcct cctgttcctg tccatccgca cccactatct tcatgttgtt 32520

gcagatgaag cgcgcaagac cgtctgaaga taccttcaac cccgtgtatc catatgacac 32580

ggaaaccggt cctccaactg tgccttttct tactcctccc tttgtatccc ccaatgggtt 32640

tcaagagagt ccccctgggg tactctcttt gcgcctatcc gaacctctag ttacctccaa 32700

tggcatgctt gcgctcaaaa tgggcaacgg cctctctctg gacgaggccg gcaaccttac 32760

ctcccaaaat gtaaccactg tgagcccacc tctcaaaaaa accaagtcaa acataaacct 32820

ggaaatatct gcacccctca cagttacctc agaagcccta actgtggctg ccgccgcacc 32880

tctaatggtc gcgggcaaca cactcaccat gcaatcacag gccccgctaa ccgtgcacga 32940

ctccaaactt agcattgcca cccaaggacc cctcacagtg tcagaaggaa agctagccct 33000

gcaaacatca ggccccctca ccaccaccga tagcagtacc cttactatca ctgcctcacc 33060

ccctctaact actgccactg gtagcttggg cattgacttg aaagagccca tttatacaca 33120

aaatggaaaa ctaggactaa agtacggggc tcctttgcat gtaacagacg acctaaacac 33180

tttgaccgta gcaactggtc caggtgtgac tattaataat acttccttgc aaactaaagt 33240

tactggagcc ttgggttttg attcacaagg caatatgcaa cttaatgtag caggaggact 33300

aaggattgat tctcaaaaca gacgccttat acttgatgtt agttatccgt ttgatgctca 33360

aaaccaacta aatctaagac taggacaggg ccctcttttt ataaactcag cccacaactt 33420

ggatattaac tacaacaaag gcctttactt gtttacagct tcaaacaatt ccaaaaagct 33480

tgaggttaac ctaagcactg ccaaggggtt gatgtttgac gctacagcca tagccattaa 33540

tgcaggagat gggcttgaat ttggttcacc taatgcacca aacacaaatc ccctcaaaac 33600

aaaaattggc catggcctag aatttgattc aaacaaggct atggttccta aactaggaac 33660

tggccttagt tttgacagca caggtgccat tacagtagga aacaaaaata atgataagct 33720

aaccctatgg acaggtccaa aaccagaagc caactgcata attgaatacg ggaaacaaaa 33780

cccagatagc aaactaactt taatccttgt aaaaaatgga ggaattgtta atggatatgt 33840

aacgctaatg ggagcctcag actacgttaa caccttattt aaaaacaaaa atgtctccat 33900

taatgtagaa ctatactttg atgccactgg tcatatatta ccagactcat cttctcttaa 33960

aacagatcta gaactaaaat acaagcaaac cgctgacttt agtgcaagag gttttatgcc 34020

aagtactaca gcgtatccat ttgtccttcc taatgcggga acacataatg aaaattatat 34080

ttttggtcaa tgctactaca aagcaagcga tggtgccctt tttccgttgg aagttactgt 34140

tatgcttaat aaacgcctgc cagatagtcg cacatcctat gttatgactt ttttatggtc 34200

cttgaatgct ggtctagctc cagaaactac tcaggcaacc ctcataacct ccccatttac 34260

cttttcctat attagagaag atgactaata aactctaaag aatcgtttgt gttatgtttc 34320

aacgtgttta tttttcaatt gcagaaaatt tcaagtcatt tttcattcag tagtatagcc 34380

ccaccaccac atagcttata cagatcaccg taccttaatc aaactcacag aaccctagta 34440

ttcaacctgc cacctccctc ccaacacaca gagtacacag tcctttctcc ccggctggcc 34500

ttaaaaagca tcatatcatg ggtaacagac atattcttag gtgttatatt ccacacggtt 34560

tcctgtcgag ccaaacgctc atcagtgata ttaataaact ccccgggcag ctcacttaag 34620

ttcatgtcgc tgtccagctg ctgagccaca ggctgctgtc caacttgcgg ttgcttaacg 34680

ggcggcgaag gagaagtcca cgcctacatg ggggtagagt cataatcgtg catcaggata 34740

gggcggtggt gctgcagcag cgcgcgaata aactgctgcc gccgccgctc cgtcctgcag 34800

gaatacaaca tggcagtggt ctcctcagcg atgattcgca ccgcccgcag cataaggcgc 34860

cttgtcctcc gggcacagca gcgcaccctg atctcactta aatcagcaca gtaactgcag 34920

cacagcacca caatattgtt caaaatccca cagtgcaagg cgctgtatcc aaagctcatg 34980

gcggggacca cagaacccac gtggccatca taccacaagc gcaggtagat taagtggcga 35040

cccctcataa acacgctgga cataaacatt acctcttttg gcatgttgta attcaccacc 35100

tcccggtacc atataaacct ctgattaaac atggcgccat ccaccaccat cctaaaccag 35160

ctggccaaaa cctgcccgcc ggctatacac tgcagggaac cgggactgga acaatgacag 35220

tggagagccc aggactcgta accatggatc atcatgctcg tcatgatatc aatgttggca 35280

caacacaggc acacgtgcat acacttcctc aggattacaa gctcctcccg cgttagaacc 35340

atatcccagg gaacaaccca ttcctgaatc agcgtaaatc ccacactgca gggaagacct 35400

cgcacgtaac tcacgttgtg cattgtcaaa gtgttacatt cgggcagcag cggatgatcc 35460

tccagtatgg tagcgcgggt ttctgtctca aaaggaggta gacgatccct actgtacgga 35520

gtgcgccgag acaaccgaga tcgtgttggt cgtagtgtca tgccaaatgg aacgccggac 35580

gtagtcatat ttcctgaagc aaaaccaggt gcgggcgtga caaacagatc tgcgtctccg 35640

gtctcgccgc ttagatcgct ctgtgtagta gttgtagtat atccactctc tcaaagcatc 35700

caggcgcccc ctggcttcgg gttctatgta aactccttca tgcgccgctg ccctgataac 35760

atccaccacc gcagaataag ccacacccag ccaacctaca cattcgttct gcgagtcaca 35820

cacgggagga gcgggaagag ctggaagaac catgtttttt tttttattcc aaaagattat 35880

ccaaaacctc aaaatgaaga tctattaagt gaacgcgctc ccctccggtg gcgtggtcaa 35940

actctacagc caaagaacag ataatggcat ttgtaagatg ttgcacaatg gcttccaaaa 36000

ggcaaacggc cctcacgtcc aagtggacgt aaaggctaaa cccttcaggg tgaatctcct 36060

ctataaacat tccagcacct tcaaccatgc ccaaataatt ctcatctcgc caccttctca 36120

atatatctct aagcaaatcc cgaatattaa gtccggccat tgtaaaaatc tgctccagag 36180

cgccctccac cttcagcctc aagcagcgaa tcatgattgc aaaaattcag gttcctcaca 36240

gacctgtata agattcaaaa gcggaacatt aacaaaaata ccgcgatccc gtaggtccct 36300

tcgcagggcc agctgaacat aatcgtgcag gtctgcacgg accagcgcgg ccacttcccc 36360

gccaggaacc atgacaaaag aacccacact gattatgaca cgcatactcg gagctatgct 36420

aaccagcgta gccccgatgt aagcttgttg catgggcggc gatataaaat gcaaggtgct 36480

gctcaaaaaa tcaggcaaag cctcgcgcaa aaaagaaagc acatcgtagt catgctcatg 36540

cagataaagg caggtaagct ccggaaccac cacagaaaaa gacaccattt ttctctcaaa 36600

catgtctgcg ggtttctgca taaacacaaa ataaaataac aaaaaaacat ttaaacatta 36660

gaagcctgtc ttacaacagg aaaaacaacc cttataagca taagacggac tacggccatg 36720

ccggcgtgac cgtaaaaaaa ctggtcaccg tgattaaaaa gcaccaccga cagctcctcg 36780

gtcatgtccg gagtcataat gtaagactcg gtaaacacat caggttgatt cacatcggtc 36840

agtgctaaaa agcgaccgaa atagcccggg ggaatacata cccgcaggcg tagagacaac 36900

attacagccc ccataggagg tataacaaaa ttaataggag agaaaaacac ataaacacct 36960

gaaaaaccct cctgcctagg caaaatagca ccctcccgct ccagaacaac atacagcgct 37020

tccacagcgg cagccataac agtcagcctt accagtaaaa aagaaaacct attaaaaaaa 37080

caccactcga cacggcacca gctcaatcag tcacagtgta aaaaagggcc aagtgcagag 37140

cgagtatata taggactaaa aaatgacgta acggttaaag tccacaaaaa acacccagaa 37200

aaccgcacgc gaacctacgc ccagaaacga aagccaaaaa acccacaact tcctcaaatc 37260

gtcacttccg ttttcccacg ttacgtcact tcccatttta agaaaactac aattcccaac 37320

acatacaagt tactccgccc taaaacctac gtcacccgcc ccgttcccac gccccgcgcc 37380

acgtcacaaa ctccaccccc tcattatcat attggcttca atccaaaata aggtatatta 37440

ttgatgatgt taat 37454

<210> 2
<211> 37705
<212> DNA
<213> Artificial Sequence

<220>
<223> D24-PADI1-P2A-TIMP2

<400> 2
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520

taccaacatg ccaaatgtga acgagttcat gtttaccaat aagtttaagg cgcgggtgat 14580

ggtgtcgcgc ttgcctacta aggacaatca ggtggagctg aaatacgagt gggtggagtt 14640

cacgctgccc gagggcaact actccgagac catgaccata gaccttatga acaacgcgat 14700

cgtggagcac tacttgaaag tgggcagaca gaacggggtt ctggaaagcg acatcggggt 14760

aaagtttgac acccgcaact tcagactggg gtttgacccc gtcactggtc ttgtcatgcc 14820

tggggtatat acaaacgaag ccttccatcc agacatcatt ttgctgccag gatgcggggt 14880

ggacttcacc cacagccgcc tgagcaactt gttgggcatc cgcaagcggc aacccttcca 14940

ggagggcttt aggatcacct acgatgatct ggagggtggt aacattcccg cactgttgga 15000

tgtggacgcc taccaggcga gcttgaaaga tgacaccgaa cagggcgggg gtggcgcagg 15060

cggcagcaac agcagtggca gcggcgcgga agagaactcc aacgcggcag ccgcggcaat 15120

gcagccggtg gaggacatga acgatcatgc cattcgcggc gacacctttg ccacacgggc 15180

tgaggagaag cgcgctgagg ccgaagcagc ggccgaagct gccgcccccg ctgcgcaacc 15240

cgaggtcgag aagcctcaga agaaaccggt gatcaaaccc ctgacagagg acagcaagaa 15300

acgcagttac aacctaataa gcaatgacag caccttcacc cagtaccgca gctggtacct 15360

tgcatacaac tacggcgacc ctcagaccgg aatccgctca tggaccctgc tttgcactcc 15420

tgacgtaacc tgcggctcgg agcaggtcta ctggtcgttg ccagacatga tgcaagaccc 15480

cgtgaccttc cgctccacgc gccagatcag caactttccg gtggtgggcg ccgagctgtt 15540

gcccgtgcac tccaagagct tctacaacga ccaggccgtc tactcccaac tcatccgcca 15600

gtttacctct ctgacccacg tgttcaatcg ctttcccgag aaccagattt tggcgcgccc 15660

gccagccccc accatcacca ccgtcagtga aaacgttcct gctctcacag atcacgggac 15720

gctaccgctg cgcaacagca tcggaggagt ccagcgagtg accattactg acgccagacg 15780

ccgcacctgc ccctacgttt acaaggccct gggcatagtc tcgccgcgcg tcctatcgag 15840

ccgcactttt tgagcaagca tgtccatcct tatatcgccc agcaataaca caggctgggg 15900

cctgcgcttc ccaagcaaga tgtttggcgg ggccaagaag cgctccgacc aacacccagt 15960

gcgcgtgcgc gggcactacc gcgcgccctg gggcgcgcac aaacgcggcc gcactgggcg 16020

caccaccgtc gatgacgcca tcgacgcggt ggtggaggag gcgcgcaact acacgcccac 16080

gccgccacca gtgtccacag tggacgcggc cattcagacc gtggtgcgcg gagcccggcg 16140

ctatgctaaa atgaagagac ggcggaggcg cgtagcacgt cgccaccgcc gccgacccgg 16200

cactgccgcc caacgcgcgg cggcggccct gcttaaccgc gcacgtcgca ccggccgacg 16260

ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660

gccggcaacg ccacaacata aagaagcaag caacatcaac aacagctgcc gccatgggct 21720

ccagtgagca ggaactgaaa gccattgtca aagatcttgg ttgtgggcca tattttttgg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800
aaagtcaaat tatcggtacc tttgagctgc agggtccctc gcctgacgaa aagtccgcgg 25860
ctccggggtt gaaactcact ccggggctgt ggacgtcggc ttaccttcgc aaatttgtac 25920
ctgaggacta ccacgcccac gagattaggt tctacgaaga ccaatcccgc ccgcctaatg 25980
cggagcttac cgcctgcgtc attacccagg gccacattct tggccaattg caagccatca 26040
acaaagcccg ccaagagttt ctgctacgaa agggacgggg ggtttacttg gacccccagt 26100
ccggcgagga gctcaaccca atccccccgc cgccgcagcc ctatcagcag cagccgcggg 26160
cccttgcttc ccaggatggc acccaaaaag aagctgcagc tgccgccgcc acccacggac 26220
gaggaggaat actgggacag tcaggcagag gaggttttgg acgaggagga ggaggacatg 26280
atggaagact gggagagcct agacgaggaa gcttccgagg tcgaagaggt gtcagacgaa 26340
acaccgtcac cctcggtcgc attcccctcg ccggcgcccc agaaatcggc aaccggttcc 26400
agcatggcta caacctccgc tcctcaggcg ccgccggcac tgcccgttcg ccgacccaac 26460
cgtagatggg acaccactgg aaccagggcc ggtaagtcca agcagccgcc gccgttagcc 26520
caagagcaac aacagcgcca aggctaccgc tcatggcgcg ggcacaagaa cgccatagtt 26580
gcttgcttgc aagactgtgg gggcaacatc tccttcgccc gccgctttct tctctaccat 26640
cacggcgtgg ccttcccccg taacatcctg cattactacc gtcatctcta cagcccatac 26700
tgcaccggcg gcagcggcag caacagcagc ggccacacag aagcaaaggc gaccggatag 26760
caagactctg acaaagccca agaaatccac agcggcggca gcagcaggag gaggagcgct 26820
gcgtctggcg cccaacgaac ccgtatcgac ccgcgagctt agaaacagga tttttcccac 26880
tctgtatgct atatttcaac agagcagggg ccaagaacaa gagctgaaaa taaaaaacag 26940
gtctctgcga tccctcaccc gcagctgcct gtatcacaaa agcgaagatc agcttcggcg 27000
cacgctggaa gacgcggagg ctctcttcag taaatactgc gcgctgactc ttaaggacta 27060
gtttcgcgcc ctttctcaaa tttaagcgcg aaaactacgt catctccagc ggccacaccc 27120
ggcgccagca cctgttgtca gcgccattat gagcaaggaa attcccacgc cctacatgtg 27180
gagttaccag ccacaaatgg gacttgcggc tggagctgcc caagactact caacccgaat 27240
aaactacatg agcgcgggac cccacatgat atcccgggtc aacggaatac gcgcccaccg 27300
aaaccgaatt ctcctggaac aggcggctat taccaccaca cctcgtaata accttaatcc 27360
ccgtagttgg cccgctgccc tggtgtacca ggaaagtccc gctcccacca ctgtggtact 27420
tcccagagac gcccaggccg aagttcagat gactaactca ggggcgcagc ttgcgggcgg 27480
ctttcgtcac agggtgcggt cgcccgggca gggtataact cacctgacaa tcagagggcg 27540
aggtattcag ctcaacgacg agtcggtgag ctcctcgctt ggtctccgtc cggacgggac 27600
atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tctttgttgc catctctgtg ctgagtataa 28140

taaatacaga aattaaaata tactggggct cctatcgcca tcctgtaaac gccaccgtct 28200

tcacccgccc aagcaaacca aggcgaacct tacctggtac ttttaacatc tctccctctg 28260

tgatttacaa cagtttcaac ccagacggag tgagtctacg agagaacctc tccgagctca 28320

gctactccat cagaaaaaac accaccctcc ttacctgccg ggaacgtacg atggccccaa 28380

agagagttgt gcagctgtcc ctgaagatgc ctacccatgc cgtgtgtgtg gtgggagtcg 28440

aggcacatgt ggacattcac agtgatgtgc ccaagggtgc caacagcttc agggtctctg 28500

gaagctccgg ggtggaggtc ttcatggtct acaaccgcac acgtgtgaaa gagcccatag 28560

gcaaggcccg ttggccgcta gacactgatg cagacatggt cgtatctgtg ggcacagcca 28620

gtaaggaatt aaaggacttc aaggtgaggg tctcctactt tggggagcag gaagaccaag 28680

ctctgggccg cagcgtgctt tacctcactg gcgtcgatat ttcccttgag gttgacacag 28740

gccgcacagg caaggtgaag aggagccaag gggacaagaa aacctggcgc tggggccctg 28800

agggctatgg ggctatcttg ctggtgaact gtgaccggga caatcacagg tccgcggagc 28860

ctgacctcac ccacagctgg ctgatgtcgc tggctgacct gcaggacatg tccccaatgc 28920

tgctgagctg caatggcccc gacaagctct tcgacagcca caagcttgtc ttgaacgtgc 28980

ccttttctga ttccaaaaga gtgagggtct tctgtgccag gggtgggaat tctctctcgg 29040

actacaaaca ggtgctgggg ccccagtgtc tgtcctatga agttgagcga cagccagggg 29100

agcaggagat caagttctat gtggaggggc tgaccttccc cgatgccgat ttcctagggc 29160

tggtttccct cagtgtcagc ctggtggacc cggggaccct gcccgaggtg accctcttca 29220

cagacactgt gggcttccgc atggccccct ggatcatgac gcccaacact cagcctcctg 29280

aggagctgta tgtgtgcaga gtgatggaca ctcatggctc caatgagaaa ttcctggagg 29340

acatgtctta tctgacattg aaagccaact gcaagctgac catctgccct caagttgaaa 29400

atcgaaatga ccgctggatc caggacgaga tggagtttgg ctacatcgag gcccctcaca 29460

aatccttccc cgtggtcttt gactccccca ggaacagggg cctgaaagat ttcccctata 29520

agaggatcct gggtcctgac tttggatatg ttacccggga gatcccgctc cctggtccct 29580

ccagccttga ctccttcggc aacctggacg tcagcccgcc cgtcacgggg ggcggcacgg 29640

aataccccct gggccggatc ctcatcggga gcagcttccc caagtccggt gggcggcaga 29700

tggccagggc agtgcggaac ttcctgaagg cacagcaggt gcaggcaccc gtggagctct 29760

actcggactg gctctctgtg ggccatgtgg acgagtttct gacctttgtg cctacctctg 29820

accagaaggg cttccggctg ctcctggcta gccccagcgc ttgccccaaa ctcttccaag 29880

agaagaaaga agagggttat ggggaggcag cccagtttga tgggttaaaa caccaggcaa 29940

aaagaagcat taatgagatg ctggcagaca gacacctcca gagagacaat cttcatgcac 30000

agaaatgcat tgactggaac cgtaatgtgc tgaagcggga gctgggcctg gcagagagtg 30060

acatcgtgga cattccccag ctcttcttcc tgaaaaactt ctacgcggaa gccttcttcc 30120

cagacatggt taacatggtg gtcttaggca agtacctggg catccccaag ccctacgggc 30180

ccatcatcaa tggccgctgc tgcctggagg agaaggtgca gtccctgctg gagcctctgg 30240

gcctgcactg catcttcatt gatgactact tgtcctacca cgagctgcag ggggagatcc 30300

actgtggcac caacgtgcgc aggaagccct ttcccttcaa atggtggaac atggtgcccg 30360

gcagcggcgc cactaacttc tccctgttaa agcaagcagg cgatgttgaa gaaaaccccg 30420

ggcctatggg cgccgcggcc cgcaccctgc ggctggcgct cggcctcctg ctgctggcga 30480

cgctgcttcg cccggccgac gcctgcagct gctccccggt gcacccgcaa caggcgtttt 30540

gcaatgcaga tgtagtgatc agggccaaag cggtcagtga gaaggaagtg gactctggaa 30600

acgacattta tggcaaccct atcaagagga tccagtatga gatcaagcag ataaagatgt 30660

tcaaagggcc tgagaaggat atagagttta tctacacggc cccctcctcg gcagtgtgtg 30720

gggtctcgct ggacgttgga ggaaagaagg aatatctcat tgcaggaaag gccgaggggg 30780

acggcaagat gcacatcacc ctctgtgact tcatcgtgcc ctgggacacc ctgagcacca 30840

cccagaagaa gagcctgaac cacaggtacc agatgggctg cgagtgcaag atcacgcgct 30900

gccccatgat cccgtgctac atctcctccc cggacgagtg cctctggatg gactgggtca 30960

cagagaagaa catcaacggg caccaggcca agttcttcgc ctgcatcaag agaagtgacg 31020

gctcctgtgc gtggtaccgc ggcgcggcgc cccccaagca ggagtttctc gacatcgagg 31080

acccataaca attgactcta tgtgggatat gctccagcgc tacaaccttg aagtcaggct 31140

tcctggatgt cagcatctga ctttggccag cacctgtccc gcggatttgt tccagtccaa 31200

ctacagcgac ccaccctaac agagatgacc aacacaacca acgcggccgc cgctaccgga 31260

cttacatcta ccacaaatac accccaagtt tctgcctttg tcaataactg ggataacttg 31320

ggcatgtggt ggttctccat agcgcttatg tttgtatgcc ttattattat gtggctcatc 31380

tgctgcctaa agcgcaaacg cgcccgacca cccatctata gtcccatcat tgtgctacac 31440

ccaaacaatg atggaatcca tagattggac ggactgaaac acatgttctt ttctcttaca 31500

gtatgattaa atgagacatg attcctcgag tttttatatt actgaccctt gttgcgcttt 31560

tttgtgcgtg ctccacattg gctgcggttt ctcacatcga agtagactgc attccagcct 31620

tcacagtcta tttgctttac ggatttgtca ccctcacgct catctgcagc ctcatcactg 31680

tggtcatcgc ctttatccag tgcattgact gggtctgtgt gcgctttgca tatctcagac 31740

accatcccca gtacagggac aggactatag ctgagcttct tagaattctt taattatgaa 31800

atttactgtg acttttctgc tgattatttg caccctatct gcgttttgtt ccccgacctc 31860

caagcctcaa agacatatat catgcagatt cactcgtata tggaatattc caagttgcta 31920

caatgaaaaa agcgatcttt ccgaagcctg gttatatgca atcatctctg ttatggtgtt 31980

ctgcagtacc atcttagccc tagctatata tccctacctt gacattggct ggaacgcaat 32040

agatgccatg aaccacccaa ctttccccgc gcccgctatg cttccactgc aacaagttgt 32100

tgccggcggc tttgtcccag ccaatcagcc tcgcccacct tctcccaccc ccactgaaat 32160

cagctacttt aatctaacag gaggagatga ctgacaccct agatctagaa atggacggaa 32220

ttattacaga gcagcgcctg ctagaaagac gcagggcagc ggccgagcaa cagcgcatga 32280

atcaagagct ccaagacatg gttaacttgc accagtgcaa aaggggtatc ttttgtctcg 32340

taaagcaggc caaagtcacc tacgacagta ataccaccgg acaccgcctt agctacaagt 32400

tgccaaccaa gcgtcagaaa ttggtggtca tggtgggaga aaagcccatt accataactc 32460

agcactcggt agaaaccgaa ggctgcattc actcaccttg tcaaggacct gaggatctct 32520

gcacccttat taagaccctg tgcggtctca aagatcttat tccctttaac taataaaaaa 32580

aaataataaa gcatcactta cttaaaatca gttagcaaat ttctgtccag tttattcagc 32640

agcacctcct tgccctcctc ccagctctgg tattgcagct tcctcctggc tgcaaacttt 32700

ctccacaatc taaatggaat gtcagtttcc tcctgttcct gtccatccgc acccactatc 32760

ttcatgttgt tgcagatgaa gcgcgcaaga ccgtctgaag ataccttcaa ccccgtgtat 32820

ccatatgaca cggaaaccgg tcctccaact gtgccttttc ttactcctcc ctttgtatcc 32880

cccaatgggt ttcaagagag tccccctggg gtactctctt tgcgcctatc cgaacctcta 32940

gttacctcca atggcatgct tgcgctcaaa atgggcaacg gcctctctct ggacgaggcc 33000

ggcaacctta cctcccaaaa tgtaaccact gtgagcccac ctctcaaaaa aaccaagtca 33060

aacataaacc tggaaatatc tgcacccctc acagttacct cagaagccct aactgtggct 33120

gccgccgcac ctctaatggt cgcgggcaac acactcacca tgcaatcaca ggccccgcta 33180

accgtgcacg actccaaact tagcattgcc acccaaggac ccctcacagt gtcagaagga 33240

aagctagccc tgcaaacatc aggccccctc accaccaccg atagcagtac ccttactatc 33300

actgcctcac cccctctaac tactgccact ggtagcttgg gcattgactt gaaagagccc 33360
atttatacac aaaatggaaa actaggacta aagtacgggg ctcctttgca tgtaacagac 33420
gacctaaaca ctttgaccgt agcaactggt ccaggtgtga ctattaataa tacttccttg 33480
caaactaaag ttactggagc cttgggtttt gattcacaag gcaatatgca acttaatgta 33540
gcaggaggac taaggattga ttctcaaaac agacgcctta tacttgatgt tagttatccg 33600
tttgatgctc aaaaccaact aaatctaaga ctaggacagg gccctctttt tataaactca 33660
gcccacaact tggatattaa ctacaacaaa ggcctttact tgtttacagc ttcaaacaat 33720
tccaaaaagc ttgaggttaa cctaagcact gccaaggggt tgatgtttga cgctacagcc 33780
atagccatta atgcaggaga tgggcttgaa tttggttcac ctaatgcacc aaacacaaat 33840
cccctcaaaa caaaaattgg ccatggccta gaatttgatt caaacaaggc tatggttcct 33900
aaactaggaa ctggccttag ttttgacagc acaggtgcca ttacagtagg aaacaaaaat 33960
aatgataagc taaccctatg gacaggtcca aaaccagaag ccaactgcat aattgaatac 34020
gggaaacaaa acccagatag caaactaact ttaatccttg taaaaaatgg aggaattgtt 34080
aatggatatg taacgctaat gggagcctca gactacgtta acaccttatt taaaaacaaa 34140
aatgtctcca ttaatgtaga actatacttt gatgccactg gtcatatatt accagactca 34200
tcttctctta aaacagatct agaactaaaa tacaagcaaa ccgctgactt tagtgcaaga 34260
ggttttatgc caagtactac agcgtatcca tttgtccttc ctaatgcggg aacacataat 34320
gaaaattata tttttggtca atgctactac aaagcaagcg atggtgccct ttttccgttg 34380
gaagttactg ttatgcttaa taaacgcctg ccagatagtc gcacatccta tgttatgact 34440
tttttatggt ccttgaatgc tggtctagct ccagaaacta ctcaggcaac cctcataacc 34500
tccccattta ccttttccta tattagagaa gatgactaat aaactctaaa gaatcgtttg 34560
tgttatgttt caacgtgttt atttttcaat tgcagaaaat ttcaagtcat ttttcattca 34620
gtagtatagc cccaccacca catagcttat acagatcacc gtaccttaat caaactcaca 34680
gaaccctagt attcaacctg ccacctccct cccaacacac agagtacaca gtcctttctc 34740
cccggctggc cttaaaaagc atcatatcat gggtaacaga catattctta ggtgttatat 34800
tccacacggt ttcctgtcga gccaaacgct catcagtgat attaataaac tccccgggca 34860
gctcacttaa gttcatgtcg ctgtccagct gctgagccac aggctgctgt ccaacttgcg 34920
gttgcttaac gggcggcgaa ggagaagtcc acgcctacat gggggtagag tcataatcgt 34980
gcatcaggat agggcggtgg tgctgcagca gcgcgcgaat aaactgctgc cgccgccgct 35040
ccgtcctgca ggaatacaac atggcagtgg tctcctcagc gatgattcgc accgcccgca 35100
gcataaggcg ccttgtcctc cgggcacagc agcgcaccct gatctcactt aaatcagcac 35160
agtaactgca gcacagcacc acaatattgt tcaaaatccc acagtgcaag gcgctgtatc 35220

caaagctcat ggcggggacc acagaaccca cgtggccatc ataccacaag cgcaggtaga 35280

ttaagtggcg acccctcata aacacgctgg acataaacat tacctctttt ggcatgttgt 35340

aattcaccac ctcccggtac catataaacc tctgattaaa catggcgcca tccaccacca 35400

tcctaaacca gctggccaaa acctgcccgc cggctataca ctgcagggaa ccgggactgg 35460

aacaatgaca gtggagagcc caggactcgt aaccatggat catcatgctc gtcatgatat 35520

caatgttggc acaacacagg cacacgtgca tacacttcct caggattaca agctcctccc 35580

gcgttagaac catatcccag ggaacaaccc attcctgaat cagcgtaaat cccacactgc 35640

agggaagacc tcgcacgtaa ctcacgttgt gcattgtcaa agtgttacat tcgggcagca 35700

gcggatgatc ctccagtatg gtagcgcggg tttctgtctc aaaaggaggt agacgatccc 35760

tactgtacgg agtgcgccga gacaaccgag atcgtgttgg tcgtagtgtc atgccaaatg 35820

gaacgccgga cgtagtcata tttcctgaag caaaaccagg tgcgggcgtg acaaacagat 35880

ctgcgtctcc ggtctcgccg cttagatcgc tctgtgtagt agttgtagta tatccactct 35940

ctcaaagcat ccaggcgccc cctggcttcg ggttctatgt aaactccttc atgcgccgct 36000

gccctgataa catccaccac cgcagaataa gccacaccca gccaacctac acattcgttc 36060

tgcgagtcac acacgggagg agcgggaaga gctggaagaa ccatgttttt ttttttattc 36120

caaaagatta tccaaaacct caaaatgaag atctattaag tgaacgcgct cccctccggt 36180

ggcgtggtca aactctacag ccaaagaaca gataatggca tttgtaagat gttgcacaat 36240

ggcttccaaa aggcaaacgg ccctcacgtc caagtggacg taaaggctaa acccttcagg 36300

gtgaatctcc tctataaaca ttccagcacc ttcaaccatg cccaaataat tctcatctcg 36360

ccaccttctc aatatatctc taagcaaatc ccgaatatta agtccggcca ttgtaaaaat 36420

ctgctccaga gcgccctcca ccttcagcct caagcagcga atcatgattg caaaaattca 36480

ggttcctcac agacctgtat aagattcaaa agcggaacat taacaaaaat accgcgatcc 36540

cgtaggtccc ttcgcagggc cagctgaaca taatcgtgca ggtctgcacg gaccagcgcg 36600

gccacttccc cgccaggaac catgacaaaa gaacccacac tgattatgac acgcatactc 36660

ggagctatgc taaccagcgt agccccgatg taagcttgtt gcatgggcgg cgatataaaa 36720

tgcaaggtgc tgctcaaaaa atcaggcaaa gcctcgcgca aaaaagaaag cacatcgtag 36780

tcatgctcat gcagataaag gcaggtaagc tccggaacca ccacagaaaa agacaccatt 36840

tttctctcaa acatgtctgc gggtttctgc ataaacacaa aataaaataa caaaaaaaca 36900

tttaaacatt agaagcctgt cttacaacag gaaaaacaac ccttataagc ataagacgga 36960

ctacggccat gccggcgtga ccgtaaaaaa actggtcacc gtgattaaaa agcaccaccg 37020

acagctcctc ggtcatgtcc ggagtcataa tgtaagactc ggtaaacaca tcaggttgat 37080

tcacatcggt cagtgctaaa aagcgaccga aatagcccgg gggaatacat acccgcaggc 37140

gtagagacaa cattacagcc cccataggag gtataacaaa attaatagga gagaaaaaca 37200

cataaacacc tgaaaaaccc tcctgcctag gcaaaatagc accctcccgc tccagaacaa 37260

catacagcgc ttccacagcg gcagccataa cagtcagcct taccagtaaa aaagaaaacc 37320

tattaaaaaa acaccactcg acacggcacc agctcaatca gtcacagtgt aaaaaagggc 37380

caagtgcaga gcgagtatat ataggactaa aaaatgacgt aacggttaaa gtccacaaaa 37440

aacacccaga aaaccgcacg cgaacctacg cccagaaacg aaagccaaaa aacccacaac 37500

ttcctcaaat cgtcacttcc gttttcccac gttacgtcac ttcccatttt aagaaaacta 37560

caattcccaa cacatacaag ttactccgcc ctaaaaccta cgtcacccgc cccgttccca 37620

cgccccgcgc cacgtcacaa actccacccc ctcattatca tattggcttc aatccaaaat 37680

aaggtatatt attgatgatg ttaat 37705

<210> 3
<211> 36979
<212> DNA
<213> Artificial Sequence

<220>
<223> Ad5-D24-PADI1

<400> 3
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct ttggaccagc tgatcgaaga ggtactggct gataatcttc 660

cacctcctag ccatttgaac cacctaccct tcacgaactg tatgatttag acgtgacggc 720

ccccgaagat cccaacgagg aggcggtttc gcagattttt cccgactctg taatgttggc 780

ggtgcaggaa gggattgact tactcacttt tccgccggcg cccggttctc cggagccgcc 840

tcacctttcc cggcagcccg agcagccgga gcagagagcc ttgggtccgg tttctatgcc 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaagggttc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520

taccaacatg ccaaatgtga acgagttcat gtttaccaat aagtttaagg cgcgggtgat 14580

ggtgtcgcgc ttgcctacta aggacaatca ggtggagctg aaatacgagt gggtggagtt 14640

cacgctgccc gagggcaact actccgagac catgaccata gaccttatga acaacgcgat 14700

cgtggagcac tacttgaaag tgggcagaca gaacggggtt ctggaaagcg acatcggggt 14760

aaagtttgac acccgcaact tcagactggg gtttgacccc gtcactggtc ttgtcatgcc 14820

tggggtatat acaaacgaag ccttccatcc agacatcatt ttgctgccag gatgcggggt 14880

ggacttcacc cacagccgcc tgagcaactt gttgggcatc cgcaagcggc aacccttcca 14940

ggagggcttt aggatcacct acgatgatct ggagggtggt aacattcccg cactgttgga 15000

tgtggacgcc taccaggcga gcttgaaaga tgacaccgaa cagggcgggg gtggcgcagg 15060

cggcagcaac agcagtggca gcggcgcgga agagaactcc aacgcggcag ccgcggcaat 15120

gcagccggtg gaggacatga acgatcatgc cattcgcggc gacacctttg ccacacgggc 15180

tgaggagaag cgcgctgagg ccgaagcagc ggccgaagct gccgcccccg ctgcgcaacc 15240

cgaggtcgag aagcctcaga agaaaccggt gatcaaaccc ctgacagagg acagcaagaa 15300

acgcagttac aacctaataa gcaatgacag caccttcacc cagtaccgca gctggtacct 15360

tgcatacaac tacggcgacc ctcagaccgg aatccgctca tggaccctgc tttgcactcc 15420

tgacgtaacc tgcggctcgg agcaggtcta ctggtcgttg ccagacatga tgcaagaccc 15480

cgtgaccttc cgctccacgc gccagatcag caactttccg gtggtgggcg ccgagctgtt 15540

gcccgtgcac tccaagagct tctacaacga ccaggccgtc tactcccaac tcatccgcca 15600

gtttacctct ctgacccacg tgttcaatcg ctttcccgag aaccagattt tggcgcgccc 15660

gccagccccc accatcacca ccgtcagtga aaacgttcct gctctcacag atcacgggac 15720

gctaccgctg cgcaacagca tcggaggagt ccagcgagtg accattactg acgccagacg 15780

ccgcacctgc ccctacgttt acaaggccct gggcatagtc tcgccgcgcg tcctatcgag 15840

ccgcactttt tgagcaagca tgtccatcct tatatcgccc agcaataaca caggctgggg 15900

cctgcgcttc ccaagcaaga tgtttggcgg ggccaagaag cgctccgacc aacacccagt 15960

gcgcgtgcgc gggcactacc gcgcgccctg gggcgcgcac aaacgcggcc gcactgggcg 16020

caccaccgtc gatgacgcca tcgacgcggt ggtggaggag gcgcgcaact acacgcccac 16080

gccgccacca gtgtccacag tggacgcggc cattcagacc gtggtgcgcg gagcccggcg 16140

ctatgctaaa atgaagagac ggcggaggcg cgtagcacgt cgccaccgcc gccgacccgg 16200

cactgccgcc caacgcgcgg cggcggccct gcttaaccgc gcacgtcgca ccggccgacg 16260

ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860
gttctggcgg gccaagcatc gcagttgaat gctgttgtag atttgcaaga cagaaacaca 19920
gagctttcat accagctttt gcttgattcc attggtgata gaaccaggta cttttctatg 19980
tggaatcagg ctgttgacag ctatgatcca gatgttagaa ttattgaaaa tcatggaact 20040
gaagatgaac ttccaaatta ctgctttcca ctgggaggtg tgattaatac agagactctt 20100
accaaggtaa aacctaaaac aggtcaggaa aatggatggg aaaaagatgc tacagaattt 20160
tcagataaaa atgaaataag agttggaaat aattttgcca tggaaatcaa tctaaatgcc 20220
aacctgtgga gaaatttcct gtactccaac atagcgctgt atttgcccga caagctaaag 20280
tacagtcctt ccaacgtaaa aatttctgat aacccaaaca cctacgacta catgaacaag 20340
cgagtggtgg ctcccgggct agtggactgc tacattaacc ttggagcacg ctggtccctt 20400
gactatatgg acaacgtcaa cccatttaac caccaccgca atgctggcct gcgctaccgc 20460
tcaatgttgc tgggcaatgg tcgctatgtg cccttccaca tccaggtgcc tcagaagttc 20520
tttgccatta aaaacctcct tctcctgccg ggctcataca cctacgagtg gaacttcagg 20580
aaggatgtta acatggttct gcagagctcc ctaggaaatg acctaagggt tgacggagcc 20640
agcattaagt ttgatagcat ttgcctttac gccaccttct tccccatggc ccacaacacc 20700
gcctccacgc ttgaggccat gcttagaaac gacaccaacg accagtcctt taacgactat 20760
ctctccgccg ccaacatgct ctaccctata cccgccaacg ctaccaacgt gcccatatcc 20820
atcccctccc gcaactgggc ggctttccgc ggctgggcct tcacgcgcct taagactaag 20880
gaaaccccat cactgggctc gggctacgac ccttattaca cctactctgg ctctataccc 20940
tacctagatg gaacctttta cctcaaccac acctttaaga aggtggccat tacctttgac 21000
tcttctgtca gctggcctgg caatgaccgc ctgcttaccc ccaacgagtt tgaaattaag 21060
cgctcagttg acggggaggg ttacaacgtt gcccagtgta acatgaccaa agactggttc 21120
ctggtacaaa tgctagctaa ctataacatt ggctaccagg gcttctatat cccagagagc 21180
tacaaggacc gcatgtactc cttctttaga aacttccagc ccatgagccg tcaggtggtg 21240
gatgatacta aatacaagga ctaccaacag gtgggcatcc tacaccaaca caacaactct 21300
ggatttgttg gctaccttgc ccccaccatg cgcgaaggac aggcctaccc tgctaacttc 21360
ccctatccgc ttataggcaa gaccgcagtt gacagcatta cccagaaaaa gtttctttgc 21420
gatcgcaccc tttggcgcat cccattctcc agtaacttta tgtccatggg cgcactcaca 21480
gacctgggcc aaaaccttct ctacgccaac tccgcccacg cgctagacat gacttttgag 21540
gtggatccca tggacgagcc cacccttctt tatgttttgt ttgaagtctt tgacgtggtc 21600
cgtgtgcacc agccgcaccg cggcgtcatc gaaaccgtgt acctgcgcac gcccttctcg 21660
gccggcaacg ccacaacata aagaagcaag caacatcaac aacagctgcc gccatgggct 21720

ccagtgagca ggaactgaaa gccattgtca aagatcttgg ttgtgggcca tattttttgg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tctttgttgc catctctgtg ctgagtataa 28140

taaatacaga aattaaaata tactggggct cctatcgcca tcctgtaaac gccaccgtct 28200

tcacccgccc aagcaaacca aggcgaacct tacctggtac ttttaacatc tctccctctg 28260

tgatttacaa cagtttcaac ccagacggag tgagtctacg agagaacctc tccgagctca 28320

gctactccat cagaaaaaac accaccctcc ttacctgccg ggaacgtacg atggccccaa 28380

agagagttgt gcagctgtcc ctgaagatgc ctacccatgc cgtgtgtgtg gtgggagtcg 28440

aggcacatgt ggacattcac agtgatgtgc ccaagggtgc caacagcttc agggtctctg 28500

gaagctccgg ggtggaggtc ttcatggtct acaaccgcac acgtgtgaaa gagcccatag 28560

gcaaggcccg ttggccgcta gacactgatg cagacatggt cgtatctgtg ggcacagcca 28620

gtaaggaatt aaaggacttc aaggtgaggg tctcctactt tggggagcag gaagaccaag 28680

ctctgggccg cagcgtgctt tacctcactg gcgtcgatat ttcccttgag gttgacacag 28740

gccgcacagg caaggtgaag aggagccaag gggacaagaa aacctggcgc tggggccctg 28800

agggctatgg ggctatcttg ctggtgaact gtgaccggga caatcacagg tccgcggagc 28860

ctgacctcac ccacagctgg ctgatgtcgc tggctgacct gcaggacatg tccccaatgc 28920

tgctgagctg caatggcccc gacaagctct tcgacagcca caagcttgtc ttgaacgtgc 28980

ccttttctga ttccaaaaga gtgagggtct tctgtgccag gggtgggaat tctctctcgg 29040

actacaaaca ggtgctgggg ccccagtgtc tgtcctatga agttgagcga cagccagggg 29100

agcaggagat caagttctat gtggaggggc tgaccttccc cgatgccgat ttcctagggc 29160

tggtttccct cagtgtcagc ctggtggacc cggggaccct gcccgaggtg accctcttca 29220

cagacactgt gggcttccgc atggccccct ggatcatgac gcccaacact cagcctcctg 29280

aggagctgta tgtgtgcaga gtgatggaca ctcatggctc caatgagaaa ttcctggagg 29340

acatgtctta tctgacattg aaagccaact gcaagctgac catctgccct caagttgaaa 29400

atcgaaatga ccgctggatc caggacgaga tggagtttgg ctacatcgag gcccctcaca 29460

aatccttccc cgtggtcttt gactccccca ggaacagggg cctgaaagat ttcccctata 29520

agaggatcct gggtcctgac tttggatatg ttacccggga gatcccgctc cctggtccct 29580

ccagccttga ctccttcggc aacctggacg tcagcccgcc cgtcacgggg ggcggcacgg 29640

aataccccct gggccggatc ctcatcggga gcagcttccc caagtccggt gggcggcaga 29700

tggccagggc agtgcggaac ttcctgaagg cacagcaggt gcaggcaccc gtggagctct 29760

actcggactg gctctctgtg ggccatgtgg acgagtttct gacctttgtg cctacctctg 29820

accagaaggg cttccggctg ctcctggcta gccccagcgc ttgccccaaa ctcttccaag 29880

agaagaaaga agagggttat ggggaggcag cccagtttga tgggttaaaa caccaggcaa 29940

aaagaagcat taatgagatg ctggcagaca gacacctcca gagagacaat cttcatgcac 30000

agaaatgcat tgactggaac cgtaatgtgc tgaagcggga gctgggcctg gcagagagtg 30060

acatcgtgga cattccccag ctcttcttcc tgaaaaactt ctacgcggaa gccttcttcc 30120

cagacatggt taacatggtg gtcttaggca agtacctggg catccccaag ccctacgggc 30180

ccatcatcaa tggccgctgc tgcctggagg agaaggtgca gtccctgctg gagcctctgg 30240

gcctgcactg catcttcatt gatgactact tgtcctacca cgagctgcag ggggagatcc 30300

actgtggcac caacgtgcgc aggaagccct ttcccttcaa atggtggaac atggtgccct 30360

gacaattgac tctatgtggg atatgctcca gcgctacaac cttgaagtca ggcttcctgg 30420

atgtcagcat ctgactttgg ccagcacctg tcccgcggat ttgttccagt ccaactacag 30480

cgacccaccc taacagagat gaccaacaca accaacgcgg ccgccgctac cggacttaca 30540

tctaccacaa atacacccca agtttctgcc tttgtcaata actgggataa cttgggcatg 30600

tggtggttct ccatagcgct tatgtttgta tgccttatta ttatgtggct catctgctgc 30660

ctaaagcgca aacgcgcccg accacccatc tatagtccca tcattgtgct acacccaaac 30720

aatgatggaa tccatagatt ggacggactg aaacacatgt tcttttctct tacagtatga 30780

ttaaatgaga catgattcct cgagttttta tattactgac ccttgttgcg cttttttgtg 30840

cgtgctccac attggctgcg gtttctcaca tcgaagtaga ctgcattcca gccttcacag 30900

tctatttgct ttacggattt gtcaccctca cgctcatctg cagcctcatc actgtggtca 30960

tcgcctttat ccagtgcatt gactgggtct gtgtgcgctt tgcatatctc agacaccatc 31020

cccagtacag ggacaggact atagctgagc ttcttagaat tctttaatta tgaaatttac 31080

tgtgactttt ctgctgatta tttgcaccct atctgcgttt tgttccccga cctccaagcc 31140

tcaaagacat atatcatgca gattcactcg tatatggaat attccaagtt gctacaatga 31200

aaaaagcgat ctttccgaag cctggttata tgcaatcatc tctgttatgg tgttctgcag 31260

taccatctta gccctagcta tatatcccta ccttgacatt ggctggaacg caatagatgc 31320

catgaaccac ccaactttcc ccgcgcccgc tatgcttcca ctgcaacaag ttgttgccgg 31380

cggctttgtc ccagccaatc agcctcgccc accttctccc acccccactg aaatcagcta 31440

ctttaatcta acaggaggag atgactgaca ccctagatct agaaatggac ggaattatta 31500

cagagcagcg cctgctagaa agacgcaggg cagcggccga gcaacagcgc atgaatcaag 31560

agctccaaga catggttaac ttgcaccagt gcaaaagggg tatcttttgt ctcgtaaagc 31620

aggccaaagt cacctacgac agtaatacca ccggacaccg ccttagctac aagttgccaa 31680

ccaagcgtca gaaattggtg gtcatggtgg gagaaaagcc cattaccata actcagcact 31740

cggtagaaac cgaaggctgc attcactcac cttgtcaagg acctgaggat ctctgcaccc 31800

ttattaagac cctgtgcggt ctcaaagatc ttattccctt taactaataa aaaaaaataa 31860

taaagcatca cttacttaaa atcagttagc aaatttctgt ccagtttatt cagcagcacc 31920

tccttgccct cctcccagct ctggtattgc agcttcctcc tggctgcaaa ctttctccac 31980

aatctaaatg gaatgtcagt ttcctcctgt tcctgtccat ccgcacccac tatcttcatg 32040

ttgttgcaga tgaagcgcgc aagaccgtct gaagatacct tcaaccccgt gtatccatat 32100

gacacggaaa ccggtcctcc aactgtgcct tttcttactc ctccctttgt atcccccaat 32160

gggtttcaag agagtccccc tggggtactc tctttgcgcc tatccgaacc tctagttacc 32220

tccaatggca tgcttgcgct caaaatgggc aacggcctct ctctggacga ggccggcaac 32280

cttacctccc aaaatgtaac cactgtgagc ccacctctca aaaaaaccaa gtcaaacata 32340

aacctggaaa tatctgcacc cctcacagtt acctcagaag ccctaactgt ggctgccgcc 32400

gcacctctaa tggtcgcggg caacacactc accatgcaat cacaggcccc gctaaccgtg 32460

cacgactcca aacttagcat tgccacccaa ggacccctca cagtgtcaga aggaaagcta 32520

gccctgcaaa catcaggccc cctcaccacc accgatagca gtacccttac tatcactgcc 32580

tcaccccctc taactactgc cactggtagc ttgggcattg acttgaaaga gcccatttat 32640

acacaaaatg gaaaactagg actaaagtac ggggctcctt tgcatgtaac agacgaccta 32700

aacactttga ccgtagcaac tggtccaggt gtgactatta ataatacttc cttgcaaact 32760

aaagttactg gagccttggg ttttgattca caaggcaata tgcaacttaa tgtagcagga 32820

ggactaagga ttgattctca aaacagacgc cttatacttg atgttagtta tccgtttgat 32880

gctcaaaacc aactaaatct aagactagga cagggccctc tttttataaa ctcagcccac 32940

aacttggata ttaactacaa caaaggcctt tacttgttta cagcttcaaa caattccaaa 33000

aagcttgagg ttaacctaag cactgccaag gggttgatgt ttgacgctac agccatagcc 33060

attaatgcag gagatgggct tgaatttggt tcacctaatg caccaaacac aaatcccctc 33120

aaaacaaaaa ttggccatgg cctagaattt gattcaaaca aggctatggt tcctaaacta 33180

ggaactggcc ttagttttga cagcacaggt gccattacag taggaaacaa aaataatgat 33240

aagctaaccc tatggacagg tccaaaacca gaagccaact gcataattga atacgggaaa 33300

caaaacccag atagcaaact aactttaatc cttgtaaaaa atggaggaat tgttaatgga 33360

tatgtaacgc taatgggagc ctcagactac gttaacacct tatttaaaaa caaaaatgtc 33420

tccattaatg tagaactata ctttgatgcc actggtcata tattaccaga ctcatcttct 33480

cttaaaacag atctagaact aaaatacaag caaaccgctg actttagtgc aagaggtttt 33540

atgccaagta ctacagcgta tccatttgtc cttcctaatg cgggaacaca taatgaaaat 33600

tatatttttg gtcaatgcta ctacaaagca agcgatggtg ccctttttcc gttggaagtt 33660

actgttatgc ttaataaacg cctgccagat agtcgcacat cctatgttat gactttttta 33720

tggtccttga atgctggtct agctccagaa actactcagg caaccctcat aacctcccca 33780

tttacctttt cctatattag agaagatgac taataaactc taaagaatcg tttgtgttat 33840

gtttcaacgt gtttattttt caattgcaga aaatttcaag tcatttttca ttcagtagta 33900

tagccccacc accacatagc ttatacagat caccgtacct taatcaaact cacagaaccc 33960

tagtattcaa cctgccacct ccctcccaac acacagagta cacagtcctt tctccccggc 34020

tggccttaaa aagcatcata tcatgggtaa cagacatatt cttaggtgtt atattccaca 34080

cggtttcctg tcgagccaaa cgctcatcag tgatattaat aaactccccg ggcagctcac 34140

ttaagttcat gtcgctgtcc agctgctgag ccacaggctg ctgtccaact tgcggttgct 34200

taacgggcgg cgaaggagaa gtccacgcct acatgggggt agagtcataa tcgtgcatca 34260

ggatagggcg gtggtgctgc agcagcgcgc gaataaactg ctgccgccgc cgctccgtcc 34320

tgcaggaata caacatggca gtggtctcct cagcgatgat tcgcaccgcc cgcagcataa 34380

ggcgccttgt cctccgggca cagcagcgca ccctgatctc acttaaatca gcacagtaac 34440

tgcagcacag caccacaata ttgttcaaaa tcccacagtg caaggcgctg tatccaaagc 34500

tcatggcggg gaccacagaa cccacgtggc catcatacca caagcgcagg tagattaagt 34560

ggcgacccct cataaacacg ctggacataa acattacctc ttttggcatg ttgtaattca 34620

ccacctcccg gtaccatata aacctctgat taaacatggc gccatccacc accatcctaa 34680

accagctggc caaaacctgc ccgccggcta tacactgcag ggaaccggga ctggaacaat 34740

gacagtggag agcccaggac tcgtaaccat ggatcatcat gctcgtcatg atatcaatgt 34800

tggcacaaca caggcacacg tgcatacact tcctcaggat tacaagctcc tcccgcgtta 34860

gaaccatatc ccagggaaca acccattcct gaatcagcgt aaatcccaca ctgcagggaa 34920

gacctcgcac gtaactcacg ttgtgcattg tcaaagtgtt acattcgggc agcagcggat 34980

gatcctccag tatggtagcg cgggtttctg tctcaaaagg aggtagacga tccctactgt 35040

acggagtgcg ccgagacaac cgagatcgtg ttggtcgtag tgtcatgcca aatggaacgc 35100

cggacgtagt catatttcct gaagcaaaac caggtgcggg cgtgacaaac agatctgcgt 35160

ctccggtctc gccgcttaga tcgctctgtg tagtagttgt agtatatcca ctctctcaaa 35220

gcatccaggc gccccctggc ttcgggttct atgtaaactc cttcatgcgc cgctgccctg 35280

ataacatcca ccaccgcaga ataagccaca cccagccaac ctacacattc gttctgcgag 35340

tcacacacgg gaggagcggg aagagctgga agaaccatgt tttttttttt attccaaaag 35400

attatccaaa acctcaaaat gaagatctat taagtgaacg cgctcccctc cggtggcgtg 35460

gtcaaactct acagccaaag aacagataat ggcatttgta agatgttgca caatggcttc 35520

caaaaggcaa acggccctca cgtccaagtg gacgtaaagg ctaaaccctt cagggtgaat 35580

ctcctctata aacattccag caccttcaac catgcccaaa taattctcat ctcgccacct 35640

tctcaatata tctctaagca aatcccgaat attaagtccg gccattgtaa aaatctgctc 35700

cagagcgccc tccaccttca gcctcaagca gcgaatcatg attgcaaaaa ttcaggttcc 35760

tcacagacct gtataagatt caaaagcgga acattaacaa aaataccgcg atcccgtagg 35820

tcccttcgca gggccagctg aacataatcg tgcaggtctg cacggaccag cgcggccact 35880

tccccgccag gaaccatgac aaaagaaccc acactgatta tgacacgcat actcggagct 35940

atgctaacca gcgtagcccc gatgtaagct tgttgcatgg gcggcgatat aaaatgcaag 36000

gtgctgctca aaaaatcagg caaagcctcg cgcaaaaaag aaagcacatc gtagtcatgc 36060

tcatgcagat aaaggcaggt aagctccgga accaccacag aaaaagacac catttttctc 36120

tcaaacatgt ctgcgggttt ctgcataaac acaaaataaa ataacaaaaa aacatttaaa 36180

cattagaagc ctgtcttaca acaggaaaaa caacccttat aagcataaga cggactacgg 36240

ccatgccggc gtgaccgtaa aaaaactggt caccgtgatt aaaaagcacc accgacagct 36300

cctcggtcat gtccggagtc ataatgtaag actcggtaaa cacatcaggt tgattcacat 36360

cggtcagtgc taaaaagcga ccgaaatagc ccgggggaat acatacccgc aggcgtagag 36420

acaacattac agcccccata ggaggtataa caaaattaat aggagagaaa aacacataaa 36480

cacctgaaaa accctcctgc ctaggcaaaa tagcaccctc ccgctccaga acaacataca 36540

gcgcttccac agcggcagcc ataacagtca gccttaccag taaaaaagaa aacctattaa 36600

aaaaacacca ctcgacacgg caccagctca atcagtcaca gtgtaaaaaa gggccaagtg 36660

cagagcgagt atatatagga ctaaaaaatg acgtaacggt taaagtccac aaaaaacacc 36720

cagaaaaccg cacgcgaacc tacgcccaga aacgaaagcc aaaaaaccca caacttcctc 36780

aaatcgtcac ttccgttttc ccacgttacg tcacttccca ttttaagaaa actacaattc 36840

ccaacacata caagttactc cgccctaaaa cctacgtcac ccgccccgtt cccacgcccc 36900

gcgccacgtc acaaactcca ccccctcatt atcatattgg cttcaatcca aaataaggta 36960

tattattgat gatgttaat 36979

<210> 4
<211> 35650
<212> DNA
<213> Artificial Sequence

<220>
<223> Ad5-D24-TIMP2

<400> 4
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcg gggctgtgcc ttttactgct tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520

taccaacatg ccaaatgtga acgagttcat gtttaccaat aagtttaagg cgcgggtgat 14580

ggtgtcgcgc ttgcctacta aggacaatca ggtggagctg aaatacgagt gggtggagtt 14640

cacgctgccc gagggcaact actccgagac catgaccata gaccttatga acaacgcgat 14700

cgtggagcac tacttgaaag tgggcagaca gaacggggtt ctggaaagcg acatcggggt 14760

aaagtttgac acccgcaact tcagactggg gtttgacccc gtcactggtc ttgtcatgcc 14820

tggggtatat acaaacgaag ccttccatcc agacatcatt ttgctgccag gatgcggggt 14880

ggacttcacc cacagccgcc tgagcaactt gttgggcatc cgcaagcggc aacccttcca 14940

ggagggcttt aggatcacct acgatgatct ggagggtggt aacattcccg cactgttgga 15000

tgtggacgcc taccaggcga gcttgaaaga tgacaccgaa cagggcgggg gtggcgcagg 15060

cggcagcaac agcagtggca gcggcgcgga agagaactcc aacgcggcag ccgcggcaat 15120

gcagccggtg gaggacatga acgatcatgc cattcgcggc gacacctttg ccacacgggc 15180

tgaggagaag cgcgctgagg ccgaagcagc ggccgaagct gccgcccccg ctgcgcaacc 15240

cgaggtcgag aagcctcaga agaaaccggt gatcaaaccc ctgacagagg acagcaagaa 15300

acgcagttac aacctaataa gcaatgacag caccttcacc cagtaccgca gctggtacct 15360

tgcatacaac tacggcgacc ctcagaccgg aatccgctca tggaccctgc tttgcactcc 15420

tgacgtaacc tgcggctcgg agcaggtcta ctggtcgttg ccagacatga tgcaagaccc 15480

cgtgaccttc cgctccacgc gccagatcag caactttccg gtggtgggcg ccgagctgtt 15540

gcccgtgcac tccaagagct tctacaacga ccaggccgtc tactcccaac tcatccgcca 15600

gtttacctct ctgacccacg tgttcaatcg ctttcccgag aaccagattt tggcgcgccc 15660

gccagccccc accatcacca ccgtcagtga aaacgttcct gctctcacag atcacgggac 15720

gctaccgctg cgcaacagca tcggaggagt ccagcgagtg accattactg acgccagacg 15780

ccgcacctgc ccctacgttt acaaggccct gggcatagtc tcgccgcgcg tcctatcgag 15840

ccgcactttt tgagcaagca tgtccatcct tatatcgccc agcaataaca caggctgggg 15900

cctgcgcttc ccaagcaaga tgtttggcgg ggccaagaag cgctccgacc aacacccagt 15960

gcgcgtgcgc gggcactacc gcgcgccctg gggcgcgcac aaacgcggcc gcactgggcg 16020

caccaccgtc gatgacgcca tcgacgcggt ggtggaggag gcgcgcaact acacgcccac 16080

gccgccacca gtgtccacag tggacgcggc cattcagacc gtggtgcgcg gagcccggcg 16140

ctatgctaaa atgaagagac ggcggaggcg cgtagcacgt cgccaccgcc gccgacccgg 16200

cactgccgcc caacgcgcgg cggcggccct gcttaaccgc gcacgtcgca ccggccgacg 16260

ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660

gccggcaacg ccacaacata aagaagcaag caacatcaac aacagctgcc gccatgggct 21720

ccagtgagca ggaactgaaa gccattgtca aagatcttgg ttgtgggcca tattttttgg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tctttgttgc catctctgtg ctgagtataa 28140

taaatacaga aattaaaata tactggggct cctatcgcca tcctgtaaac gccaccgtct 28200

tcacccgccc aagcaaacca aggcgaacct tacctggtac ttttaacatc tctccctctg 28260

tgatttacaa cagtttcaac ccagacggag tgagtctacg agagaacctc tccgagctca 28320

gctactccat cagaaaaaac accaccctcc ttacctgccg ggaacgtacg atgggcgccg 28380

cggcccgcac cctgcggctg gcgctcggcc tcctgctgct ggcgacgctg cttcgcccgg 28440

ccgacgcctg cagctgctcc ccggtgcacc cgcaacaggc gttttgcaat gcagatgtag 28500

tgatcagggc caaagcggtc agtgagaagg aagtggactc tggaaacgac atttatggca 28560

accctatcaa gaggatccag tatgagatca agcagataaa gatgttcaaa gggcctgaga 28620

aggatataga gtttatctac acggccccct cctcggcagt gtgtggggtc tcgctggacg 28680

ttggaggaaa gaaggaatat ctcattgcag gaaaggccga gggggacggc aagatgcaca 28740

tcaccctctg tgacttcatc gtgccctggg acaccctgag caccacccag aagaagagcc 28800

tgaaccacag gtaccagatg ggctgcgagt gcaagatcac gcgctgcccc atgatcccgt 28860

gctacatctc ctccccggac gagtgcctct ggatggactg ggtcacagag aagaacatca 28920

acgggcacca ggccaagttc ttcgcctgca tcaagagaag tgacggctcc tgtgcgtggt 28980

accgcggcgc ggcgcccccc aagcaggagt ttctcgacat cgaggaccca taacaattga 29040

ctctatgtgg gatatgctcc agcgctacaa ccttgaagtc aggcttcctg gatgtcagca 29100

tctgactttg gccagcacct gtcccgcgga tttgttccag tccaactaca gcgacccacc 29160

ctaacagaga tgaccaacac aaccaacgcg gccgccgcta ccggacttac atctaccaca 29220

aatacacccc aagtttctgc ctttgtcaat aactgggata acttgggcat gtggtggttc 29280

tccatagcgc ttatgtttgt atgccttatt attatgtggc tcatctgctg cctaaagcgc 29340

aaacgcgccc gaccacccat ctatagtccc atcattgtgc tacacccaaa caatgatgga 29400

atccatagat tggacggact gaaacacatg ttcttttctc ttacagtatg attaaatgag 29460

acatgattcc tcgagttttt atattactga cccttgttgc gcttttttgt gcgtgctcca 29520

cattggctgc ggtttctcac atcgaagtag actgcattcc agccttcaca gtctatttgc 29580

tttacggatt tgtcaccctc acgctcatct gcagcctcat cactgtggtc atcgccttta 29640

tccagtgcat tgactgggtc tgtgtgcgct ttgcatatct cagacaccat ccccagtaca 29700

gggacaggac tatagctgag cttcttagaa ttctttaatt atgaaattta ctgtgacttt 29760

tctgctgatt atttgcaccc tatctgcgtt ttgttccccg acctccaagc ctcaaagaca 29820

tatatcatgc agattcactc gtatatggaa tattccaagt tgctacaatg aaaaaagcga 29880

tctttccgaa gcctggttat atgcaatcat ctctgttatg gtgttctgca gtaccatctt 29940

agccctagct atatatccct accttgacat tggctggaac gcaatagatg ccatgaacca 30000

cccaactttc cccgcgcccg ctatgcttcc actgcaacaa gttgttgccg gcggctttgt 30060

cccagccaat cagcctcgcc caccttctcc cacccccact gaaatcagct actttaatct 30120

aacaggagga gatgactgac accctagatc tagaaatgga cggaattatt acagagcagc 30180

gcctgctaga aagacgcagg gcagcggccg agcaacagcg catgaatcaa gagctccaag 30240

acatggttaa cttgcaccag tgcaaaaggg gtatcttttg tctcgtaaag caggccaaag 30300

tcacctacga cagtaatacc accggacacc gccttagcta caagttgcca accaagcgtc 30360

agaaattggt ggtcatggtg ggagaaaagc ccattaccat aactcagcac tcggtagaaa 30420

ccgaaggctg cattcactca ccttgtcaag gacctgagga tctctgcacc cttattaaga 30480

ccctgtgcgg tctcaaagat cttattccct ttaactaata aaaaaaaata ataaagcatc 30540

acttacttaa aatcagttag caaatttctg tccagtttat tcagcagcac ctccttgccc 30600

tcctcccagc tctggtattg cagcttcctc ctggctgcaa actttctcca caatctaaat 30660

ggaatgtcag tttcctcctg ttcctgtcca tccgcaccca ctatcttcat gttgttgcag 30720

atgaagcgcg caagaccgtc tgaagatacc ttcaaccccg tgtatccata tgacacggaa 30780

accggtcctc caactgtgcc ttttcttact cctccctttg tatcccccaa tgggtttcaa 30840

gagagtcccc ctggggtact ctctttgcgc ctatccgaac ctctagttac ctccaatggc 30900

atgcttgcgc tcaaaatggg caacggcctc tctctggacg aggccggcaa ccttacctcc 30960

caaaatgtaa ccactgtgag cccacctctc aaaaaaacca agtcaaacat aaacctggaa 31020

atatctgcac ccctcacagt tacctcagaa gccctaactg tggctgccgc cgcacctcta 31080

atggtcgcgg gcaacacact caccatgcaa tcacaggccc cgctaaccgt gcacgactcc 31140

aaacttagca ttgccaccca aggacccctc acagtgtcag aaggaaagct agccctgcaa 31200

acatcaggcc ccctcaccac caccgatagc agtaccctta ctatcactgc ctcaccccct 31260

ctaactactg ccactggtag cttgggcatt gacttgaaag agcccattta tacacaaaat 31320

ggaaaactag gactaaagta cggggctcct ttgcatgtaa cagacgacct aaacactttg 31380

accgtagcaa ctggtccagg tgtgactatt aataatactt ccttgcaaac taaagttact 31440

ggagccttgg gttttgattc acaaggcaat atgcaactta atgtagcagg aggactaagg 31500

attgattctc aaaacagacg ccttatactt gatgttagtt atccgtttga tgctcaaaac 31560

caactaaatc taagactagg acagggccct ctttttataa actcagccca caacttggat 31620

attaactaca acaaaggcct ttacttgttt acagcttcaa acaattccaa aaagcttgag 31680

gttaacctaa gcactgccaa ggggttgatg tttgacgcta cagccatagc cattaatgca 31740

ggagatgggc ttgaatttgg ttcacctaat gcaccaaaca caaatcccct caaaacaaaa 31800

attggccatg gcctagaatt tgattcaaac aaggctatgg ttcctaaact aggaactggc 31860

cttagttttg acagcacagg tgccattaca gtaggaaaca aaaataatga taagctaacc 31920

ctatggacag gtccaaaacc agaagccaac tgcataattg aatacgggaa acaaaaccca 31980

gatagcaaac taactttaat ccttgtaaaa aatggaggaa ttgttaatgg atatgtaacg 32040

ctaatgggag cctcagacta cgttaacacc ttatttaaaa acaaaaatgt ctccattaat 32100

gtagaactat actttgatgc cactggtcat atattaccag actcatcttc tcttaaaaca 32160

gatctagaac taaaatacaa gcaaaccgct gactttagtg caagaggttt tatgccaagt 32220

actacagcgt atccatttgt ccttcctaat gcgggaacac ataatgaaaa ttatattttt 32280

ggtcaatgct actacaaagc aagcgatggt gccctttttc cgttggaagt tactgttatg 32340

cttaataaac gcctgccaga tagtcgcaca tcctatgtta tgactttttt atggtccttg 32400

aatgctggtc tagctccaga aactactcag gcaaccctca taacctcccc atttaccttt 32460

tcctatatta gagaagatga ctaataaact ctaaagaatc gtttgtgtta tgtttcaacg 32520

tgtttatttt tcaattgcag aaaatttcaa gtcatttttc attcagtagt atagccccac 32580

caccacatag cttatacaga tcaccgtacc ttaatcaaac tcacagaacc ctagtattca 32640

acctgccacc tccctcccaa cacacagagt acacagtcct ttctccccgg ctggccttaa 32700

aaagcatcat atcatgggta acagacatat tcttaggtgt tatattccac acggtttcct 32760

gtcgagccaa acgctcatca gtgatattaa taaactcccc gggcagctca cttaagttca 32820

tgtcgctgtc cagctgctga gccacaggct gctgtccaac ttgcggttgc ttaacgggcg 32880

gcgaaggaga agtccacgcc tacatggggg tagagtcata atcgtgcatc aggatagggc 32940

ggtggtgctg cagcagcgcg cgaataaact gctgccgccg ccgctccgtc ctgcaggaat 33000

acaacatggc agtggtctcc tcagcgatga ttcgcaccgc ccgcagcata aggcgccttg 33060

tcctccgggc acagcagcgc accctgatct cacttaaatc agcacagtaa ctgcagcaca 33120

gcaccacaat attgttcaaa atcccacagt gcaaggcgct gtatccaaag ctcatggcgg 33180

ggaccacaga acccacgtgg ccatcatacc acaagcgcag gtagattaag tggcgacccc 33240

tcataaacac gctggacata aacattacct cttttggcat gttgtaattc accacctccc 33300

ggtaccatat aaacctctga ttaaacatgg cgccatccac caccatccta aaccagctgg 33360

ccaaaacctg cccgccggct atacactgca gggaaccggg actggaacaa tgacagtgga 33420

gagcccagga ctcgtaacca tggatcatca tgctcgtcat gatatcaatg ttggcacaac 33480

acaggcacac gtgcatacac ttcctcagga ttacaagctc ctcccgcgtt agaaccatat 33540

cccagggaac aacccattcc tgaatcagcg taaatcccac actgcaggga agacctcgca 33600

cgtaactcac gttgtgcatt gtcaaagtgt tacattcggg cagcagcgga tgatcctcca 33660

gtatggtagc gcgggtttct gtctcaaaag gaggtagacg atccctactg tacggagtgc 33720

gccgagacaa ccgagatcgt gttggtcgta gtgtcatgcc aaatggaacg ccggacgtag 33780

tcatatttcc tgaagcaaaa ccaggtgcgg gcgtgacaaa cagatctgcg tctccggtct 33840

cgccgcttag atcgctctgt gtagtagttg tagtatatcc actctctcaa agcatccagg 33900

cgccccctgg cttcgggttc tatgtaaact ccttcatgcg ccgctgccct gataacatcc 33960

accaccgcag aataagccac acccagccaa cctacacatt cgttctgcga gtcacacacg 34020

ggaggagcgg gaagagctgg aagaaccatg tttttttttt tattccaaaa gattatccaa 34080

aacctcaaaa tgaagatcta ttaagtgaac gcgctcccct ccggtggcgt ggtcaaactc 34140

tacagccaaa gaacagataa tggcatttgt aagatgttgc acaatggctt ccaaaaggca 34200

aacggccctc acgtccaagt ggacgtaaag gctaaaccct tcagggtgaa tctcctctat 34260

aaacattcca gcaccttcaa ccatgcccaa ataattctca tctcgccacc ttctcaatat 34320

atctctaagc aaatcccgaa tattaagtcc ggccattgta aaaatctgct ccagagcgcc 34380

ctccaccttc agcctcaagc agcgaatcat gattgcaaaa attcaggttc ctcacagacc 34440

tgtataagat tcaaaagcgg aacattaaca aaaataccgc gatcccgtag gtcccttcgc 34500

agggccagct gaacataatc gtgcaggtct gcacggacca gcgcggccac ttccccgcca 34560

ggaaccatga caaaagaacc cacactgatt atgacacgca tactcggagc tatgctaacc 34620

agcgtagccc cgatgtaagc ttgttgcatg ggcggcgata taaaatgcaa ggtgctgctc 34680

aaaaaatcag gcaaagcctc gcgcaaaaaa gaaagcacat cgtagtcatg ctcatgcaga 34740

taaaggcagg taagctccgg aaccaccaca gaaaaagaca ccatttttct ctcaaacatg 34800

tctgcgggtt tctgcataaa cacaaaataa aataacaaaa aaacatttaa acattagaag 34860

cctgtcttac aacaggaaaa acaaccctta taagcataag acggactacg gccatgccgg 34920

cgtgaccgta aaaaaactgg tcaccgtgat taaaaagcac caccgacagc tcctcggtca 34980

tgtccggagt cataatgtaa gactcggtaa acacatcagg ttgattcaca tcggtcagtg 35040

ctaaaaagcg accgaaatag cccgggggaa tacatacccg caggcgtaga gacaacatta 35100

cagcccccat aggaggtata acaaaattaa taggagagaa aaacacataa acacctgaaa 35160

aaccctcctg cctaggcaaa atagcaccct cccgctccag aacaacatac agcgcttcca 35220

cagcggcagc cataacagtc agccttacca gtaaaaaaga aaacctatta aaaaaacacc 35280

actcgacacg gcaccagctc aatcagtcac agtgtaaaaa agggccaagt gcagagcgag 35340

tatatatagg actaaaaaat gacgtaacgg ttaaagtcca caaaaaacac ccagaaaacc 35400

gcacgcgaac ctacgcccag aaacgaaagc caaaaaaccc acaacttcct caaatcgtca 35460

cttccgtttt cccacgttac gtcacttccc attttaagaa aactacaatt cccaacacat 35520

acaagttact ccgccctaaa acctacgtca cccgccccgt tcccacgccc cgcgccacgt 35580

cacaaactcc accccctcat tatcatattg gcttcaatcc aaaataaggt atattattga 35640

tgatgttaat 35650

<210> 5
<211> 8
<212> PRT
<213> Artificial Sequence

<220>
<223> Binding site for Rb protein in E1A

<400> 5
Leu Thr Cys His Glu Ala Gly Phe
1               5

<210> 6
<211> 45
<212> DNA
<213> Artificial Sequence

<220>
<223> CR2-D24

<400> 6
cttgtaccgg aggtgatcga tccacccagt gacgacgagg atgaa 45

<210> 7
<211> 663
<212> DNA
<213> Artificial Sequence

<220>
<223> TIMP2 sequence

<400> 7
atgggcgccg cggcccgcac cctgcggctg gcgctcggcc tcctgctgct ggcgacgctg 60

cttcgcccgg ccgacgcctg cagctgctcc ccggtgcacc cgcaacaggc gttttgcaat 120

gcagatgtag tgatcagggc caaagcggtc agtgagaagg aagtggactc tggaaacgac 180

atttatggca accctatcaa gaggatccag tatgagatca agcagataaa gatgttcaaa 240

gggcctgaga aggatataga gtttatctac acggccccct cctcggcagt gtgtggggtc 300

tcgctggacg ttggaggaaa gaaggaatat ctcattgcag gaaaggccga gggggacggc 360

aagatgcaca tcaccctctg tgacttcatc gtgccctggg acaccctgag caccacccag 420

aagaagagcc tgaaccacag gtaccagatg ggctgcgagt gcaagatcac gcgctgcccc 480

atgatcccgt gctacatctc ctccccggac gagtgcctct ggatggactg ggtcacagag 540

aagaacatca acgggcacca ggccaagttc ttcgcctgca tcaagagaag tgacggctcc 600

tgtgcgtggt accgcggcgc ggcgcccccc aagcaggagt ttctcgacat cgaggaccca 660

taa 663

<210> 8
<211> 1764
<212> DNA
<213> Artificial Sequence

<220>
<223> Ad5/3 fiber

<400> 8
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 9
<211> 45
<212> DNA
<213> Artificial Sequence

<220>
<223> CR2-D24

<400> 9
cttgtaccgg aggtgatcga tccacccagt gacgacgagg atgaa 45

<210> 10
<211> 1764
<212> DNA
<213> Artificial Sequence

<220>
<223> Ad5/3 fiber

<400> 10
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 11
<211> 1992
<212> DNA
<213> Artificial Sequence

<220>
<223> PADI1

<400> 11
atggccccaa agagagttgt gcagctgtcc ctgaagatgc ctacccatgc cgtgtgtgtg 60

gtgggagtcg aggcacatgt ggacattcac agtgatgtgc ccaagggtgc caacagcttc 120

agggtctctg gaagctccgg ggtggaggtc ttcatggtct acaaccgcac acgtgtgaaa 180

gagcccatag gcaaggcccg ttggccgcta gacactgatg cagacatggt cgtatctgtg 240

ggcacagcca gtaaggaatt aaaggacttc aaggtgaggg tctcctactt tggggagcag 300

gaagaccaag ctctgggccg cagcgtgctt tacctcactg gcgtcgatat ttcccttgag 360

gttgacacag gccgcacagg caaggtgaag aggagccaag gggacaagaa aacctggcgc 420

tggggccctg agggctatgg ggctatcttg ctggtgaact gtgaccggga caatcacagg 480

tccgcggagc ctgacctcac ccacagctgg ctgatgtcgc tggctgacct gcaggacatg 540

tccccaatgc tgctgagctg caatggcccc gacaagctct tcgacagcca caagcttgtc 600

ttgaacgtgc ccttttctga ttccaaaaga gtgagggtct tctgtgccag gggtgggaat 660

tctctctcgg actacaaaca ggtgctgggg ccccagtgtc tgtcctatga agttgagcga 720

cagccagggg agcaggagat caagttctat gtggaggggc tgaccttccc cgatgccgat 780

ttcctagggc tggtttccct cagtgtcagc ctggtggacc cggggaccct gcccgaggtg 840

accctcttca cagacactgt gggcttccgc atggccccct ggatcatgac gcccaacact 900

cagcctcctg aggagctgta tgtgtgcaga gtgatggaca ctcatggctc caatgagaaa 960

ttcctggagg acatgtctta tctgacattg aaagccaact gcaagctgac catctgccct 1020

caagttgaaa atcgaaatga ccgctggatc caggacgaga tggagtttgg ctacatcgag 1080

gcccctcaca aatccttccc cgtggtcttt gactccccca ggaacagggg cctgaaagat 1140

ttcccctata agaggatcct gggtcctgac tttggatatg ttacccggga gatcccgctc 1200

cctggtccct ccagccttga ctccttcggc aacctggacg tcagcccgcc cgtcacgggg 1260

ggcggcacgg aataccccct gggccggatc ctcatcggga gcagcttccc caagtccggt 1320

gggcggcaga tggccagggc agtgcggaac ttcctgaagg cacagcaggt gcaggcaccc 1380

gtggagctct actcggactg gctctctgtg ggccatgtgg acgagtttct gacctttgtg 1440

cctacctctg accagaaggg cttccggctg ctcctggcta gccccagcgc ttgccccaaa 1500

ctcttccaag agaagaaaga agagggttat ggggaggcag cccagtttga tgggttaaaa 1560

caccaggcaa aaagaagcat taatgagatg ctggcagaca gacacctcca gagagacaat 1620

cttcatgcac agaaatgcat tgactggaac cgtaatgtgc tgaagcggga gctgggcctg 1680

gcagagagtg acatcgtgga cattccccag ctcttcttcc tgaaaaactt ctacgcggaa 1740

gccttcttcc cagacatggt taacatggtg gtcttaggca agtacctggg catccccaag 1800

ccctacgggc ccatcatcaa tggccgctgc tgcctggagg agaaggtgca gtccctgctg 1860

gagcctctgg gcctgcactg catcttcatt gatgactact tgtcctacca cgagctgcag 1920

ggggagatcc actgtggcac caacgtgcgc aggaagccct ttcccttcaa atggtggaac 1980

atggtgccct ga 1992

<210> 12
<211> 45
<212> DNA
<213> Artificial Sequence

<220>
<223> CR2-D24

<400> 12
cttgtaccgg aggtgatcga tccacccagt gacgacgagg atgaa                    45


<210> 13
<211> 1764
<212> DNA
<213> Artificial Sequence


<220>
<223> Ad5/3 fiber

<400> 13
atgaagcgcg caagaccgtc tgaagatacc ttcaaccccg tgtatccata tgacacggaa      60

accggtcctc caactgtgcc ttttcttact cctccctttg tatcccccaa tgggtttcaa     120

gagagtcccc ctggggtact ctctttgcgc ctatccgaac ctctagttac ctccaatggc     180

atgcttgcgc tcaaaatggg caacggcctc tctctggacg aggccggcaa ccttacctcc     240

caaaatgtaa ccactgtgag cccacctctc aaaaaaacca agtcaaacat aaacctggaa     300

atatctgcac ccctcacagt tacctcagaa gccctaactg tggctgccgc cgcacctcta     360

atggtcgcgg gcaacacact caccatgcaa tcacaggccc cgctaaccgt gcacgactcc     420

aaacttagca ttgccaccca aggacccctc acagtgtcag aaggaaagct agccctgcaa     480

acatcaggcc ccctcaccac caccgatagc agtaccctta ctatcactgc ctcaccccct     540

ctaactactg ccactggtag cttgggcatt gacttgaaag agcccattta tacacaaaat     600

ggaaaactag gactaaagta cggggctcct ttgcatgtaa cagacgacct aaacactttg     660

accgtagcaa ctggtccagg tgtgactatt aataatactt ccttgcaaac taaagttact     720

ggagccttgg gttttgattc acaaggcaat atgcaactta atgtagcagg aggactaagg     780

attgattctc aaaacagacg ccttatactt gatgttagtt atccgtttga tgctcaaaac     840

caactaaatc taagactagg acagggccct ctttttataa actcagccca caacttggat     900

attaactaca acaaaggcct ttacttgttt acagcttcaa acaattccaa aaagcttgag     960

gttaacctaa gcactgccaa ggggttgatg tttgacgcta cagccatagc cattaatgca    1020

ggagatgggc ttgaatttgg ttcacctaat gcaccaaaca caaatcccct caaaacaaaa    1080

attggccatg gcctagaatt tgattcaaac aaggctatgg ttcctaaact aggaactggc    1140

cttagttttg acagcacagg tgccattaca gtaggaaaca aaaataatga taagctaacc    1200

ctatggacag gtccaaaacc agaagccaac tgcataattg aatacgggaa acaaaaccca    1260

gatagcaaac taactttaat ccttgtaaaa aatggaggaa ttgttaatgg atatgtaacg    1320

ctaatgggag cctcagacta cgttaacacc ttatttaaaa acaaaaatgt ctccattaat    1380

gtagaactat actttgatgc cactggtcat atattaccag actcatcttc tcttaaaaca    1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 14
<211> 1989
<212> DNA
<213> Artificial Sequence

<220>
<223> PADI1

<400> 14
atggccccaa agagagttgt gcagctgtcc ctgaagatgc ctacccatgc cgtgtgtgtg 60

gtgggagtcg aggcacatgt ggacattcac agtgatgtgc ccaagggtgc caacagcttc 120

agggtctctg gaagctccgg ggtggaggtc ttcatggtct acaaccgcac acgtgtgaaa 180

gagcccatag gcaaggcccg ttggccgcta gacactgatg cagacatggt cgtatctgtg 240

ggcacagcca gtaaggaatt aaaggacttc aaggtgaggg tctcctactt tggggagcag 300

gaagaccaag ctctgggccg cagcgtgctt tacctcactg gcgtcgatat ttcccttgag 360

gttgacacag gccgcacagg caaggtgaag aggagccaag gggacaagaa aacctggcgc 420

tggggccctg agggctatgg ggctatcttg ctggtgaact gtgaccggga caatcacagg 480

tccgcggagc ctgacctcac ccacagctgg ctgatgtcgc tggctgacct gcaggacatg 540

tccccaatgc tgctgagctg caatggcccc gacaagctct tcgacagcca caagcttgtc 600

ttgaacgtgc ccttttctga ttccaaaaga gtgagggtct tctgtgccag gggtgggaat 660

tctctctcgg actacaaaca ggtgctgggg ccccagtgtc tgtcctatga agttgagcga 720

cagccagggg agcaggagat caagttctat gtggaggggc tgaccttccc cgatgccgat 780

ttcctagggc tggtttccct cagtgtcagc ctggtggacc cggggaccct gcccgaggtg 840

accctcttca cagacactgt gggcttccgc atggccccct ggatcatgac gcccaacact 900

cagcctcctg aggagctgta tgtgtgcaga gtgatggaca ctcatggctc caatgagaaa 960

ttcctggagg acatgtctta tctgacattg aaagccaact gcaagctgac catctgccct 1020

caagttgaaa atcgaaatga ccgctggatc caggacgaga tggagtttgg ctacatcgag 1080

gcccctcaca aatccttccc cgtggtcttt gactccccca ggaacagggg cctgaaagat 1140

ttcccctata agaggatcct gggtcctgac tttggatatg ttacccggga gatcccgctc 1200

cctggtccct ccagccttga ctccttcggc aacctggacg tcagcccgcc cgtcacgggg 1260

ggcggcacgg aataccccct gggccggatc ctcatcggga gcagcttccc caagtccggt 1320

gggcggcaga tggccagggc agtgcggaac ttcctgaagg cacagcaggt gcaggcaccc 1380

gtggagctct actcggactg gctctctgtg ggccatgtgg acgagtttct gacctttgtg 1440

cctacctctg accagaaggg cttccggctg ctcctggcta gccccagcgc ttgccccaaa 1500

ctcttccaag agaagaaaga agagggttat ggggaggcag cccagtttga tgggttaaaa 1560

caccaggcaa aaagaagcat taatgagatg ctggcagaca gacacctcca gagagacaat 1620

cttcatgcac agaaatgcat tgactggaac cgtaatgtgc tgaagcggga gctgggcctg 1680

gcagagagtg acatcgtgga cattccccag ctcttcttcc tgaaaaactt ctacgcggaa 1740

gccttcttcc cagacatggt taacatggtg gtcttaggca agtacctggg catccccaag 1800

ccctacgggc ccatcatcaa tggccgctgc tgcctggagg agaaggtgca gtccctgctg 1860

gagcctctgg gcctgcactg catcttcatt gatgactact tgtcctacca cgagctgcag 1920

ggggagatcc actgtggcac caacgtgcgc aggaagccct ttcccttcaa atggtggaac 1980

atggtgccc 1989

<210> 15
<211> 57
<212> DNA
<213> Artificial Sequence

<220>
<223> P2A

<400> 15
gccactaact tctccctgtt aaagcaagca ggcgatgttg aagaaaaccc cgggcct 57

<210> 16
<211> 663
<212> DNA
<213> Artificial Sequence

<220>
<223> TIMP2

<400> 16
atgggcgccg cggcccgcac cctgcggctg gcgctcggcc tcctgctgct ggcgacgctg 60

cttcgcccgg ccgacgcctg cagctgctcc ccggtgcacc cgcaacaggc gttttgcaat 120

gcagatgtag tgatcagggc caaagcggtc agtgagaagg aagtggactc tggaaacgac 180

atttatggca accctatcaa gaggatccag tatgagatca agcagataaa gatgttcaaa 240

gggcctgaga aggatataga gtttatctac acggccccct cctcggcagt gtgtggggtc 300

tcgctggacg ttggaggaaa gaaggaatat ctcattgcag gaaaggccga gggggacggc 360

aagatgcaca tcaccctctg tgacttcatc gtgccctggg acaccctgag caccacccag 420

aagaagagcc tgaaccacag gtaccagatg ggctgcgagt gcaagatcac gcgctgcccc 480

atgatcccgt gctacatctc ctccccggac gagtgcctct ggatggactg ggtcacagag 540

aagaacatca acgggcacca ggccaagttc ttcgcctgca tcaagagaag tgacggctcc 600

tgtgcgtggt accgcggcgc ggcgcccccc aagcaggagt ttctcgacat cgaggaccca 660

taa 663

<210> 17
<211> 45
<212> DNA
<213> Artificial Sequence

<220>
<223> CR2-D24

<400> 17
cttgtaccgg aggtgatcga tccacccagt gacgacgagg atgaa 45

<210> 18
<211> 1764
<212> DNA
<213> Artificial Sequence

<220>
<223> Ad5/3 fiber

<400> 18
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 19
<211> 534
<212> DNA
<213> Artificial Sequence

<220>
<223> CMV promoter/enhancer

<400> 19
ctagctagtt attaatagta atcaattacg gggtcattag ttcatagccc atatatggag 60

ttccgcgtta cataacttac ggtaaatggc ccgcctggct gaccgcccaa cgacccccgc 120

ccattgacgt caataatgac gtatgttccc atagtaacgc caatagggac tttccattga 180

cgtcaatggg tggagtattt acggtaaact gcccacttgg cagtacatca agtgtatcat 240

atgccaagtc cgccccctat tgacgtcaat gacggtaaat ggcccgcctg gcattatgcc 300

cagtacatga ccttacggga ctttcctact tggcagtaca tctacgtatt agtcatcgct 360

attaccatgg tgatgcggtt ttggcagtac accaatgggc gtggatagcg gtttgactca 420

cggggatttc caagtctcca ccccattgac gtcaatggga gtttgttttg gcaccaaaat 480

caacgggact ttccaaaatg tcgtaatacc cccgccccgt tgacgcaaat gggc 534

<210> 20
<211> 26
<212> DNA
<213> Artificial Sequence

<220>
<223> CuO

<400> 20
caaacagacc agattgtctg tttgtt                                            26

<210> 21
<211> 73
<212> DNA
<213> Artificial Sequence

<220>
<223> CMV 5\222UTR

<400> 21
tcatatcgcc tggagacgcc atccacgctg ttttgacctc catagaagac accgggaccg      60

atccagcctc cgc                                                          73

<210> 22
<211> 1992
<212> DNA
<213> Artificial Sequence

<220>
<223> PADI1

<400> 22
atggccccaa agagagttgt gcagctgtcc ctgaagatgc ctacccatgc cgtgtgtgtg      60

gtgggagtcg aggcacatgt ggacattcac agtgatgtgc ccaagggtgc caacagcttc     120

agggtctctg gaagctccgg ggtggaggtc ttcatggtct acaaccgcac acgtgtgaaa     180

gagcccatag gcaaggcccg ttggccgcta gacactgatg cagacatggt cgtatctgtg     240

ggcacagcca gtaaggaatt aaaggacttc aaggtgaggg tctcctactt tggggagcag     300

gaagaccaag ctctgggccg cagcgtgctt tacctcactg gcgtcgatat ttcccttgag     360

gttgacacag gccgcacagg caaggtgaag aggagccaag gggacaagaa aacctggcgc     420

tggggccctg agggctatgg ggctatcttg ctggtgaact gtgaccggga caatcacagg     480

tccgcggagc ctgacctcac ccacagctgg ctgatgtcgc tggctgacct gcaggacatg     540

tccccaatgc tgctgagctg caatggcccc gacaagctct tcgacagcca caagcttgtc     600

ttgaacgtgc ccttttctga ttccaaaaga gtgagggtct tctgtgccag gggtgggaat     660

tctctctcgg actacaaaca ggtgctgggg ccccagtgtc tgtcctatga agttgagcga     720

cagccagggg agcaggagat caagttctat gtggaggggc tgaccttccc cgatgccgat 780

ttcctagggc tggtttccct cagtgtcagc ctggtggacc cggggaccct gcccgaggtg 840

accctcttca cagacactgt gggcttccgc atggccccct ggatcatgac gcccaacact 900

cagcctcctg aggagctgta tgtgtgcaga gtgatggaca ctcatggctc caatgagaaa 960

ttcctggagg acatgtctta tctgacattg aaagccaact gcaagctgac catctgccct 1020

caagttgaaa atcgaaatga ccgctggatc caggacgaga tggagtttgg ctacatcgag 1080

gcccctcaca aatccttccc cgtggtcttt gactccccca ggaacagggg cctgaaagat 1140

ttcccctata agaggatcct gggtcctgac tttggatatg ttacccggga gatcccgctc 1200

cctggtccct ccagccttga ctccttcggc aacctggacg tcagcccgcc cgtcacgggg 1260

ggcggcacgg aataccccct gggccggatc ctcatcggga gcagcttccc caagtccggt 1320

gggcggcaga tggccagggc agtgcggaac ttcctgaagg cacagcaggt gcaggcaccc 1380

gtggagctct actcggactg gctctctgtg ggccatgtgg acgagtttct gacctttgtg 1440

cctacctctg accagaaggg cttccggctg ctcctggcta gccccagcgc ttgccccaaa 1500

ctcttccaag agaagaaaga agagggttat ggggaggcag cccagtttga tgggttaaaa 1560

caccaggcaa aaagaagcat taatgagatg ctggcagaca gacacctcca gagagacaat 1620

cttcatgcac agaaatgcat tgactggaac cgtaatgtgc tgaagcggga gctgggcctg 1680

gcagagagtg acatcgtgga cattccccag ctcttcttcc tgaaaaactt ctacgcggaa 1740

gccttcttcc cagacatggt taacatggtg gtcttaggca agtacctggg catccccaag 1800

ccctacgggc ccatcatcaa tggccgctgc tgcctggagg agaaggtgca gtccctgctg 1860

gagcctctgg gcctgcactg catcttcatt gatgactact tgtcctacca cgagctgcag 1920

ggggagatcc actgtggcac caacgtgcgc aggaagccct ttcccttcaa atggtggaac 1980

atggtgccct ga 1992

<210> 23
<211> 572
<212> DNA
<213> Artificial Sequence

<220>
<223> IRES (30,896-31,467bp)

<400> 23
cccctctccc tcccccccct aacgttactg gccgaagccg cttggaataa ggccggtgtg 60

cgtttgtcta tatgttattt tccaccatat tgccgtcttt tggcaatgtg agggcccgga 120

aacctggccc tgtcttcttg acgagcattc ctaggggtct ttcccctctc gccaaaggaa 180

tgcaaggtct gttgaatgtc gtgaaggaag cagttcctct ggaagcttct tgaagacaaa 240

caacgtctgt agcgaccctt tgcaggcagc ggaacccccc acctggcgac aggtgcctct 300

gcggccaaaa gccacgtgta taagatacac ctgcaaaggc ggcacaaccc cagtgccacg 360

ttgtgagttg gatagttgtg gaaagagtca aatggctctc ctcaagcgta ttcaacaagg 420

ggctgaagga tgcccagaag gtaccccatt gtatgggatc tgatctgggg cctcggtgca 480

catgctttac atgtgtttag tcgaggttaa aaaaacgtct aggccccccg aaccacgggg 540

acgtggtttt cctttgaaaa acacgatgat aa 572

<210> 24
<211> 658
<212> DNA
<213> Artificial Sequence

<220>
<223> TIMP2

<400> 24
cgccgcggcc cgcaccctgc ggctggcgct cggcctcctg ctgctggcga cgctgcttcg 60

cccggccgac gcctgcagct gctccccggt gcacccgcaa caggcgtttt gcaatgcaga 120

tgtagtgatc agggccaaag cggtcagtga gaaggaagtg gactctggaa acgacattta 180

tggcaaccct atcaagagga tccagtatga gatcaagcag ataaagatgt tcaaagggcc 240

tgagaaggat atagagttta tctacacggc cccctcctcg gcagtgtgtg gggtctcgct 300

ggacgttgga ggaaagaagg aatatctcat tgcaggaaag gccgaggggg acggcaagat 360

gcacatcacc ctctgtgact tcatcgtgcc ctgggacacc ctgagcacca cccagaagaa 420

gagcctgaac cacaggtacc agatgggctg cgagtgcaag atcacgcgct gccccatgat 480

cccgtgctac atctcctccc cggacgagtg cctctggatg gactgggtca cagagaagaa 540

catcaacggg caccaggcca agttcttcgc ctgcatcaag agaagtgacg gctcctgtgc 600

gtggtaccgc ggcgcggcgc cccccaagca ggagtttctc gacatcgagg acccataa 658

<210> 25
<211> 122
<212> DNA
<213> Artificial Sequence

<220>
<223> SV40pA

<400> 25
aacttgttta ttgcagctta taatggttac aaataaagca atagcatcac aaatttcaca 60

aataaagcat ttttttcact gcattctagt tgtggtttgt ccaaactcat caatgtatct 120

ta 122

<210> 26
<211> 66
<212> DNA
<213> Artificial Sequence

<220>
<223> Primer 288-13

<400> 26
ggcagcggcg ccactaactt ctccctgtta aagcaagcag gcgatgttga agaaaacccc 60

gggcct 66

<210> 27
<211> 66
<212> DNA
<213> Artificial Sequence

<220>
<223> Primer 288-14

<400> 27
aggcccgggg ttttcttcaa catcgcctgc ttgctttaac agggagaagt tagtggcgcc 60

gctgcc 66

<210> 28
<211> 40
<212> DNA
<213> Artificial Sequence

<220>
<223> Primer 288-15

<400> 28
aatggtggaa catggtgccc ggcagcggcg ccactaactt 40

<210> 29
<211> 40
<212> DNA
<213> Artificial Sequence

<220>
<223> Primer 288-16

<400> 29
gtgcgggccg cggcgcccat aggcccgggg ttttcttcaa 40

<210> 30
<211> 26
<212> DNA
<213> Artificial Sequence

<220>

<223> Primer 288-21

<400> 30
tagttaaagg gaataagatc ggtacc                                          26


<210> 31
<211> 61
<212> DNA
<213> Artificial Sequence


<220>
<223> Primer 288-22

<400> 31
acaccgggac cgatccagcc tccgcgggcc gccaccatgg ccccaaagag agttgtgcag     60

c                                                                     61


<210> 32
<211> 40
<212> DNA
<213> Artificial Sequence


<220>
<223> Primer 288-23

<400> 32
   ggtggaacat ggtgccctga cccctctccc tcccccccct                            40


<210> 33
<211> 40
<212> DNA
<213> Artificial Sequence


<220>
<223> Primer 288-24

<400> 33
gtgcgggccg cggcgcccat gtcgactcta gaggatcccg                           40


<210> 34
<211> 40
<212> DNA
<213> Artificial Sequence


<220>
<223> Primer 288-25

<400> 34
tcgacatcga ggacccataa aacttgttta ttgcagctta                           40


<210> 35
<211> 25
<212> DNA

<213> Artificial Sequence

<220>
<223> Primer 288-42

<400> 35
tcagggcacc atgttccacc atttg                                          25

## Description

### FIELD OF THE DISCLOSURE

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing peptidylarginine deiminase and tissue inhibitors of metalloproteinase in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

### BACKGROUND OF THE DISCLOSURE

During the last twenty years gene transfer technology has been under intensive examination. The aim of cancer gene therapies is to introduce a therapeutic gene into a tumor cell. These therapeutic genes introduced to a target cell may for example correct mutated genes, suppress active oncogenes or generate additional properties to the cell. Suitable exogenous therapeutic genes include but are not limited to immunotherapeutic, anti-angiogenic, chemoprotective and "suicide" genes, and they can be introduced to a cell by utilizing modified virus vectors or non-viral methods including electroporation, gene gun and lipid or polymer coatings.

Requirements of optimal viral vectors include an efficient capability to find specific target cells and express the viral genome in the target cells. Furthermore, optimal vectors have to stay active in the target tissues or cells. All these properties of viral vectors have been developed during the last decades and for example retroviral, adenoviral and adeno-associated viral vectors have been widely studied in biomedicine.

To further improve tumor penetration and local amplification of the anti-tumor effect, selectively oncolytic agents, e.g. conditionally replicating adenoviruses, have been constructed. Oncolytic adenoviruses are a promising tool for treatment of cancers. Tumor cells are killed by oncolytic adenoviruses due to a replication of the virus in a tumor cell, the last phase of the replication resulting in a release of thousands of virions into the surrounding tumor tissues for effective tumor penetration and vascular re-infection. Tumor cells allow replication of the virus while normal cells are spared due to engineered changes in the virus genome, which prevent replication in non-tumor cells.

In addition to replication mediated cell killing, oncolytic adenoviruses can also be armed with different therapeutic transgenes. This approach combines the advantages of conventional gene delivery with the potency of replication competent agents. One goal of arming viruses is induction of an immune reaction towards the cells that allow virus replication. Virus replication alone, although immunogenic, is normally not enough to induce effective anti-tumor immunity. To strengthen induction of therapeutic immunity, viruses can be armed with stimulatory proteins such as cytokines for facilitation of the introduction of tumor antigens to antigen presenting cells such as dendritic cells, and their stimulation and/or maturation. Introduction of immunotherapeutic genes into tumor cells and furthermore, translation of the proteins, leads to activation of the immune response and efficient destruction of tumor cells. The most relevant immune cells in this regard are natural killer cells (NK), CD4+ T helper cells and cytotoxic CD8+ T-cells.

More than 50 different serotypes of adenoviruses have been found in humans. Adenovirus serotype 5 (Ad5) is known to cause respiratory diseases and it is the most common serotype studied in the field of gene therapy. In the first Ad5 vectors E1 and/or E3 regions were deleted enabling insertion of foreign DNA to the vectors. Furthermore, deletions of other regions as well as further mutations have provided extra properties to viral vectors, indeed, various modifications of adenoviruses have been suggested for achieving efficient antitumor effects.

Matrix metalloproteinases (MMPs) are endopeptidases. The MMPs belong to a larger family of proteases known as the metzincin superfamily. these enzymes are capable of degrading all kinds of extracellular matrix proteins. They are known to be involved in the cleavage of cell surface receptors, the release of apoptotic ligands and chemokine/cytokine inactivation. MMPs are also thought to play a major role in cell proliferation, migration (adhesion/dispersion), differentiation, angiogenesis, apoptosis, and host defense.

The matrix metalloproteinases are inhibited by specific endogenous tissue inhibitors of metalloproteinases (TIMPs), which comprise a family of four protease inhibitors: TIMP1, TIMP2, TIMP3 and TIMP4. Of the four TIMPs, TIMP1 overexpression or TIMP3 silencing is consistently associated with cancer progression or poor patient prognosis.

In enzymology, a protein-arginine deiminase (PADI) is an enzyme that catalyzes a form of post translational modification called arginine de-imination or citrullination. This enzyme belongs to the family of hydrolases, those acting on carbon-nitrogen bonds other than peptide bonds, specifically in linear amidines. The systematic name of this enzyme class is protein-L-arginine iminohydrolase. This enzyme is also called peptidylarginine deiminase. There are four members in this family, namely PADI1, PADI2, PADI3 and PADI4. PADI1 (peptidyl arginine deiminase 1), an enzyme isolated from Mycoplasma, degrades arginine into its citrulline precursor. It has been shown that deprivation of arginine (tumor starvation) has impact on tumor regression (G1 cell cycle arrest with apoptosis and anti-angiogenesis properties), (melanoma, prostate, hepatocellular carcinoma seems to be a good target). Arginine deiminase is a novel anticancer enzyme that produces depletion of arginine, which is a nonessential amino acid in humans. These tumours that are sensitive to arginine depletion do not express argininosuccinate synthetase, a key enzyme in the synthesis of arginine from citrulline (Feun and Savaraj, 2006, Jahani et al., 2018).

EP2379586B1 discloses an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding a granulocyte-macrophage colony-stimulating factor (GM-CSF) in the place of the deleted gp19k/6.7K in the adenoviral E3 region. US20160339066A1 discloses to adjunct therapies that include co-administration and co-formulation of a complement inhibitor and/or a lipid emulsion composition with the oncolytic virus. The document also discloses that oncolytic virus, such as adenovirus, can be made to express an anti-metastatic gene, such as TIMP-2. WO2014055960A1 relates to composition containing a nucleic acid encoding a chromophore producing enzyme(s). Anti-metastatic agents, such as TIMP-2, expressed by the oncolytic virus can directly or indirectly inhibit one or more steps of the metastatic cascade.

Still, more efficient and accurate gene transfer as well as increased specificity and sufficient tumor killing ability of gene therapies are warranted. Safety records of therapeutic vectors must also be excellent. The present invention provides a cancer therapeutic tool with these aforementioned properties by utilizing both oncolytic and immunotherapeutic properties of adenoviruses in a novel and inventive way.

### BRIEF DESCRIPTION OF THE DISCLOSURE

The object of the invention is to provide novel methods and means for achieving the above-mentioned properties of adenoviruses and thus, solving the problems of conventional cancer therapies. More specifically, the invention provides novel methods and means for gene therapy.

TIMP-2 is known to be able to inhibit tumor growth, invasion, angiogenesis and metastasis in experimental models, which has been associated with their MMP inhibitory activity (Bourboulia et al., 2013, Seo et al., 2003). The present inventors hypothesized that the members of TIMP family as transgenes could contribute in cancer treatment and enhance properties of an oncolytic virus. PADI1 is a potential inhibitor of angiogenesis and tumour growth. The present inventors hypothesized the members of peptidylarginine deiminase family could be highly beneficial in cancer therapy as they can function both as an antiproliferative and an antiangiogenic agent (Park et al., 2003). The present inventors found that the double transgene virus coding for TIMP2 and PADI1 will induce innate and adaptive immune system leading to the development of anti-tumor responses after local administration. Additionally, it was found that the expression of TIMP2 inhibits the MMPs functionality and thus inhibit the tumor growth. PADI1 can eliminate arginine from the tumor microenvironment inhibiting the tumor growth and leading to tumor starvation. The strategy of using the members of TIMP and peptidylarginine deiminase proteins expressed by the oncolytic virus is to utilize different mechanism of actions that should exhibit synergistic anti-tumor effects.

The double transgene virus was found to be a more efficient model compared to that where both transgenes were encoded by separate viruses but used together. The double transgene simplifies the production process especially in clinical studies over combining two viruses expressing the transgenes separately.

The present application describes construction of recombinant viral vectors, methods related to the vectors, and their use in tumor cells lines and animal models.

The present invention relates to an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding peptidylarginine deiminase and a nucleic acid sequence encoding TIMP connected with IRES sequence in adenovirus E3 region. Alternatively, the nucleic acid sequence encoding peptidylarginine deiminase and the nucleic acid sequence encoding TIMP can be connected with P2A sequence. The nucleic acid sequence encoding peptidylarginine deiminase and the nucleic acid sequence encoding TIMP can be at the same place, where GM-CSF was inserted in ONCOS-102, i.e. in the place of deleted gp19k/6.7K in adenovirus E3 region. Alternatively, the nucleic acid sequence encoding peptidylarginine deiminase and the nucleic acid sequence encoding TIMP can be inserted in the place of deleted 2.7kbp in E3 region. Preferably, the vector contains an adenovirus 3 serotype knob replacing the wild type serotype 5 knob (5/3 chimeric). More preferably, the peptidylarginine deiminase is PADI1 and TIMP is TIMP2.

The present invention further relates to a cell comprising the adenoviral vector of the invention.

The present invention also relates to a pharmaceutical composition comprising the adenoviral vector of the invention.

The present invention also relates to a use of the adenoviral vector of the invention in the manufacture of a medicament for treating cancer in a subject.

The present invention also relates to a method of treating cancer in a subject, wherein the method comprises administration of the vector or pharmaceutical composition of the invention to a subject.

Furthermore, the present invention also relates to a method of producing peptidylarginine deiminase and TIMP in a cell, wherein the method comprises: a) carrying a vehicle comprising an oncolytic adenoviral vector of the invention to a cell, and b) expressing peptidylarginine deiminase and TIMP of said vector in the cell. Preferably, PADI1 and TIMP2 are expressed.

Furthermore, the present invention also relates to a method of increasing tumor specific immune response in a subject, wherein the method comprises: a) carrying a vehicle comprising an oncolytic adenoviral vector of the invention to a target cell or tissue, b) expressing peptidylarginine deiminase and TIMP of said vector in the cell, and c) increasing amount of cytotoxic T cells and/or natural killer cells in said target cell or tissue. Preferably, PADI1 and TIMP2 are expressed.

Still, the present invention also relates to a use of the oncolytic adenoviral vector of the invention for producing peptidylarginine deiminase and TIMP in a cell. Preferably, PADI1 and TIMP2 are produced.

Still, the present invention also relates to a use of the oncolytic adenoviral vector of the invention for increasing tumor specific immune response in a subject.

The present invention provides a tool for treatment of cancers, some of which are refractory to current approaches. Also, restrictions regarding tumor types suitable for treatment remain few compared to many other treatments. In fact, all solid tumors may be treated with the proposed invention. Larger tumors by mass and more complex tumors can be treated by using the present invention. The treatment can be given intratumorally, intracavitary, intravenously and in a combination of these. The approach can give systemic efficacy despite local injection. The approach can also eradicate cells proposed as tumor initiating ("cancer stem cells").

### BRIEF DESCRIPTION OF THE DRAWINGS

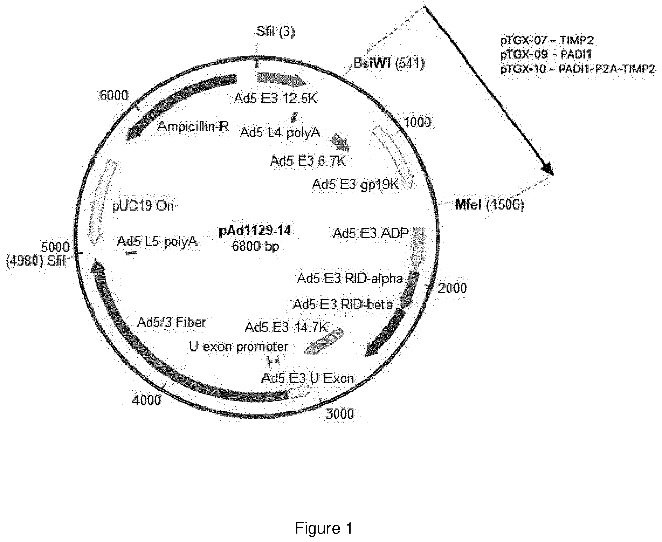

Figure 1 illustrates the shuttle plasmid constructions for the shuttle plasmids for pTGX-07, -09 and -10.

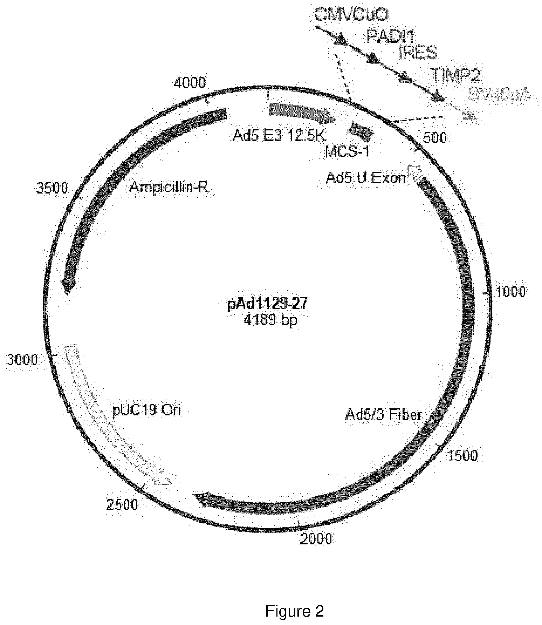

Figure 2 illustrates the shuttle plasmid constructions for the shuttle plasmid for pTGX-12 (PADI1-IRES-TIMP2).

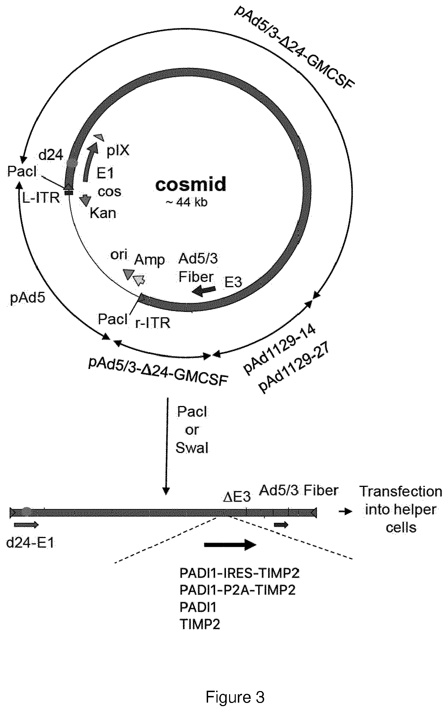

Figure 3 illustrates the cosmid constructions for TGX-07, TGX-09, TGX-10 and TGX-12.

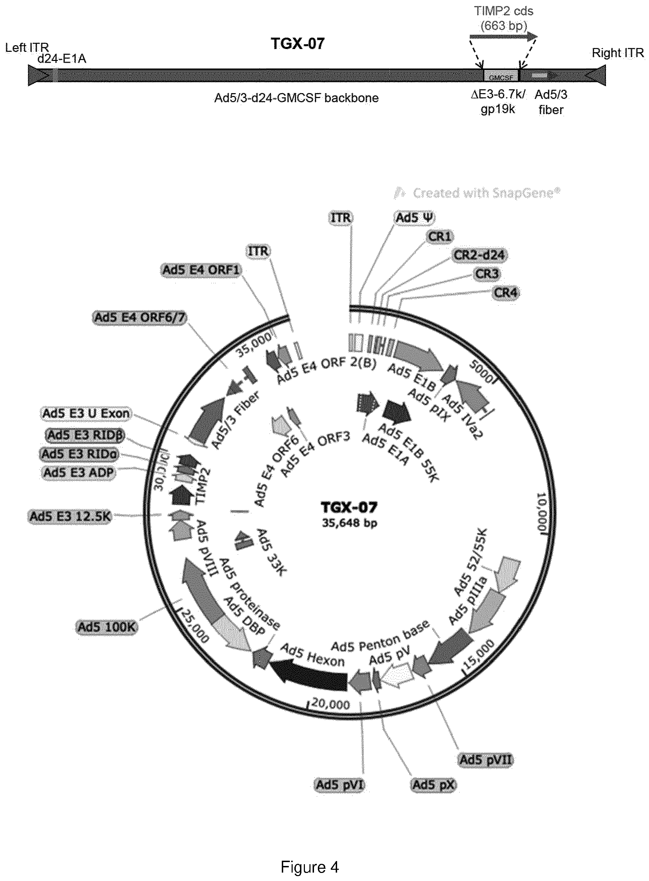

Figure 4 shows TGX-07 virus structure. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 30,721 - 32,484bp) and codes for the functional TIMP2 protein (functional expression cassette, location: 28,371 - 29,033bp).

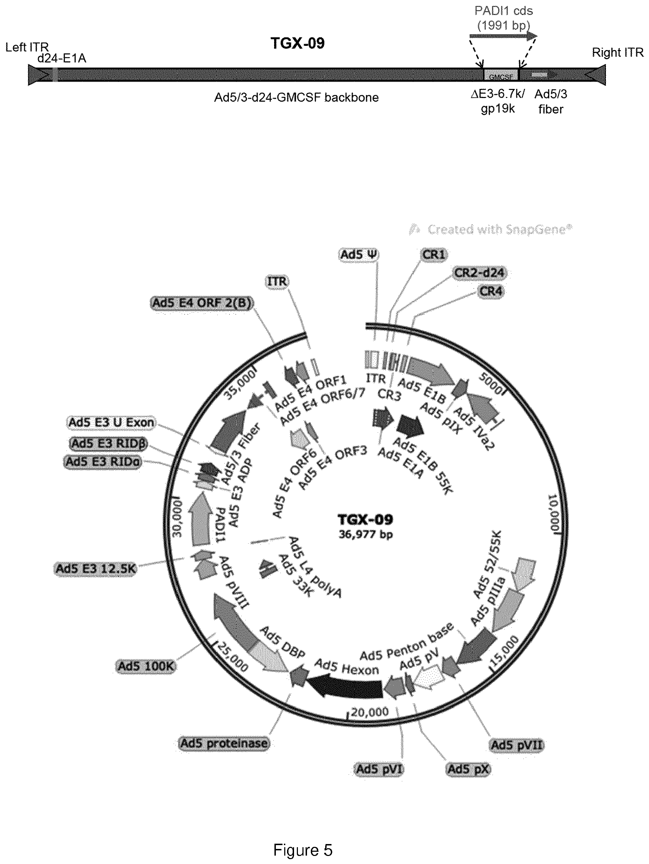

Figure 5 shows TGX-09 virus structure. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,050 - 33,813bp) and codes for the functional PADI1 protein (functional expression cassette, location: 28,371 - 30,362bp).

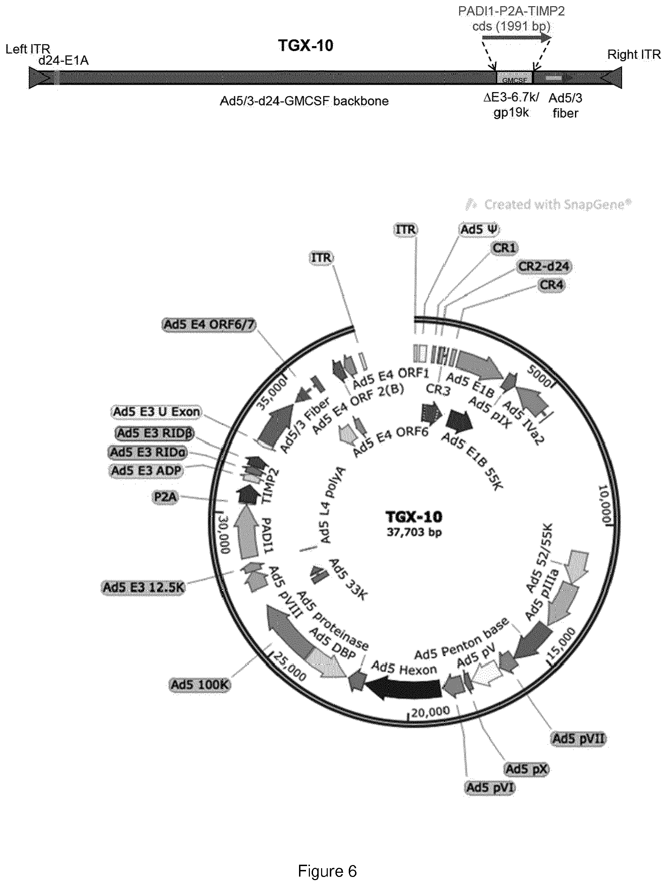

Figure 6 shows TGX-10 virus structure. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,776 - 34,539bp) and codes for the functional PADI1 (functional expression cassette, location: 28,371 - 30,359bp) and TIMP-2 protein (functional expression cassette, location: 30,426 - 31,088bp) connected by P2A sequence.

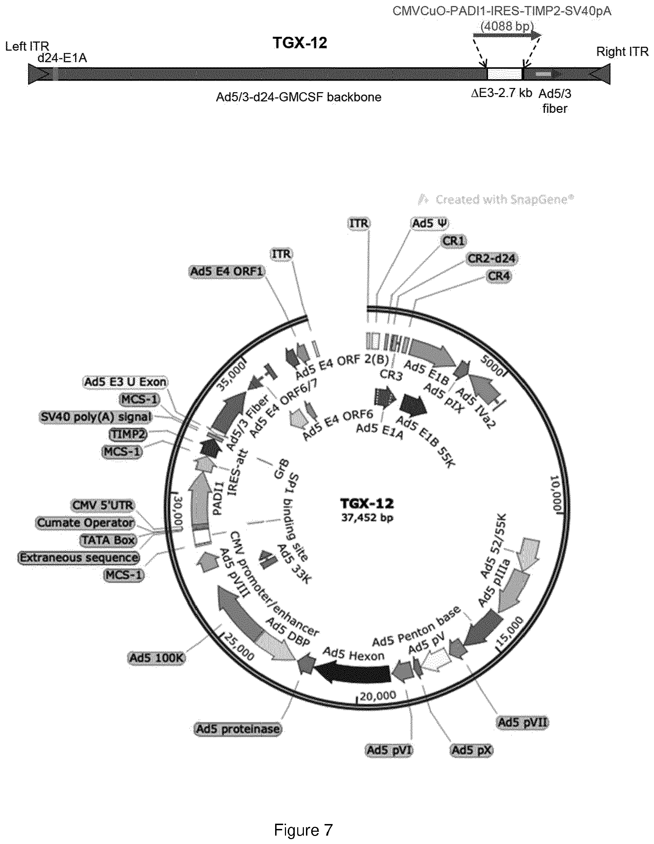

Figure 7 shows TGX-12 virus structure. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,525-34,288bp) and codes for the functional PADI1 (functional expression cassette, location: 28,904-30,895bp) and TIMP-2 protein (functional expression cassette, location: 31,506-32,163bp)-SV40pA connected by IRES sequence under CMC(CuO) promoter.

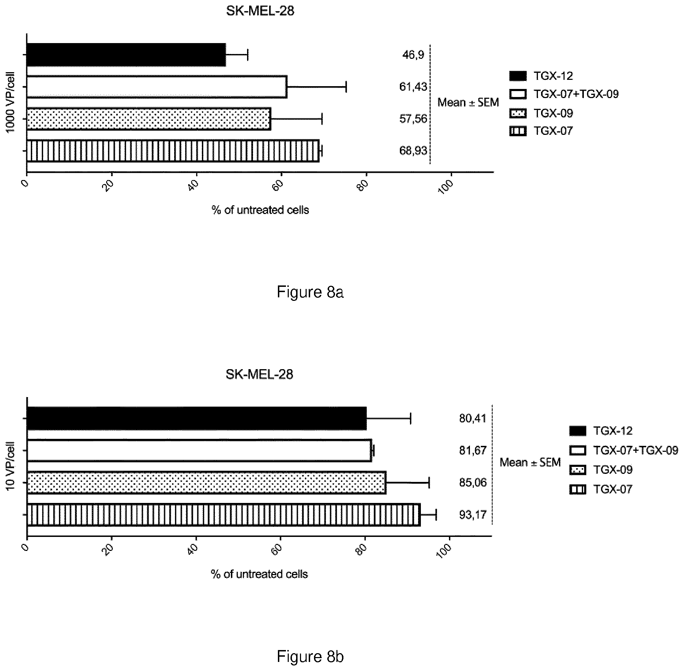

Figure 8. Figure 8 a) shows the effects of tested oncolytic adenoviruses on viability of human melanoma cell line Sk-Mel-28 at the concentration of 1000 VP/cell at 72 hours post infection. Absorbance of untreated cells is expressed as 100% and absorbance of treated cells is expressed as a percentage of absorbance of untreated cells. Results are expressed as mean +/- SEM. Figure 8 b) illustrates the effects of tested oncolytic adenoviruses on viability of human melanoma cell line Sk-Mel-28 at the concentration of 10 VP/cell after 72 hours post infection. Absorbance of untreated cells is expressed as 100% and absorbance of treated cells is expressed as a percentage of absorbance of untreated cells. Results are expressed as mean +/- SEM.

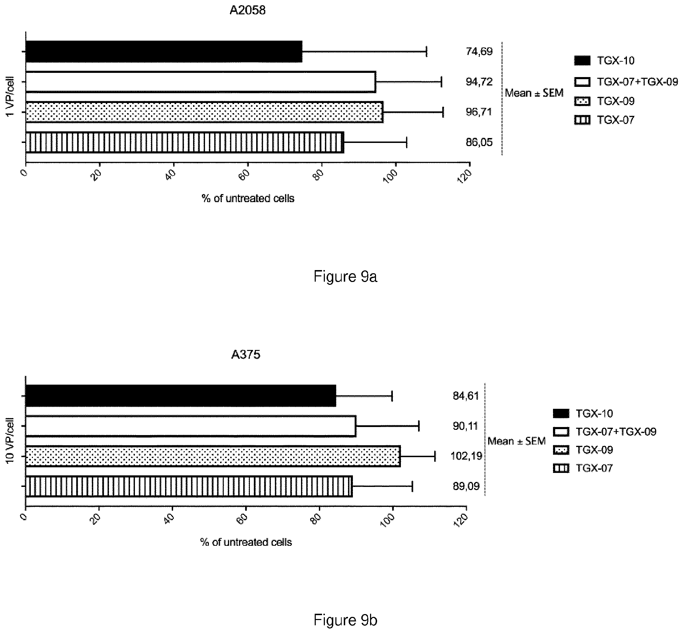

Figure 9. Figure 9a) shows the effects of tested oncolytic adenoviruses on viability of human melanoma cell line A2058 at the concentration of 1 VP/cell at 72 hours post infection. Absorbance of untreated cells is expressed as 100% and absorbance of treated cells is expressed as a percentage of absorbance of untreated cells. Results are expressed as mean +/- SEM. Figure 9 b) shows the effects of tested oncolytic adenoviruses on viability of human melanoma cell line A375 at the concentration of 10 VP/cell at 72 hours post infection. Absorbance of untreated cells is expressed as 100% and absorbance of treated cells is expressed as a percentage of absorbance of untreated cells. Results are expressed as mean +/- SEM.

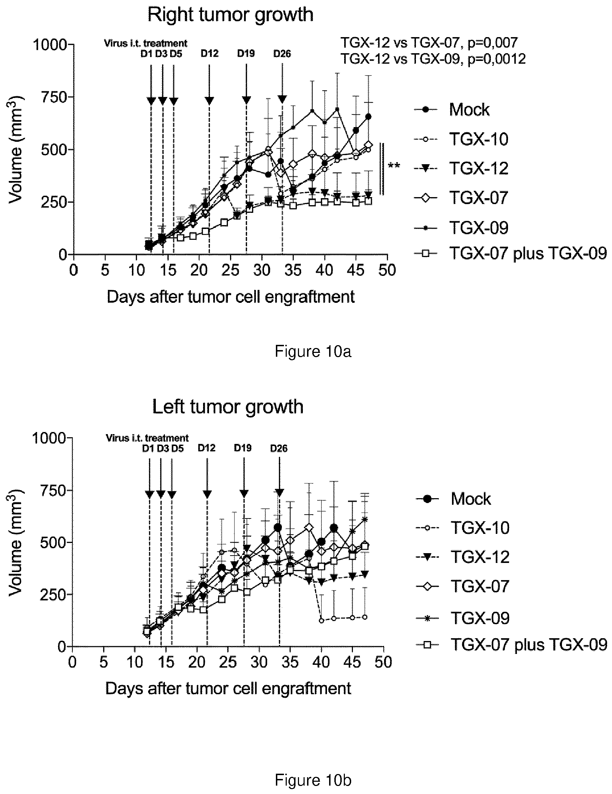

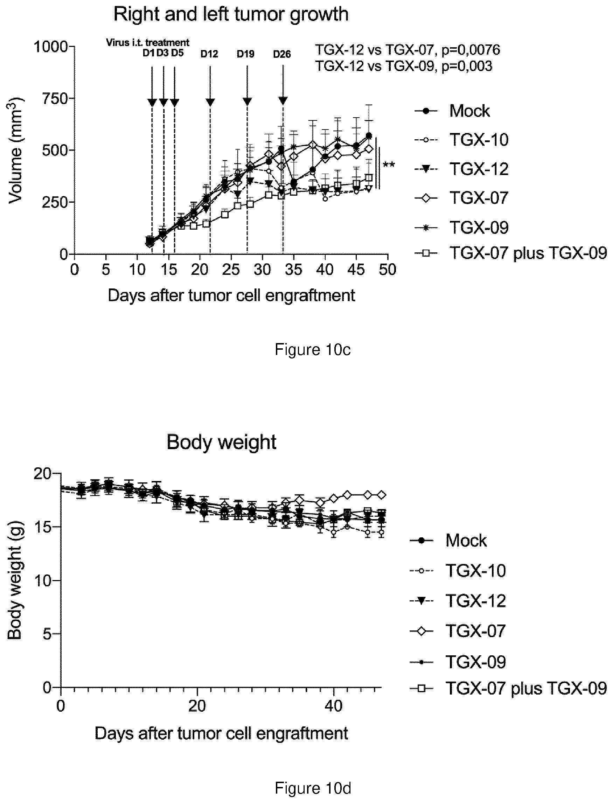

Figure 10. Figure 10 a) illustrates the effect of oncolytic viruses on A2058 right flank tumor growth in BALB/c nude model. Results are expressed as mean +/- SEM. n=1-8 per group. Tumor volume difference is shown from Day 12 to Day 47. Figure 10 b) shows the effect of oncolytic viruses on A2058 left flank tumor growth in BALB/c nude model. Results are expressed as mean +/- SEM. n=1-8 per group. Tumor volume difference is shown from Day 12 to Day 47. Figure 10 c9 shows the effect of oncolytic viruses on A2058 right and left flanks tumor growth in BALB/c nude model. Results are expressed as mean +/- SEM. n=1-8 per group. Tumor volume difference is shown from Day 12 to Day 47. Figure 10 d) shows the body weight difference over time, after tumor cell engraftment. n=1-8 per group, results represent mean +/- SEM. Body weight difference is shown from Day 12 to Day 47.

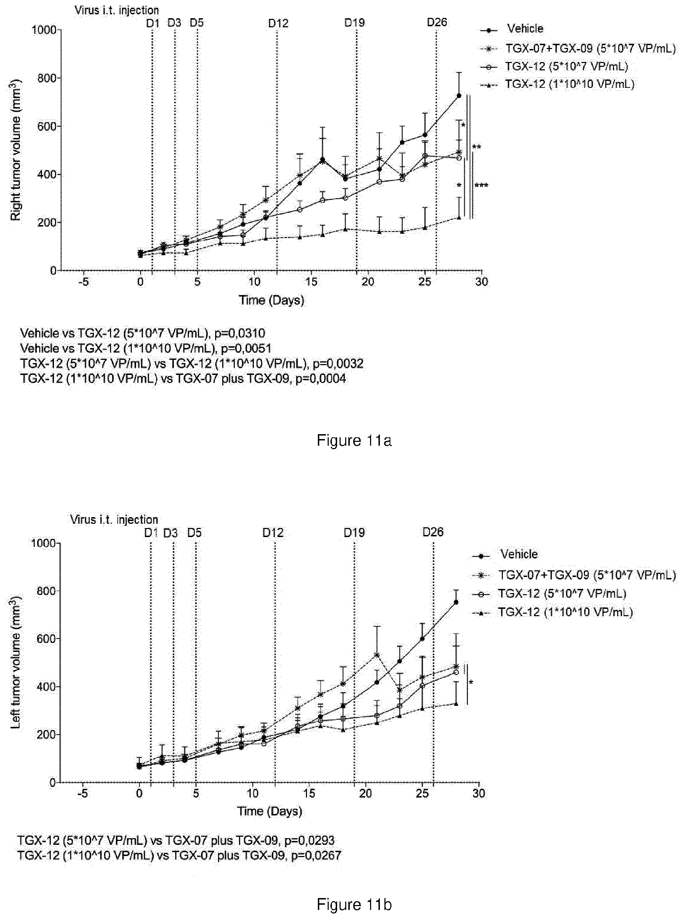

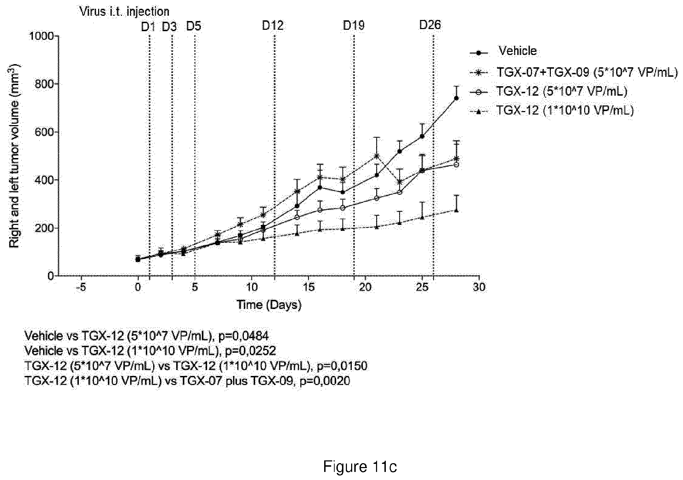

Figure 11. Figure 11 a) shows the effect of oncolytic viruses on A2058 right flank tumor growth in humanized melanoma mouse model. Results are expressed as mean +/- SEM. n=1-8 per group. Tumor volume difference is shown from Day 12 to Day 28. Figure 11 b) illustrates the effect of oncolytic viruses on A2058 left flank tumor growth in humanized melanoma mouse model. Results are expressed as mean +/- SEM. n=1-8 per group. Tumor volume difference is shown from Day 12 to Day 28. Figure 11 c) illustrates the effect of oncolytic viruses on A2058 right and left flank tumor growth in humanized melanoma mouse model. Results are expressed as mean +/- SEM. n=1-8 per group. Tumor volume difference is shown from Day 12 to Day 28.

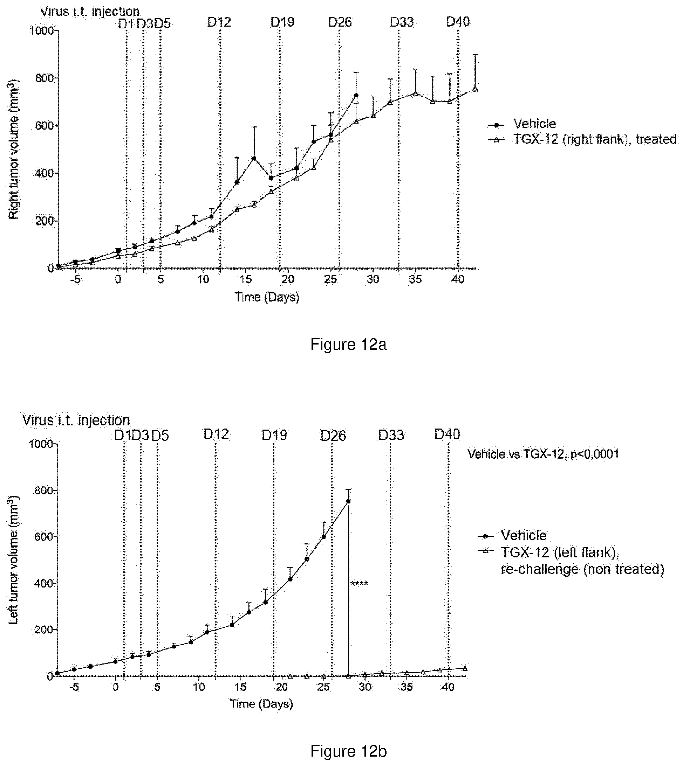

Figure 12. Figure 12 a) shows the results of an abscopal effect study. Effect of oncolytic viruses on A2058 right flank tumor growth (treated flank) in humanized melanoma mouse model. Results are expressed as mean +/- SEM. n=1-6 per group. Tumor volume difference is shown from Day 12 to Day 42. Figure 12 b) shows the results of an abscopal effect study. Effect of oncolytic viruses on A2058 left flank tumor growth (non-treated flank) in humanized melanoma mouse model. Results are expressed as mean +/- SEM. n=1-6 per group. Tumor volume difference is shown from Day 12 to Day 42.

### SEQUENCE LISTING

Sequence ID NO: 1. A nucleic acid sequence encoding an oncolytic adenoviral vector Ad5/3-D24-CMV(CuO)PADI1-IRES-TIMP2-SV40pA (TGX-12) comprising the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,776 - 34,539bp) and codes for the functional PADI1 (functional expression cassette, location: 28,371 - 30,359bp) and TIMP-2 protein (functional expression cassette, location: 30,426 - 31,088bp).
Sequence ID NO: 2 A nucleic acid sequence encoding an oncolytic adenoviral vector Ad5/3-D24-PADI1-P2A-TIMP2 (TGX-10) The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,776 - 34,539bp) and codes for the functional PADI1 (functional expression cassette, location: 28,371 - 30,359bp) and TIMP-2 protein (functional expression cassette, location: 30,426 - 31,088bp).
Sequence ID NO: 3. A nucleic acid sequence encoding an oncolytic adenoviral vector Ad5/3-D24-PADI1 (TGX-09) comprising the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,050 - 33,813bp) and codes for the functional PADI1 protein (functional expression cassette, location: 28,371 - 30,362bp).
Sequence ID NO: 4. A nucleic acid sequence encoding an oncolytic adenoviral vector Ad5/3-D24-TIMP2 (TGX-07), comprising the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 30,721 - 32,484bp) and codes for the functional TIMP2 protein (functional expression cassette, location: 28,371 - 29,033bp).
Sequence ID NO: 5. A binding site for Rb protein in E1A.
Sequence ID NO: 6. CR2-D24 (TGX-07).
Sequence ID NO: 7. TIMP2 sequence (TGX-07).
Sequence ID NO: 8. Ad5/3 fiber (TGX-07).
Sequence ID NO: 9. CR2-D24 (TGX-09).
Sequence ID NO: 10. Ad5/3 fiber (TGX-09).
Sequence ID NO: 11. PADI1 (TGX-09).
Sequence ID NO: 12. CR2-D24 (TGX-10).
Sequence ID NO: 13. Ad5/3 fiber (TGX-10).
Sequence ID NO: 14. PADI1 (TGX-10).
Sequence ID NO: 15. P2A (TGX-10).
Sequence ID NO: 16. TIMP2 (TGX-10).
Sequence ID NO: 17. CR2-D24 (TGX-12).
Sequence ID NO: 18. Ad5/3 fiber (TGX-12).
Sequence ID NO: 19. CMV promoter/enhancer (TGX-12).
Sequence ID NO: 20. CuO (TGX-12).
Sequence ID NO: 21. CMV 5'UTR (TGX-12).
Sequence ID NO: 22. PADI1 (TGX-12).
Sequence ID NO: 23. IRES (TGX-12).
Sequence ID NO: 24. TIMP2 (TGX-12).
Sequence ID NO: 25. SV40pA (TGX-12).
Sequence ID NO: 26. Primer sequence 282-13.
Sequence ID NO: 27. Primer sequence 282-14.
Sequence ID NO: 28. Primer sequence 282-15.
Sequence ID NO: 29. Primer sequence 282-16.
Sequence ID NO: 25. Primer sequence 282-21.
Sequence ID NO: 25. Primer sequence 282-22.
Sequence ID NO: 25. Primer sequence 282-23.
Sequence ID NO: 25. Primer sequence 282-24.
Sequence ID NO: 25. Primer sequence 282-25.
Sequence ID NO: 25. Primer sequence 282-42.

### DETAILED DESCRIPTION OF THE DISCLOSURE

The double transgene oncolytic adenoviral vector of the present invention is based on an adenovirus serotype 5 (Ad5) nucleic acid backbone with a 24 bp deletion (D24) in the Rb binding constant region 2 (CR2) of adenoviral E1 and a nucleic acid sequence encoding a peptidylarginine deiminase and a nucleic acid sequence encoding a TIMP in the place of the deleted 2.7kbp in the adenoviral E3 region. In a preferred embodiment of the invention, the adenoviral vector backbone is ONCOS-102 as disclosed in EP2379586B1 but comprising of a nucleic acid sequence encoding peptidylarginine deiminase and a nucleic acid sequence encoding TIMP in the place of GM-CSF/E3 region. These sequences can be connected via IRES or P2A sequence.

Peptidylarginine deiminase can be PADI1, PADI2, PADI3 or PADI4. Preferably, peptidylarginine deiminase is PADI1. More preferably, peptidylarginine deiminase is human PADI1. TIMP can be TIMP1, TIMP2, TIMP3 or TIMP4. Preferably, TIMP is human TIMP2. More preferably, TIMP is human TIMP2.

The results of the experiments showed superior effect of double transgene viruses. In *in vitro* cell viability assay (MTS), double transgene virus coding for PADI1 and TIMP2 (TGX-12) showed anti-cancer superiority over singe transgene viruses TGX-07 (coding for TIMP-2) and TGX-09 (coding for PADI1). Importantly, the double transgene virus showed stronger anticancer effect than the combinatory therapy of TGX-07 plus TGX-09 (Figure 8a). Sign of double transgene anti-cancer superiority was also reported at lower concentration, where double transgene exhibited stronger anti-cancer effect over single transgene viruses (Figure 8b). Similarly, the second construct coding for double transgenes (TGX-10) exhibited stronger anti-cancer effect than single transgene viruses TGX-07, TGX-09 and combinatory treatment (Figures 9a and 9b).

The combination of single transgene viruses TGX-07 and TGX-09 showed the best anticancer properties in terms of tumor volume reduction comparing to mock and single transgene viruses (Figure 10c). In addition, mice treated with TGX-12 and TGX-07 plus TGX-09 maintained the right tumor volumes below 500 mm3 for all animals until the end of the experiment (Figure 10a).

Surprisingly, double transgene viruses TGX-10 and TGX-12 along with TGX-07 plus TGX-09 viruses showed synergistic effect as they were able to reduce the tumor volume to a greater extent rather than viruses expressing single transgenes TGX-07 and TGX-09 (Table 2). Also, dose-dependent effect of TGX-12 was observed, as mice treated with higher concentration had greater tumor reduction and anti-cancer superiority was observed for the double transgene virus (Figure 11a, 11b, 11c). Surprisingly, mice treated with TGX-12 into only right flank showed abscopal effect as the re-challenged tumor on the left flank exhibited inhibited, low kinetic growth (Figures 12a and 12b), which is most likely due to development of protective immune memory cells.

Compared to a wild type adenovirus genome, the adenoviral vector of the invention lacks 24 base pairs from CR2 in E1 region, specifically in E1A region, and 2.7kbp deletion in E3 region. 24 base pair deletion (D24) of E1 affects CR2 domain, which is responsible for binding the Rb tumor suppressor/cell cycle regulator protein and thus, allows the induction of the synthesis (S) phase i.e. DNA synthesis or replication phase. pRb and E1A interaction requires eight amino acids 121 to 127 of the E1A protein conserved region, which are deleted in the present invention. Viruses with the D24 are known to have a reduced ability to overcome the G1-S checkpoint and replicate efficiently in cells where this interaction is not necessary, e.g. in tumor cells defective in the Rb-p16 pathway.

The E3 region is nonessential for viral replication *in vitro,* but the E3 proteins have an important role in the regulation of host immune response i.e. in the inhibition of both innate and specific immune responses. The E3 deletion along with gp19k/6.7K deletion in E3 refers to a deletion of 2.7kb base pairs from the adenoviral E3A region. In a resulting adenoviral construct, both gp19k and 6.7K genes are deleted. The gp19k gene product is known to bind and sequester major histocompatibility complex I (MHC1) molecules in the endoplasmic reticulum, and to prevent the recognition of infected cells by cytotoxic T-lymphocytes. Since many tumors are deficient in MHC1, deletion of gp19k increases tumor selectivity of viruses {virus is cleared faster than wild type virus from normal cells but there is no difference in tumor cells). 6.7K proteins are expressed on cellular surfaces and they take part in downregulating TNF-related apoptosis inducing ligand (TRAIL) receptor 2.

An internal ribosome entry site, abbreviated IRES, is an RNA element that allows for translation initiation in a cap-independent manner, as part of the greater process of protein synthesis. In eukaryotic translation, initiation typically occurs at the 5' end of mRNA molecules, since 5' cap recognition is required for the assembly of the initiation complex.

Instead of these large IRES sequences, a much shorter, self-cleaving 2A peptide can be used. Virus-derived peptide sequences mediate a ribosome-skipping event enables generation of multiple separate peptide products from one mRNA. Peptides from porcine teschovirus-1 (P2A) facilitate expression of multiple transgenes.

In the present invention, the peptidylarginine deiminase and TIMP transgenes are placed into a 2.7kbp deleted part in the E3 region, under the E3 promoter. This restricts transgene expression to tumor cells that allow replication of the virus and subsequent activation of the E3 promoter. In another embodiment, peptidylarginine deiminase and TIMP are placed under CMV(Cuo) promoter. E3 promoter may be any exogenous or endogenous promoter known in the art, preferably endogenous promoter. In a preferred embodiment of the invention, a nucleic acid sequence encoding peptidylarginine deiminase and TIMP are under the control of the viral E3 promoter. CMV(CuO) promoter contains a copy of the cumate operator (CuO). In "regular" 293 cells and other cell lines, the CuO operator is inactive and transcription takes place from the CMV promoter. In 293-CymR cells, which express the CymR repressor, the CymR repressor binds onto the CuO operator, thereby preventing the transcription of the transgene. Preferably, the transgenes are PADI1 and TIMP2.

The vectors of the invention may also comprise other modifications than partial deletions of CR2 and E3 and insertion of peptidylarginine deiminase and TIMP sequences as mentioned above. In a preferred embodiment of the invention, all the other regions of the Ad5 vector are of a wild type. In another preferred embodiment of the invention, the E4 region is of a wild type. In a preferred embodiment of the invention, a wild type region is located upstream of the E1 region. "Upstream" refers to immediately before the E1 region in the direction of expression. E1 B region may also be modified in the vector of the invention.

As used herein, "Ad5/3 chimerism" of the capsid refers to a chimerism, wherein the knob part of the fiber is from Ad serotype 3, and the rest of the fiber is from Ad serotype 5.

Expression cassettes are used for expressing transgenes in a target, such as a cell, by utilizing vectors. As used herein, the expression "expression cassette" refers to a DNA vector or a part thereof comprising nucleotide sequences, which encode cDNAs or genes, and nucleotide sequences, which control and/or regulate the expression of said cDNAs or genes. Similar or different expression cassettes may be inserted to one vector or to several different vectors. Ad5 vectors of the present invention may comprise either several or one expression cassettes. However, only one expression cassette is adequate. In a preferred embodiment of the invention, the oncolytic adenoviral vector comprises at least one expression cassette. In a preferred embodiment of the invention, the oncolytic adenoviral vector comprises only one expression cassette.

A cell comprising the adenoviral vector of the invention may be any cell such as a eukaryotic ceil, bacterial cell, animal cell, human cell, mouse cell etc. A cell may be an *in vitro, ex vivo* or *in vivo* cell. For example, the cell may be used for producing the adenoviral vector *in vitro, ex vivo* or *in vivo,* or the cell may be a target, such as a tumor cell, which has been infected with the adenoviral vector.

In a method of producing peptidylarginine deiminase and TIMP transgenes in a cell, a vehicle comprising the vector of the invention is carried into a cell and furthermore peptidylarginine deiminase and TIMP transgenes are expressed and the protein is translated and secreted in a paracrine manner. "A vehicle" may be any viral vector, plasmid or other tool, such as a particle, which is able to deliver the vector of the invention to a target cell. Any conventional method known in the art can be used for delivering the vector to the cell.

Tumor specific immune response may be increased in a subject by the present invention. T helper and cytotoxic T cells and/or natural killer cells are stimulated, produced and targeted because of the construct properties.

The recombinant Ad5 vectors of the invention have been constructed for replication competence in cells, which have defects in the Rb-pathway, specifically Rb-p16 pathway. These defective cells include tumor cells in animals and humans. In a preferred embodiment of the invention, the vector is capable of selectively replicating in cells having defects in the Rb-pathway. As used herein "defects in the Rb-pathway" refers to mutations and/or epigenetic changes in any genes or proteins of the pathway. Due to these defects, tumor cells overexpress E2F and thus, binding of Rb by E1A CR2, that is normally needed for effective replication, is unnecessary.

Any cancers or tumors, including both malignant and benign tumors as well as primary tumors and metastasis may be targets of gene therapies. In a specific embodiment of the invention the cancer is any solid tumor. In a preferred embodiment of the invention, the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, Hp cancer, meninges cancer, mouth cancer, nerve cancer, palate cancer, parotid gland cancer, peritoneum cancer, pharynx cancer, pleural cancer, salivary gland cancer, tongue cancer and tonsil cancer.

A pharmaceutical composition of the invention comprises at least one type of the vectors of the invention. Furthermore, the composition may comprise at least two or three different vectors of the invention. In addition to the vector of the invention, a pharmaceutical composition may also comprise any other vectors, such as other adenoviral vectors, other therapeutically effective agents, any other agents such as pharmaceutically acceptable carriers, buffers, excipients, adjuvants, antiseptics, filling, stabilising or thickening agents, and/or any components normally found in corresponding products.

The pharmaceutical composition may be in any form, such as solid, semisolid or liquid form, suitable for administration. A formulation can be selected from a group consisting of, but not limited to, solutions, emulsions, suspensions, tablets, pellets and capsules.

The effective dose of vectors depends on at least the subject in need of the treatment, tumor type, location of the tumor and stage of the tumor. The dose may vary for example from about 10e8 viral particles (VP) to about 10e14 VP, preferably from about 5x10e9 VP to about 10e13 VP and more preferably from about 8x10e9 VP to about 10e12 VP. In one specific embodiment of the invention the dose is in the range of about 5x10e10 - 5x10e11 VP.

The vector or pharmaceutical composition of the invention may be administered to any eukaryotic subject selected from a group consisting of plants, animals and human beings, in a preferred embodiment of the invention, the subject is a human or an animal. An animal may be selected from a group consisting of pets, domestic animals and production animals.

Any conventional method may be used for administration of the vector or composition to a subject. The route of administration depends on the formulation or form of the composition, the disease, location of tumors, the patient, comorbidities and other factors. In a preferred embodiment of the invention, the administration is conducted through an intratumoral, intramuscular, intra-arterial, intravenous, intrapleural, intravesicular, intracavitary or peritoneal injection, or an oral administration.

Only one administration of oncolytic adenoviral vectors of the invention may have therapeutic effects. However, in a preferred embodiment of the invention, oncolytic adenoviral vectors or pharmaceutical compositions are administered several times during the treatment period. Oncolytic adenoviral vectors or pharmaceutical compositions may be administered for example from 1 to 10 times in the first 2 weeks, 4 weeks, monthly or during the treatment period. In one embodiment of the invention, administration is done three to seven times in the first 2 weeks, then at 4 weeks and then monthly. In a specific embodiment of the invention, administration is done four times in the first 2 weeks, then at 4 weeks and then monthly. The length of the treatment period may vary, and for example may last from two to 12 months or more.

The gene therapy of the invention is effective alone, but combination of adenoviral gene therapy with any other therapies, such as traditional therapy, may be more effective than either one alone. For example, each agent of the combination therapy may work independently in the tumor tissue, the adenoviral vectors may sensitize cells to chemotherapy or radiotherapy and/or chemotherapeutic agents may enhance the level of virus replication or effect the receptor status of the target cells. The agents of combination therapy may be administered simultaneously or sequentially.

As used herein, the term "immune checkpoint inhibitor" or "checkpoint inhibitor" refers to molecules that totally or partially reduce, inhibit, interfere with or modulate one or more checkpoint proteins. Checkpoint proteins regulate T-cell activation or function. Central to the immune checkpoint process are the cytotoxic T-lymphocyte-associated antigen 4 (CTLA-4) and programmed death 1 (PD-1) immune checkpoint pathways. The CTLA-4 and PD-1 pathways are thought to operate at different stages of an immune response. CTLA-4 is considered the "leader" of the immune checkpoint inhibitors, as it stops potentially autoreactive T cells at the initial stage of naive T-cell activation, typically in lymph nodes. The PD-1 pathway regulates previously activated T cells at the later stages of an immune response, primarily in peripheral tissues.

Inhibition of the immune checkpoint pathways has led to the approval of several new drugs: ipilimumab (anti-CTLA-4; Yervoy®), pembrolizumab (anti-PD-1; Keytruda®), and nivolumab (anti-PD-1; Opdivo®). Also, PD-L1 inhibitors, such as Atezolizumab (MPDL3280), Avelumab (MSB0010718C) and Durvalumab (MEDI4736), are available. These antagonistic antibodies have been associated with objective clinical responses in cancer patients. Antibodies targeting CTLA-4 are already marketed (e.g. Ipilimumab, Yervoy, Bristol-Myers Squibb, BMS) for metastatic melanoma. Antibody therapies with anti PD-L1 (e.g. MPDL3280A, Roche), anti PD-1 (e.g. Nivolumab, BMS) are also ongoing.

Other immune-checkpoint inhibitors include lymphocyte activation gene-3 (LAG-3) inhibitors, such as IMP321, a soluble Ig fusion protein. Other immune checkpoint inhibitors include B7 inhibitors, such as B7-H3 and B7-H4 inhibitors. In particular, the anti-B7-H3 antibody MGA271. Also included are TIM3 (T-cell immunoglobulin domain and mucin domain 3) inhibitors. In certain embodiments the PD-1 blockers include anti-PD-L1 antibodies. In certain other embodiments the PD-1 blockers include anti-PD-1 antibodies and similar binding proteins such as nivolumab (MDX 1106, BMS 936558, ONO 4538), a fully human IgG4 antibody 30 that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-L1 (B7-H1) blockade. Further examples of PD-L1 inhibitors that can be used in certain embodiments are Atezolizumab (MPDL3280), Avelumab (MSB0010718C) and Durvalumab.

Preferably, anti-PD-1 antibodies pembrolizumab (Keytruda), and nivolumab (Opdivo) are used in the invention. Also, PD-L1 inhibitors, such as durvalumab can be used in combination with anti-PD-1-antibodies. The preferred checkpoint inhibitors of the present invention are thus those for PD-1 and PD-L1.

In a preferred embodiment of the invention, the method or use further comprises administration of concurrent radiotherapy to a subject. In another preferred embodiment of the invention, the method or use further comprises administration of concurrent chemotherapy to a subject. As used herein "concurrent" refers to a therapy, which has been administered before, after or simultaneously with the gene therapy of the invention. The period for a concurrent therapy may vary from minutes to several weeks. Preferably the concurrent therapy lasts for some hours.

Agents suitable for combination therapy include but are not limited to All-trans retinoic acid, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imattnib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine.

In a preferred embodiment of the invention, the method or use further comprises administration of verapamil or another calcium channel blocker to a subject. "Calcium channel blocker" refers to a class of drugs and natural substances which disrupt the conduction of calcium channels, and it may be selected from a group consisting of verapamil, dihydropyridines, gallopamil, diltiazem, mibefradil, bepridil, fluspirilene and fendiline.

In a preferred embodiment of the invention, the method or use further comprises administration of autophagy inducing agents to a subject. Autophagy refers to a catabolic process involving the degradation of a cell's own components through the lysosomal machinery. "Autophagy inducing agents" refer to agents capable of inducing autophagy and may be selected from a group consisting of, but not limited to, mTOR inhibitors, P13K inhibitors, lithium, tamoxifen, chloroquine, bafilomycin, temsirolimus, sirolimus and temozolomide. In a specific embodiment of the invention, the method further comprises administration of temozolomide to a subject. Temozolomide may be either oral or intravenous temozolomide.

In one embodiment of the invention, the method or use further comprises administration of chemotherapy or anti-CD20 therapy or other approaches for blocking of neutralizing antibodies. "Anti-CD20 therapy" refers to agents capable of killing CD20 positive cells and may be selected from a group consisting of rituximab and other anti-CD20 monoclonal antibodies. "Approaches for blocking of neutralizing antibodies" refers to agents capable of inhibiting the generation of anti-viral antibodies that normally result from infection and may be selected from a group consisting of different chemotherapeutics, immunomodulatory substances, corticoids and other drugs. These substances may be selected from a group consisting of, but not limited to, cyclophosphamide, cyclosporin, azathioprine, methylprenisolone, etoposide, CD40L, CTLA4Ig4, FK506 (tacrolismus), IL-12, IFN-gamma, interleukin 10, anti-CD8, anti-CD4 antibodies, myeloablation and oral adenoviral proteins.

The oncolytic adenoviral vector of the invention induces virion mediated oncolysis of tumor cells and activates human immune response against tumor cells. In a preferred embodiment of the invention, the method or use further comprises administration of substances capable to downregulating regulatory T-cells in a subject. "Substances capable to down- regulating regulatory T-cells" refers to agents that reduce the number of cells identified as T-suppressor or Regulatory T-cells. These cells have been identified as consisting one or many of the following immunophenotypic markers: CD4+, CD25+, FoxP3+, CD127- and GITR+. Such agents reducing T- suppressor or Regulatory T-cells may be selected from a group consisting of anti-CD25 antibodies or chemotherapeutics.

An object of the invention is an oncolytic adenoviral vector comprising an adenovirus serotype 5 (Ad5) nucleic acid backbone, a 24 bp deletion (D24) in the Rb binding constant region 2 of E1, a nucleic acid sequence encoding peptidylarginine deiminase and a nucleic acid sequence encoding TIMP in the E3 region. The sequences of peptidylarginine deaminase and TIMP can be connected via IRES. According to another embodiment, they can be connected via P2A. Preferably, the sequences of peptidylarginine deaminase and TIMP are located in a gp19k/6.7K or 2.7kbp deletion in the E3 region. Preferably, said vector contains an adenovirus 3 serotype knob replacing the wild type serotype 5 knob (5/3 chimeric). According to a more preferred object of the invention, peptidylarginine deiminase and TIMP are placed under E3 promoter. According to a still more preferred object of the invention, peptidylarginine deiminase and TIMP are placed under CMV(Cuo) promoter. According to one preferred aspect of the invention, the oncolytic adenoviral vector according comprises a native E1 A promoter.

Preferably, peptidylarginine is PADI1 and TIMP is TIMP2. More preferably, peptidylarginine is human PADI1 and TIMP is human TIMP2.

Preferably, peptidylarginine deiminase and TIMP are connected with IRES or P2A. Another object of the invention is this oncolytic adenoviral vector further comprising an Ad5/3 chimerism as a capsid modification.

The nucleotide sequences of vectors show at least 66%, preferably at least 70%, more preferably at least 75%, even more preferably at least 80% identity to SEQ ID NO: 1, SEQ ID NO: 2, SEQ ID NO:3 or SEQ ID NO:4. Still more preferably the nucleotide sequences show at least 85% or at least 90% or 95%, more preferably at least 98%, most preferably 99% identity to the nucleic acid sequence of SEQ ID NO: 1, SEQ ID NO: 2, SEQ ID NO:3 or SEQ ID NO:4.

A further object of the invention is a method of treating cancer in a human subject in need thereof, wherein a therapeutically effective amount of a pharmaceutical formulation comprising an oncolytic adenovirus comprising nucleotide sequence having at least 70% identity to SEQ ID NO: 1 or SEQ ID NO:2 and a diluent or carrier is administered. In the most preferred embodiment the nucleotide sequence is SEQ ID NO: 1 or SEQ ID NO: 2. Another object of the invention is a method, wherein a therapeutically effective amount of a pharmaceutical formulation comprising a combination of oncolytic adenovirus comprising a nucleotide sequence having at least 70% identity to SEQ ID NO: 3 and oncolytic adenovirus comprising a nucleotide sequence having at least 70% identity to SEQ ID NO: 4 is administered. In the most preferred embodiment, the combination of adenoviruses has nucleotide sequences according to SEQ ID NO: 3 and SEQ ID NO:4.

One object of the invention is the oncolytic adenoviral vector comprising at least one expression cassette. In another object of the invention, the oncolytic adenoviral vector is capable of selectively replicating in cells having defects in the Rb-pathway.

Also, a cell comprising the adenoviral vector is one aspect of the invention.

Another aspect of the invention is a pharmaceutical composition comprising the adenoviral vector of the invention. The adenoviral vector for use in the treatment of cancer or in the manufacture of a medicament for treating cancer in a subject is also an aspect of the invention. The cancer can be selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

The subject of the treatment can be a human or an animal.

The treatment of cancer with adenoviral vector in a subject can be conducted through an intratumoral, intramuscular, intra-arterial, intravenous, intrapleural, intravesicular, intracavitary or peritoneal injection, or an oral administration. The injection or the administration can be conducted several times during the treatment period. Also, an oncolytic adenoviral vector having a different fiber knob of the capsid compared to the vector of the earlier treatment can be used. The treatment of cancer in a subject can further comprise an administration of concurrent radiotherapy and/or concurrent chemotherapy to a subject. The treatment of cancer in a subject can further comprise an administration of a checkpoint inhibitor, verapamil or another calcium channel blocker, autophagy inducing agents, temozolomide, chemotherapy or anti-CD20 therapy or other approaches for blocking of neutralizing antibodies, and/or substances capable to downregulating regulatory T-cells in a subject.

A method of producing peptidylarginine deiminase and TIMP in a cell *in vitro,* wherein the method comprises:
a) carrying a vehicle comprising an oncolytic adenoviral vector to a cell, and
b) expressing peptidylarginine deiminase and TIMP of said vector in the cell, is an aspect of the present invention.

Also, a use of the oncolytic adenoviral vector for producing peptidylarginine deiminase and TIMP2 in a cell *in vitro* is an aspect of the invention.

A further object of the invention is a method of treating cancer in a human subject in need thereof, wherein a therapeutically effective amount of a pharmaceutical formulation comprising an oncolytic adenovirus comprising SEQ ID NO: 1 or SEQ ID NO:2 and a diluent or carrier is administered. Another object of the invention is a method, wherein a therapeutically effective amount of a pharmaceutical formulation comprising a combination of oncolytic adenovirus comprising SEQ ID NO: 3 and oncolytic adenovirus comprising SEQ ID NO: 4 is administered. Said methods can further comprise administering an effective amount of an oncolytic adenoviral vector or a pharmaceutical composition comprising the oncolytic adenoviral vector to the subject through intratumoral injection, wherein the oncolytic adenoviral vector comprises the adenovirus according to the invention. In said methods the effective amount of the oncolytic adenoviral vector contains between 5X10e10 - 5X10e11 viral particles. They also induce a specific antitumor immune response, including activation of CD8+ T cells, in tumors expressing the peptidylarginine deiminase and TIMP from the oncolytic adenoviral vector. In said methods for treatment, the oncolytic adenoviral vector can be administered three to seven times in the first two weeks of treatment.

In said treatment methods cancer can be selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

According to one embodiment, the administration of the adenoviral vector or vectors or pharmaceutical composition can be conducted through an intratumoral, intramuscular, intra-arterial, intravenous, intrapleural, intravesicular, intracavitary or peritoneal injection, or an oral administration. According to another embodiment, the adenoviral vector or vectors or pharmaceutical composition can be administered several times during a treatment period. According to a preferred embodiment, concurrent radiotherapy can be administered to the subject. According to a more preferred embodiment, concurrent chemotherapy can be administered to the subject. According to a still more preferred embodiment verapamil or another calcium channel blocker can be administered to the subject.

In one preferred object of the invention, is a method for treatment with the adenoviral vector or vectors further comprising administering substances capable to downregulating regulatory T-cells in the subject.

Any method or use of the invention may be either *in vivo, ex vivo* or *in vitro* method or use.

The present invention is illustrated by the following examples, which are not intended to be limiting in any way.

### EXAMPLES

### Cloning of oncolytic viruses

Oncolytic viruses were constructed using genetic engineering tools. The construction of oncolytic vectors included the following steps:

### 1. Shuttle Plasmid Constructions:

For TGX-07/09/10, the transgenes were inserted into the E3 region at the same location as the GM-CSF coding sequence in vector Ad5/3-D24-GMCSF (see B2379586B1). In that vector, a 0.9 kb BsiWI-Mfel fragment encompassing the E3 6.7K ORF and gp19K ORF was deleted and replaced by the GM-CSF coding sequence. For this cloning, a shuttle plasmid pAd1129-14, which contains the WT E3 region and a hybrid Ad5/3 fiber, was used. The TIMP2, PADI1, PADI1-P2A-TIMP2 sequences were inserted between the BsiWI and Mfel site of pAd1129-14 using standard cloning methods (Figure 1). The identity of the shuttle plasmids was verified by restriction analysis and sequencing.

For TGX-12, the dicistronic cassettes CMVCuO-PADI1-IRES-TIMP2-SV40pA was inserted in place of the E3 region deleted for most of its genes. Shuttle plasmid pAd1129-27, which contains the Ad5 E3 region and a hybrid Ad5/3 fiber gene, was used. A 2.7 kb BgIII fragment was deleted from the E3 region and replaced with a multiple cloning site. Both dicistronic cassettes were inserted into pAd1129-27 using standard cloning methods (Figure 2). The identity of the plasmids was verified by restriction analysis and sequencing.

### 2. Cosmid Construction

The entire genomes of the recombinant adenoviruses were reconstituted in cosmids (Figure 3). Transfection-grade cosmid DNAs were purified and their identity was confirmed by restriction analysis.

### 3. Virus rescue and characterization

Excision of the adenovirus genome from the cosmids obtained in step 2, and transfection into 293 or 293-Cymr or A549 cells. Three virus plaques per construct were harvested and amplified in small scale. Their identity will be verified by restriction analysis of their genome using 3 enzymes (Hirt supernatant).

### 4. Western Blot

Expression of the various transgenes from the viruses were assessed by infecting a reporter cell line with the various virus clones, lysing the cells 24 hours post-infection, separating the total protein content by PAGE, and performing western blottings (WB), anti-TIMP2, and anti-PADI1 antibodies to detect the various transgene products by chemiluminescence. These experiments will enable the selection of virus clones for amplification and purification.

### 5. Small-scale amplification

Small-scale amplification of the viral clones, purification on two CsCI gradients, dialysis against an isotonic buffer (GTS buffer: 2.5% glycerol, 25 mM NaCl, 20 mM Tris-HCI, pH 8.0), filtration through a low-protein binding 0.22 µm filter, and virus particle (VP) concentration determination by ultraviolet absorbance (Abs260-SDS method) was carried out.

### 6. Confirmation of virus identity by sequencing

The identity of the purified recombinant adenoviruses was confirmed by sequencing regions of the virus genomes specific for these recombinant viruses, including the 24-bp deletion in the E1A CDS, the entire mono- or dicistronic cassettes inserted into the E3 region, the Ad5/3 hybrid fiber, and the junctions between the DNA fragments ligated to each other during the cosmid construction. Experimental sequences were assembled into contigs and aligned with the expected sequences of the viruses.

Viral genomic DNAs were extracted from CsCl-purified virus particles using proteinase K and resuspended into 30 µL TE pH 7.5. Their quality was assessed by spectrophotometry (Abs260/280/320 nm) and agarose gel electrophoresis. TGX viruses' DNAs were sent to a third party for shotgun library making and sequencing. Libraries were generated using a Nextera XT kit (Illumina, Inc). They were pooled, quantified by qPCR, and sequenced on a 2x300 MiSeq run (Illumina, Inc) .fastq files were processed using the program "BWA Aligner". The software Geneious v11.1.5 (Biomatters Ltd) was used to visualize the .bam files and calculate the consensus sequences. The consensus sequences were aligned to the predicted sequences of the virus genomes using the software SnapGene v4.2.6.

### 7. Titration of the viral suspension

Titration of the viral suspension: determination of the concentration of infectious units per mL virus suspension was performed by TCID50 assay.

### Cloning of TGX-07 (Ad5/3-D24-TIMP2) in more detail

To construct, amplify, purify, titrate and characterize an oncolytic adenovirus vector characterized by the following features:
- 24-bp deletion in E1A CR2
- a sequence encoding TIMP2 embedded into the E3 region
- a hybrid Ad5/3 fiber

Recombinant adenovirus TGX07 was constructed by reconstituting its entire genome in a cosmid (pTGX07). The following 3 DNA fragments were ligated to each other:
1. The 27 kb Pacl-Spel fragment from pAd5/3-d24-WTE3
2. The 8587 bp Spel-Pacl fragment from pTGX106
3. The 8 kb Pacl fragment from pAd5dSfi

Where:
- pAd5/3-d24-WTE3 (pAd5/3-d24-GMCSF)
- pTGX106 is a plasmid that contains the right end of the virus genome (Spel-Pacl sequence from pAd5/3.d24-WTE3) with the TIMP2 coding sequence inserted between the BsiWI and Mfel sites in the E3 region
- pAd5dSfil is a cosmid that contains the entire Ad5 genome. The 8 kb Pacl fragment provides the cosmid backbone for pTGX07
pTGX106 was constructed by inserting the 824 bp BsiWI-Notl DNA fragment from pAd1129-TIMP2 between the corresponding sites of pTGX101:
Where:
- pTGX101 is a plasmid that contains the right end of the Ad5/3.d24-WTE3 genome (Spel-Pacl fragment from pAd5/3.d24-WTE3)
- pAd1129-TIMP2 is a shuttle plasmid that contains the TIMP2 coding sequence between the BsiWI and Mfel site in the E3 region
pAd1129-TIMP2 was constructed by inserting a 684 bp synthetic dsDNA encoding TIMP2 between the BsiWI and Mfel sites of pAd1129-14

Where:
- pAd1129-14 is a shuttle plasmid containing the WT Ad5 E3 region and a hybrid Ad5/3 fiber
- gBlock-TIMP2 (GenBank # NM_003255.4)

### Cloning of TGX-09 (Ad5/3-D24-PADI1) in more detail

To construct, amplify, purify, titrate and characterize an oncolytic adenovirus vector characterized by the following features:
- 24-bp deletion in E1A CR2
- a sequence encoding PADI1 embedded into the E3 region
- a hybrid Ad5/3 fiber

Recombinant adenovirus TGX09 was constructed by reconstituting its entire genome in a cosmid (pTGX09). The following 3 DNA fragments were ligated to each other:
1. The 27 kb Pacl-Spel fragment from pAd5/3-d24-WTE3
2. The 9916 bp Spel-Pacl fragment from pTGX107
3. The 8 kb Pacl fragment from pAd5dSfi

Where:
- pAd5/3-d24-WTE3 (pAd5/3-d24-GMCSF)
- pTGX107 is a plasmid that contains the right end of the virus genome (Spel-Pacl sequence from pAd5/3.d24-WTE3) with the PADI1 coding sequence inserted between the BsiWI and Mfel sites in the E3 region
- pAd5dSfil is a cosmid that contains the entire Ad5 genome. The 8 kb Pacl fragment provides the cosmid backbone for pTGX09
pTGX107 was constructed by inserting the 2153 bp BsiWI-Notl DNA fragment from pAd1129-PADI1 between the corresponding sites of pTGX101:
Where:
- pAd1129-14 is a shuttle plasmid containing the WT Ad5 E3 region and a hybrid Ad5/3 fiber
- gBlock-PADI1 (GenBank # AB033768.2)

### Cloning of TGX-10 (Ad5/3-D24-PADI1-TIMP2) in more detail

To construct, amplify, purify, titrate and characterize an oncolytic adenovirus vector characterized by the following features:
- 24-bp deletion in E1A CR2
- a dicistronic cassette expressing PADI1 and TIMP2 embedded into the E3 region
- a hybrid Ad5/3 fiber

Recombinant adenovirus TGX10 was constructed by reconstituting its entire genome in a cosmid (pTGX10). The following 3 DNA fragments were ligated to each other:
1. The 27 kb Pacl-Spel fragment from pAd5/3-d24-WTE3
2. The 10642 bp Spel-Pacl fragment from pTGX109b
3. The 8 kb Pacl fragment from pAd5dSfi

Where:
- pAd5/3-d24-WTE3 (pAd5/3-d24-GMCSF)
- pTGX109b is a plasmid that contains the right end of the virus genome (Spel-Pacl sequence from pAd5/3.d24-WTE3) with the PADI1-P2A-TIMP2 cassette inserted between the BsiWI and Mfel sites in the E3 region
- pAd5dSfil is a cosmid that contains the entire Ad5 genome. The 8 kb Pacl fragment provides the cosmid backbone for pTGX10
pTGX109b was constructed by inserting the 2879 bp BsiWI-Notl DNA fragment from pTGX109 between the corresponding sites of pTGX101:
Where:
- pTGX101 is a plasmid that contains the right end of the Ad5/3.d24-WTE3 genome (Spel-Pacl fragment from pAd5/3.d24-WTE3)
- pTGX109 is a plasmid that contains the PADI-P2A-TIMP2 cassette between the BsiWI and Mfel site in the Ad5 E3 region
pTGX109 was constructed by ligating the following 4 DNA fragments to each other:
1. The 4143 bp BsiWI-Sfil fragment from pAd1129-TIMP2
2. The 1823 bp Sfil fragment from pAd1129-TIMP2
3. The 2536 bp Sfil-Mfel fragment from pAd1129-PADI1
4. A 106 bp PCR fragment encompassing a P2A element, obtained using annealed oligonucleotides (288-13 + 288-14) as template, and primers 288-15 and 288-16

Where:
- pAd1129-TIMP2 and pAd1129-PADI1 were made for the construction of viruses TGX07 and TGX09, respectively

### Primers:

288-13: GGCAGCGGCGCCACTAACTTCTCCCTGTTAAAGCAAGCAGGCGATGTT

GAAGAAAACCCCGGGCCT

288-14: AGGCCCGGGGTTTTCTTCAACATCGCCTGCTTGCTTTAACAGGGAGAA

GTTAGTGGCGCCGCTGCC

288-15: AATGGTGGAACATGGTGCCCGGCAGCGGCGCCACTAACTT
288-16: GTGCGGGCCGCGGCGCCCATAGGCCCGGGGTTTTCTTCAA

### Cloning of TGX-12 (Ad5/3-D24-PADI1-TIMP2) in more detail

To construct, amplify, purify, titrate and characterize an oncolytic adenovirus vector characterized by the following features:
- 24-bp deletion in E1A CR2
- a dicistronic cassette expressing PADI1 and TIMP2 in place of the E3 region (2.7 kb BgIII deletion)
- a hybrid Ad5/3 fiber

Recombinant adenovirus TGX12 was constructed by reconstituting its entire genome in a cosmid (pTGX12). The following 3 DNA fragments were ligated to each other:
1. The 27 kb Pacl-Spel fragment from pAd5/3-d24-WTE3
2. The 10391 bp Spel-Pacl fragment from pTGX111c
3. The 8 kb Pacl fragment from pAd5dSfi

Where:
- pAd5/3-d24-WTE3 (pAd5/3-d24-GMCSF)
- pTGX111c is a plasmid that contains the right end of the virus genome (Spel-Pacl sequence from pAd5/3.d24-WTE3) with the CMV-PADI1-IRES-TIMP2 cassette inserted in place of the E3 region.
- pAd5dSfil is a cosmid that contains the entire Ad5 genome. The 8 kb Pacl fragment provides the cosmid backbone for pTGX12
pTGX111c was constructed by ligating the following 3 DNA fragments to each other:
1. the 1349 bp Csil-Rsrll fragment from pTGX101
2. the 6784 bp Rsrll-Sphl fragment from pTGX101
3. the 4837 bp Sphl-Csil fragment from pTGX111b

Where:
- pTGX101 is a plasmid that contains the right end of the Ad5/3.d24-WTE3 genome (Spel-Pacl fragment from pAd5/3.d24-WTE3)
- pTGX111b is a plasmid that contains the CMV-PADI1-IRES-TIMP2-SV40pA signal cassette in place of the E3 region (2.7 kb BgIII deletion)
pTGX111b was constructed by digesting pTGX111 with Spel, filling-in the sticky ends with Klenow, and religating both fragments with each other, in order to destroy the Spel sites flanking the CMV-PADI1-IRES-TIMP2 cassette

Where:
- pTGX111 is a plasmid that contains the CMV-PADI1-IRES-TIMP2-SV40pA signal cassette in place of the E3 region (2.7 kb BgIII deletion)
pTGX111 was constructed by inserting the following 4 fragments between the Sacll and Acc65I sites of pAd1129-GrB-F:
1. the 2018 bp PADI1 coding sequence amplified by PCR using pAd1129-PADI1 as template and primers 288-22 and 288-42,
2. the 645 bp IRES-att element amplified by PCR using pAd1129-IRES-att as template and primers 288-23 and 288-24,
3. the 669 bp TIMP2 coding sequence isolated from plasmid pAd1129-TIMP2 by BsiWI/Mfel digestion
4. the 174 bp SV40pA signal amplified by PCR using pAd1129-GrB-F as template and primers 288-21 and 288-25

Where:
- pAd1129-PADI1 was made for the construction of TGX09
- pAd1129-TIMP2 was made for the construction of TGX07
- pAd1129-GrB-F was made for the construction of TGX01
pAd1129-IRES-att is a plasmid that contains an attenuated version of the EMCV Internal Ribosome Entry Site (IRES)

### Primers:

288-21: TAGTTAAAGGGAATAAGATCGGTACC
288-22: 
ACACCGGGACCGATCCAGCCTCCGCGGCCGCCACCATGGCCCCAAAGAGAGTTG
TGCAGC
288-23: GGTGGAACATGGTGCCCTGACCCCTCTCCCTCCCCCCCCT
288-24: GTGCGGGCCGCGGCGCCCATGTCGACTCTAGAGGATCCCG
288-25: TCGACATCGAGGACCCATAAAACTTGTTTATTGCAGCTTA
288-42: TCAGGGCACCATGTTCCACCATTTG

### Cell lines

Melanoma cell lines, A2058 (ATCC® CRL-1147™), A375 (ATCC® CRL-1619™) and SK-Mel-28 (ATCC® HTB-72™), were cultured in DMEM high glucose medium supplemented with 10% FCS and penicillin/streptomycin. Trypsin-EDTA was used to detach and subculture cells at 80% confluence for *in vitro* assays. Phosphate-buffered (PBS)-EDTA (10 mM) was used to detach A2058 melanoma cells before suspension of 20 x 10⁶ cells in isotonic PBS for subcutaneous injection.

### Data analyses

All parameters were analyzed using GraphPad Prism software (version 7). Statistical analyses have been performed using RM-one-way ANOVA followed by a Tukey post-test or T test (Mann Whitney Test). Therapeutic synergy was assessed with the FTV calculation method. FTV (mean tumor volume experimental)/(mean tumor volume control). Expected: (Mean FTV of TGX-07) x (mean FTV of TGX-09). Ratio: expected FTV by the observed FTV. A ratio of >1 indicates a synergistic effect, and a ratio of <1 indicates a less than additive effect.

### EXAMPLE 1 In vitro - cell viability assay (MTS)

Cell viability was evaluated by using the MTS Cell Proliferation Assay kit (Abcam) according to the manufacturer's instructions with the following modifications. MTS assay kit is a colorimetric method for the sensitive quantification of viable cells. It can be used to assess cell proliferation, cell viability and cytotoxicity. The MTS assay is based on the reduction of the MTS tetrazolium compound by viable mammalian cells to generate a colored formazan dye that is soluble in cell culture media. This conversion is thought to be carried out by NAD(P)H-dependent dehydrogenase enzymes in metabolically active cells. The formazan dye is quantified by measuring the absorbance at 490 nm. Cells were seeded in 96 well plate at a concentration of 3000 and 5000 cells/well. Treatment was initiated in a final volume of 200µL. Cells were incubated with and without 0.1, 1, 10, 100 and 1000 viral particles (VP) of tested virus or mix of viruses. 72 hours later, MTS assay was performed according to manufacturer protocol. The plates were shaken briefly on a shaker and absorbance measured at 490 nm. Absorbance values for untreated cells were set to correspond to 100% viability, and viability of treated cells was expressed as a percentage of the untreated control absorbance value.

In *in vitro* cell viability assay (MTS), double transgene virus coding for PADI1 and TIMP2 (TGX-12) showed anti-cancer superiority when administered at the concentration of 1000VP/cell (46,9 % of untreated cells) over singe transgene viruses TGX-07 (coding for TIMP-2, 68,93%) and TGX-09 (coding for PADI1, 57,56%) *in vitro* against SK-MEL-28 cell line. Importantly, the double transgene virus showed stronger anticancer effect than the combinatory therapy of TGX-07 plus TGX-09 (61,43%), (Figure 8a). Sign of double transgene anti-cancer superiority was also reported at lower concentration (10VP/cell), where double transgene exhibited stronger anti-cancer effect over single transgene viruses (Figure 8b).

Second construct coding for double transgenes (TGX-10) also exhibited stronger anti-cancer effect at the concentration of 1 VP/cell than single transgene viruses: TGX-07, TGX-09 and combinatory treatment: TGX-07 plus TGX-09 (respectively: 74,69; 86,05; 96,71 and 94,72 % of untreated cells) in A2058 cell line (Figure 9a). 10-fold higher concentration of the treatment (10VP/cell) also resulted in enhanced and superior efficacy of the virus TGX-10 over TGX-07, TGX-09 and combinatory treatment: TGX-07 plus TGX-09 in A375 cell line (respectively: 84,61; 89,09; 102,19 and 90,11 % of untreated cells) (Figure 9b).

### EXAMPLE 2 Animal studies - Immunodeficient xenograft A2058 melanoma mouse model

The study was carried out by TransCure bioServices SAS (TCS) at Archamps, France with 8-week-old female homozygous BALB/c nu/nu mice (provided by Charles River).

All animal experiments described in this study were reviewed and approved by the local ethic committee (CELEAG). Mice were hosted by groups of 6 individuals in TCS BSL-2 animal facility. Each mouse was uniquely identified. Animals were housed in a ventilated cage (type II 16x19x35 cm, floor area = 500 cm²) under the following controlled conditions:
- Room temperature (22 ± 2°C)
- Hygrometry (55 ± 10%)
- Photoperiod (12:12-hour light-dark cycle 7 am to 7 pm)
- Water and food (Ref. 2018, Harlan France) available ad libitum

### Cell inoculation

A2058 cell line (ATCC® CRL-11147™) was grown in DMEM medium supplemented with 10% FBS and 1% Penicillin/Streptomycin for 4 passages and detached with Trypsin-EDTA solution at 37°C for 2 min. After washing steps, cells were suspended in PBS at a concentration of 20x10⁶ cells/ml. 100 µl of the cell suspension (2x10⁶ cells) were injected subcutaneously in both flanks of each mouse (100 µl of cell suspension/flank).

### Randomization

Treatments were initiated the day after randomization (D1) and then continued at D3, D5, D12, D19 and D26. Mice were randomized into the following groups:
- Mock (n=8 mice) was treated with PBS. 50 µL of PBS were injected intratumorally into both tumors.
- Mice were treated with double transgene TGX-10 oncolytic virus (n=8 mice). Each mouse received 5x10⁶ VP (2.5x10⁶ VP/tumor/50 µL). 50 µL of solution 2 were injected intratumorally into both tumors.
- Mice were treated with double transgene TGX-12 oncolytic virus (n=8 mice). Each mouse received 5x10⁶ VP (2.5x10⁶ VP/tumor/50 µL). 50 µL of solution 2 were injected intratumorally into both tumors.
- Mice were treated with single transgene TGX-07 oncolytic virus (n=8 mice). Each mouse received 5x10⁶ VP (2.5x10⁶ VP/tumor/50 µL). 50 µL of solution 2 were injected intratumorally into both tumors.
- Mice were treated with single transgene TGX-09 oncolytic virus (n=8 mice). Each mouse received 5x10⁶ VP (2.5x10⁶ VP/tumor/50 µL). 50 µL of solution 2 were injected intratumorally into both tumors.
- Mice were treated with a mix of single transgene viruses: TGX-07 plus TGX-09 oncolytic viruses (n=8 mice). Each mouse received 5x10⁶ VP (2.5x10⁶ VP/tumor/50 µL). 50 µL of solution 2 were injected intratumorally into both tumors.

### Read-outs

Mice were monitored daily for unexpected signs of distress. Body weight and tumor volume were monitored 3 times per week until sacrifice. Tumor volumes were measured using a digital caliper. Tumor volumes (in mm³) were calculated according to the following formula: Volume = π/6 x width² x length.

Tumor volumes were measured over time and significant differences between groups were observed (Right tumors: TGX-12 vs TGX-07: p=0,007; TGX-12 vs TGX-09: p=0,0012; TGX-12 vs TG-07 plus TGX-09: p=0,0283; Left tumors: TGX-07 vs TGX-07 plus TGX-09: p=0,0081; TGX-09 vs TGX-07 plus TGX-09: p= 0,0167; Both tumors: TGX-12 vs TGX-07: p= 0,0076; TGX-12 vs TGX-09: p= 0,003; TGX-07 vs TGX-07 plus TGX-09: p=0,002; TGX-09 vs TGX-07 plus TGX-09: p<0,0001) (Figures 10a, 10b and 10c).

Double transgenes viruses (TGX-10 and TGX-12) and combinatory therapy: TGX-07 plus TGX-09 showed the best anticancer properties in terms of tumor volume reduction comparing to mock and single transgene viruses (TGX-07 and TGX-09) (Figure 10c). Mice treated with TGX-12 and TGX-07 plus TGX-09 maintained the right tumor volumes below 500 mm³ for all animals until the end of the experiment (Figure 10a).

Double transgene viruses TGX-10 and TGX-12 along with TGX-07 plus TGX-09 viruses showed synergistic effect as the they were able to reduce the tumor volume to a greater extent rather than viruses expressing single transgenes TGX-07 and TGX-09 (Table 2).

Body weight was monitored three times per week after tumor cell inoculation. Figure 10d shows body weight after tumor cell engraftment. For all groups, the body weight decreased after the initiation of the treatment. The treatment was well tolerated.

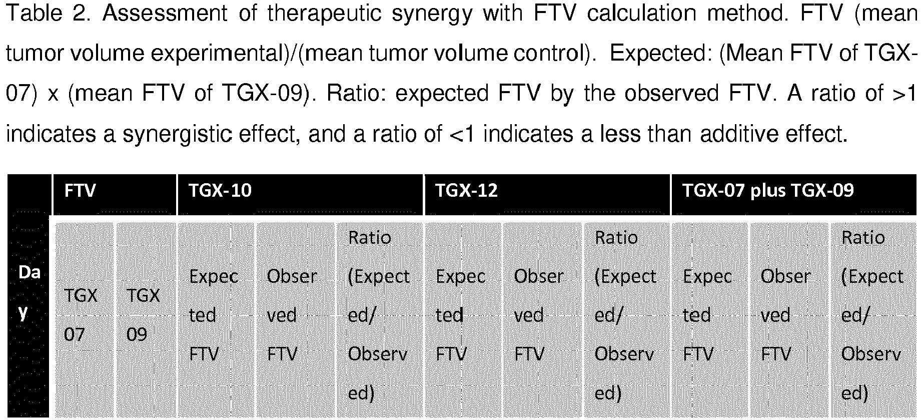

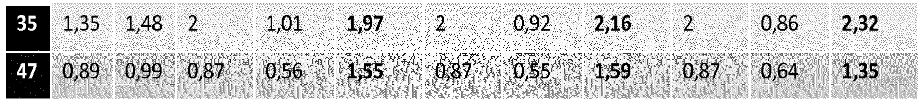

### EXAMPLE 3 Animal studies - humanized melanoma A2058 huNOG model

NOD/Shi-scid/IL-2Rynull immunodeficient (NOG) mice received chemical myoablative treatment and were reconstituted with human stem cells by intravenous injection of one of six cord blood-derived CD34+ hematopoietic stem and progenitor cells (60,000 cells per mouse; French Blood Bank, anonymized). After these injections, flow cytometry (Atune, Life Technologies) was used to assess the composition of the human CD45+ leukocytes in the blood. Humanization rate was defined as the ratio of circulating CD45+/total CD45+ (mCD45 and hCD45).

Humanized NOG mice were injected subcutaneously in each flank with 0.1 mL suspension containing 2x10⁶ A2058 melanoma cells. Mice were randomized based on tumor size (left and right) and humanization rate. Tumor dimensions (length (L) and width (W)) were measured three times per week with calipers and volumes calculated by using the formula (L x W2/2). Mice were treated intratumorally according to the Table 1. Body weight was also measured before the tumor engraftment and three times per week after engraftment.

All animal studies were reviewed and approved by the local ethics committee (01_TransCureBioServices-AB-01). Three to five mice were housed in ventilated type II cages (16 x 19 x 35 cm, 500 cm2 floor area) at room temperature (22 ± 2ºC), 55 ±10% humidity, 12/12-hour light-dark cycles (light 7 AM to 7 PM) and water and food ad libitum.

**Table 1. Treatment schedule.**

| **Cell line** | **Treatment** | **Number of mice** | **Treatment days** |
|---|---|---|---|
| **A2058** Inoculated s.c. both flanks at 2millions/flank | Mock | 8 | 1, 3, 5 (i.t. both tumors) and later on, every 7 days (on Days 12, 19, 26 - i.t. both tumors) |
| | TGX-12 | 8 | |
| | | | Virus dosage: 2,5x10"6 VP/50ul/tumor → VP/mouse 5x10^6 VP |
| | TGX-07 plus TGX-09 | 8 | 1, 3, 5 (i.t. both tumors) and later on, every 7 days (on Days 12, 19, 26 - i.t. both tumors) |
| | | | Virus dosage: 1,25x10^6 VP/50ul/tumor of TG0X7 plus 1,25x10^6 VP/50ul/tumor of TG0X9 and → VP/mouse 5x10^6 VP |
| **A2058** Inoculated s.c. into right flank; later on, on Day 19 left tumor will be inoculated at 0,5million/flank | TGX-12 (rechallenge) | 8 | 1, 3, 5 (i.t. right tumor) and later on, every 7 days (on Days 12, 19, 26 - i.t. right tumor). Left tumor will not be treated |
| | | | Virus dosage: 2,5x10^6 VP/50ul/right tumor → VP/mouse 5x10^6 VP |
| **A2058** Inoculated s.c. both flanks at 2millions/flank | TGX-12 | 6 | 1, 3, 5 (i.t. both tumors) and later on, every 7 days (on Days 12, 19, 26, 33 and 40 - i.t. both tumors) |
| | | | Virus dosage: 0,5x10^8 VP/50ul/tumor → VP/mouse 1x10^9 VP |

Double transgene virus (TGX-12) at both concentrations (5x10⁷ (464 mm³ on Day 28) and 1x10¹⁰ VP/mL (275 mm³ on Day 28)) and combinatory therapy: TGX-07 plus TGX-09 (5x10⁷ V/mL (488 mm³ on Day 28)) showed anticancer properties in terms of volume reduction comparing to mock (740 mm³ on Day 28) in humanized A2058 mouse model (Figure 11c). Dose-dependent effect of TGX-12 was observed, mice treated with higher concentration had greater tumor reduction (Figure 11a, 11b, 11c). The effect of double transgene virus (464 mm³ on Day 28) compared to combinatory therapy: TGX-07 plus TGX-09 (488 mm³ on Day 28) were comparable, however greater anti-cancer superiority was observed for the double transgene virus (Figure 11a, 11b, 11c).

Mice treated with TGX-12 (5x10⁷ VP/mL) into only right flank, showed abscopal effect as the re-challenged tumor on the left flank on 3-week time point exhibited inhibited, low kinetic growth (35 mm³ on Day 42) most likely due to development of protective immune memory cells (Figures 12a and 12b).

### REFERENCES

Bourboulia, D., Han, H., Jensen-Taubman, S., Gavil, N., Isaac, B., Wei, B., Neckers, L. & Stetler-Stevenson, W. G. 2013. TIMP-2 modulates cancer cell transcriptional profile and enhances E-cadherin/beta-catenin complex expression in A549 lung cancer cells. Oncotarget, 4, 166-76.
Feun, L. & Savaraj, N. 2006. Pegylated arginine deiminase: a novel anticancer enzyme agent. Expert Opin Investig Drugs, 15, 815-22.
Jahani, M., Noroznezhad, F. & Mansouri, K. 2018. Arginine: Challenges and opportunities of this two-faced molecule in cancer therapy. Biomed Pharmacother, 102, 594-601.
Park, I. S., Kang, S. W., Shin, Y. J., Chae, K. Y., Park, M. O., Kim, M. Y., Wheatley, D. N. & Min, B. H. 2003. Arginine deiminase: a potential inhibitor of angiogenesis and tumour growth. Br J Cancer, 89, 907-14.
Seo, D.-W., Li, H., Guedez, L., Wingfield, P. T., Diaz, T., Salloum, R., Wei, B.-Y. & Stetler-Stevenson, W. G. 2003. TIMP-2 Mediated Inhibition of Angiogenesis. Cell, 114, 171-180.

## Claims

1. An oncolytic adenoviral vector comprising an adenovirus serotype 5 (Ad5) nucleic acid backbone, a 24 bp deletion (D24) in the Rb binding constant region 2 of E1, a nucleic acid sequence encoding a peptidylarginine deiminase and a nucleic acid sequence encoding TIMP in the E3 region.

2. The oncolytic adenoviral vector according to claim 1, wherein peptidylarginine deiminase and TIMP are connected with IRES.

3. The oncolytic adenoviral vector according to claim 1, wherein peptidylarginine deiminase and TIMP are connected with P2A.

4. The oncolytic adenoviral vector according to any one of claims 1-3, wherein peptidylarginine deiminase and TIMP are placed under E3 promoter.

5. The oncolytic adenoviral vector according to any one of claims 1-3, wherein peptidylarginine deiminase and TIMP are placed under CMV(Cuo) promoter.

6. The oncolytic adenoviral vector according to any one of claims 1-5, wherein peptidylarginine deiminase is PADI1 and TIMP is TIMP2.

7. The oncolytic adenoviral vector according to any one of claims 1-6 comprising a native E1 A promoter.

8. The oncolytic adenoviral vector according to any one of claims 1-7 further comprising an Ad5/3 chimerism as a capsid modification.

9. The oncolytic adenoviral vector according to any one of claims 1-2 or 4-8 comprising a nucleotide sequence having at least 70% identity to SEQ ID NO: 1 or according to any of claims 3-4 or 5-8 comprising a nucleotide sequence having at least 70% identity to SEQ ID NO:2.

10. A cell comprising the adenoviral vector according to any one of claims 1-9.

11. A pharmaceutical composition comprising the adenoviral vector according to any one of claims 1-9.

12. The adenoviral vector according to any one of claims 1-9 for use in the treatment of cancer or in the manufacture of a medicament for treating cancer in a subject.

13. The adenoviral vector for use according to claim 12, wherein the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

14. The adenoviral vector for use according to claim 12 or 13, wherein the treatment of cancer in a subject is conducted through an intratumoral, intramuscular, intra-arterial, intravenous, intrapleural, intravesicular, intracavitary or peritoneal injection, or an oral administration.

15. The adenoviral vector for use according to claim 14, wherein the injection or the administration is conducted several times during the treatment period, or wherein an oncolytic adenoviral vector having a different fiber knob of the capsid compared to the vector of the earlier treatment is used.

16. The adenoviral vector for use according to any one of claims 12-15, wherein the treatment of cancer in a subject further comprises an administration of a checkpoint inhibitor, verapamil or another calcium channel blocker, autophagy inducing agents, temozolomide, chemotherapy or anti-CD20 therapy or other approaches for blocking of neutralizing antibodies, substances capable to downregulating regulatory T-cells in a subject to a subject.

17. A method of producing peptidylarginine deiminase and TIMP in a cell *in vitro,* wherein the method comprises:
i. carrying a vehicle comprising an oncolytic adenoviral vector according to any one of claims 1-9 to a cell, and
ii. expressing peptidylarginine deiminase and TIMP of said vector in the cell.

18. A use of the oncolytic adenoviral vector according to any one of claims 1-9 for producing peptidylarginine deiminase and TIMP in a cell *in vitro.*
